(19) Europäisches Patentamt
European Patent Office
Office européen des brevets

(11) **EP 3 004 181 B1**

(12) **EUROPEAN PATENT SPECIFICATION**

(45) Date of publication and mention
of the grant of the patent:
**02.01.2019 Bulletin 2019/01**

(51) Int Cl.:
***C08B 37/10*** (2006.01)     ***A61K 31/727*** (2006.01)
***C12N 5/02*** (2006.01)     ***A61F 2/00*** (2006.01)

(21) Application number: **14805119.6**

(22) Date of filing: **27.05.2014**

(86) International application number:
**PCT/SG2014/000230**

(87) International publication number:
**WO 2014/193308 (04.12.2014 Gazette 2014/49)**

(54) **HEPARAN SULPHATE**

HEPARANSULFAT

SULFATE D'HÉPARANE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.05.2013 SG 201304059**

(43) Date of publication of application:
**13.04.2016 Bulletin 2016/15**

(73) Proprietor: **Agency for Science, Technology and
Research
Singapore 138632 (SG)**

(72) Inventors:
• **COOL, Simon**
**Singapore 138648 (SG)**
• **NURCOMBE, Victor**
**Singapore 138648 (SG)**

(74) Representative: **Clegg, Richard Ian et al
Mewburn Ellis LLP
City Tower
40 Basinghall Street
London EC2V 5DE (GB)**

(56) References cited:
**WO-A1-2010/030244     WO-A2-2009/116951
WO-A2-2010/029278     US-A1- 2008 274 156**

• **S MURALI ET AL.: "Affinity-selected heparan
sulfate for bone repairs", BIOMATERIALS., vol.
34, no. 22, 28 April 2013 (2013-04-28), pages
5594-5605, XP28535342, ELSEVIER SCIENCE
PUBLISHERS BV., BARKING. ISSN: 0142-9612**
• **UYGUN, B. E. ET AL.: 'Effects of Immobilized
Glycosaminoglycans on the Proliferation and
Differentiation of Mesenchymal Stem Cells'
TISSUE ENGINEERING: PART A vol. 15, no. 11,
2009, pages 3499 - 3512, XP055041004**

**Description**

**Field of the Invention**

The present invention relates to heparan sulphates and particularly, although not exclusively, to heparan sulphates that bind vitronectin.

**Background to the Invention**

There is a strong correlation between the global economic cost of critical illness to society and the potential for stem cells to alleviate some of these problems [1]. This is driving stem cell therapy sales exponentially, despite their limited effectiveness [2]. The potential for human embryonic stem cell (hESC) and induced pluripotent stem cells therapy to contribute to this growing market is substantial. These cells have the ability to differentiate into all the cell types present in an adult. hESCs have been posited as being central to such regenerative therapeutic strategies if their provision can be made reliable [3]. The directed differentiation of hESCs down cardiomyocyte, hepatocyte or insulin-producing cell lineages in particular carries much promise. In 2009 and 2010, the U.S. Food and Administration (FDA) approved two clinical trials for the treatment of grade A thoracic spinal cord injury (NCT01217008 and NCT01344993) [4] as well as for macular dystrophy and dry-aged related macular degeneration (NCT01345006 and NCT01344993) [5] that employed cells derived from human embryonic stem cells. However the problem remains that it is still very difficult to continuously preserve these cells in their pristine state prior to differentiation, a property that is essential to ensure that sufficient cells will be available for the subsequent directed differentiation required to meet future clinical demand.

The first and most important challenge is to alleviate the reliance on inactivated mouse or human feeder cell layers for hESC maintenence. The second is to eliminate the use of Matrigel™, a useful but poorly defined product derived from murine sarcoma basement membrane. Thirdly, the dispensing of bovine serum albumin (BSA) and fetal calf serum (FCS) from cell culture media would be a major advance. These obstacles need to be overcome if we are to propagate cells with maximum safety in a naive state in large numbers.

Cell culture media formulation has made rapid progress, and a number of chemically-defined media such as XVIVO-10, hESF9, mTeSR™1 and STEMPRO are now available. [6-9] However, in the area of properly defined cell culture surfaces, there is still much work to be done. Although many research groups have proposed methods for culturing hESCs, including laminin (LN) [10, 11], vitronectin (VN) [12], fibronectin (FN) [13], E-cadherin [14], peptides [15, 16] or PMEDSAH polymers [17], none of these studies ever calculated the actual surface density of the applied compounds, nor their economic cost-benefits for commercial scale hESC propagation.

The most widely used method to immobilize extracellular matrix (ECM) proteins for cell culture is through passive adsorption onto culture surfaces. We have previously shown that hESCs can be successfully propagated long-term on vitronectin (VN) adsorbed surfaces [18]. However, this method had been shown by Marson *et al.* to result in the steric hindrance or conformational perturbation of the VN, resulting in a loss-of-function of the protein [19].

Glycosaminoglycans are complex, linear, highly charged carbohydrates that interact with a wide range of proteins to regulate their function; they are usually synthesized attached to core protein. GAGs are classified into nonsulfated (HA) and sulfated (CS, DS, KS, heparin and HS).

Among the GAGs, the heparan sulfate (HS) family is of particular interest because of its ability to interact with targeted proteins based on specific sequences within its domains. The family (heparin and HS) consist of repeating uronic acid-(1→4)-D-glucosamine disaccharide subunits with variable pattern of N-, and O-sulfation. For example, the anticoagulant activity of heparin requires 30-sulfation in glucosamine residue with a unique pentasaccharide arrangement [20]. A unique sulfation pattern is also apparent for ECM proteins; an avid heparin-binding variant that binds FN is particularly highly charged, with 7 to 8 N-sulfated disaccharides being required, and with a larger domain than usual (> 14 residues) [21, 22]. However, HS differs from such sulfated heparins by having highly sulfated NS domains separated by unsulfated NA domains; such dispositions provide unique arrangements for selectively binding proteins, without the side effects of heparin [23].

The disaccharide composition of HS can be elucidated through a series of enzymatic cleavages [24-26] using the *Flavobacterium heparinium* enzymes heparinase I, II and III to cleave the glycosidic bonds. More than 90 % depolymerization of heparin or HS is possible when all 3 heparinases are used in combination [27, 28]. The resulting disaccharide mixtures can be analyzed by PAGE [29], SAX-HPLC [30], or highly sensitive capillary electrophoresise (CE) [31-34] by comparison to known disaccharides standards. Numerous studies have also shown that HS is important for the maintenance and proliferation of stem cells [15, 35-38]. Uygun *et al.* demonstrated that GAG-derivatized surfaces are able to promote 5-fold increases in mesenchymal stem cell growth compared to TCPS surfaces [39]. The possibility presents itself that it may be possible to manipulate particular HS variants in such a way as to allow for the presention of VN in a more effective manner. Immobilization strategies include coupling GAGs with BSA to allow adsorption onto surfaces [40, 41]; EDC chemistry to covalently immobilize disaccharide units [42]; biotinylation of GAGs and coupling to streptavidin-coated surfaces [43-45], and positively charged plasma polymer films [19, 46]. To analyze the elemental compositions on such derivatized surfaces, techniques such as XPS could be employed [47].

We herein describe the search for an HS variant with high VN-binding ability to improve VN surface density for cell culture. We first describe the purification of a novel VN-binding HS species from a raw HS starting material by affinity chromatography, using the VN-heparin binding domain (VN-HBD) as a capture ligand. A full range of biochemical assays to confirm the binding ability of this purified HS from the non-binding and starting material were then employed. The length, sulfation pattern and composition requirements of HS were then analyzed by ELISA and CE. Three different strategies were then assessed for their suitability in the immobilization of the purified HS variant onto TCPS. A method that utilized allylamine offered distinct advantages over the other methods tested.

Utilizing such methods to present sufficient VN for hESC culture is therefore not efficient. Instead of modifying the protein, which might lead to deleterious effects, modifying the surface for efficient VN immobilization appears a more rational approach. Here we explore various strategies that allow for the capture of sufficient unmodified VN for hESC culture that are also cheap, scalable and efficient.

WO2010/029278 describes the isolation and identification of glycosaminoglycans capable of binding to proteins having a heparin-binding domain, as well as the use of the glycosaminoglycans isolated in the growth and/or development of tissue.

## Summary of the Invention

The present invention concerns a heparan sulphate preparation, heparan sulphate HS9. HS9 has been found to bind Vitronectin and show utility in providing cell culture substrates capable of supporting culture and proliferation of stem cells whilst maintaining the stemness (e.g. pluripotency or multipotency) of the cultured stem cells.

The invention is as defined in the appended claims.

HS9 refers to a novel class of structurally and functionally related isolated heparan sulphate.

In one aspect of the present invention an heparan sulphate HS9 is provided. HS9 is provided in isolated form or in purified form. This may comprise providing a composition in which the heparan sulphate component is at least 80% HS9, more preferably one of at least 85%, 90%, 95%, 96%, 97%, 98%, 99% or 100%.

In preferred aspects, HS9 is capable of binding a peptide or polypeptide having the amino acid sequence of PRPSLAKKQRFRHRNRRKGYRSQRGHSRGRNQNSRR (SEQ ID NO: 1) or RPSLAKKQRFRHRNRKGYRSQRGHSRGRNQNSRR (SEQ ID NO:3). The peptide may have one or more additional amino acids at one or both ends of this sequence. For example, the peptide may have any of 1, 2, 3, 4, 5, 6 ,7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 or more amino acids at one or both ends of this sequence.

In other aspects the polypeptide is a Vitronectin protein. In some embodiments HS9 binds to a peptide having or consisting of the amino acid sequence of any of SEQ ID NO:1, SEQ ID NO:3 or Vitronectin protein with a $K_D$ of less than $100\mu M$, more preferably less than one of $50\mu M$, $40\mu M$, $30\mu M$, $20\mu M$, $10\mu M$, $1\mu M$, 100nM, 10nM, 1nM, or 100pM.

HS9 may be obtained, identified, isolated or enriched according to the inventors' methodology described herein, which may comprise the following steps:

(i) providing a solid support having polypeptide molecules adhered to the support, wherein the polypeptide comprises or consists of a heparin-binding domain having the amino acid sequence of PRPSLAKKQRFRHRNRRKGYRSQRGHSRGRNQNSRR or RPSLAKKQRFRHRNRKGYRSQRGHSRGRNQNSRR;
(ii) contacting the polypeptide molecules with a mixture comprising glycosaminoglycans such that polypeptide-glycosaminoglycan complexes are allowed to form;
(iii) partitioning polypeptide-glycosaminoglycan complexes from the remainder of the mixture;
(iv) dissociating glycosaminoglycans from the polypeptide-glycosaminoglycan complexes;
(v) collecting the dissociated glycosaminoglycans.

In the inventors' methodology the mixture may comprise glycosaminoglycans obtained from commercially available sources. One suitable source is a heparan sulphate fraction, e.g. a commercially available heparan sulphate. One suitable heparan sulphate fraction can be obtained during isolation of heparin from porcine intestinal mucosa (e.g. Celsus Laboratories Inc. - sometimes called "Celsus HS").

Other suitable sources of heparan sulphate include heparan sulphate from any mammal (human or non-human), particularly from the kidney, lung or intestinal mucosa. In some embodiments the heparan sulphate is from pig (porcine) or cow (bovine) intestinal mucosa, kidney or lung.

In another aspect of the present invention a composition comprising HS9 according to any one of the aspects above and Vitronectin protein is provided.

In one aspect of the present invention a pharmaceutical composition or medicament is provided comprising HS9 in accordance with the aspects described above. The pharmaceutical composition or medicament may further comprise a pharmaceutically acceptable carrier, adjuvant or diluent.

In some embodiments the pharmaceutical composition is for use in a method of treatment of disease. In some embodiments the pharmaceutical composition or medicament may further comprise Vitronectin protein. In some other embodiments the pharmaceutical composition or medicament contains HS9 as the sole active ingredient.

In another aspect of the present invention HS9 is provided for use in a method of medical treatment. In a related aspect of the present invention the use of HS9 in the manufacture of a medicament for use in a method of medical treatment is provided.

In a further aspect of the present invention a biocompatible implant or prosthesis comprising a biomaterial and HS9 is provided. In some embodiments the implant or prosthesis is coated with HS9. In some embodiments the implant or prosthesis is impregnated with HS9. The implant or prosthesis may be further coated or impregnated with Vitronectin protein.

In another aspect of the present invention a method of forming a biocompatible implant or prosthesis is provided, the method comprising the step of coating or impregnating a biomaterial with HS9. In some embodiments the method further comprises coating or impregnating the biomaterial with Vitronectin protein.

In another aspect of the present invention a cell culture article or container is provided having a cell culture substrate comprising HS9. In some embodiments at least a part of the cell culture surface may be coated in HS9. The cell culture article or container may further comprise Vitronectin.

In another aspect of the present invention a method of forming a cell culture substrate is provided, the method comprising applying HS9 to a cell culture support surface.

In another aspect of the present invention an *in vitro* cell culture is provided, the culture comprising cells in contact with a cell culture substrate comprising HS9. In some embodiments the cell culture substrate further comprises Vitronectin.

In another aspect of the present invention a method of culturing cells is provided, the method comprising culturing cells *in vitro* in contact with a cell culture substrate comprising HS9. In some embodiments the cell culture substrate further comprises Vitronectin.

In another aspect of the present invention HS9 is provided for use in cell attachment to a cell culture substrate.

In a further aspect of the present invention culture media is provided, the culture media comprising HS9.

In another aspect of the present invention the use of HS9 in cell culture *in vitro* is provided.

In some aspects of the present disclosure a method of culturing stem cells *in vitro* is provided, the method comprising culturing stem cells *in vitro* in contact with heparan sulphate HS9. The HS9 is preferably exogenous and isolated, and added to the culture as a supplement, e.g. as part of the culture media.

In preferred aspects stem cells cultured whilst in contact with HS9 expand in population, i.e. increase in number of stem cells, and a high proportion of cells in the culture maintain the multipotent or pluripotent characteristics of the parent stem cell (e.g. ability of the stem cell to differentiate into specific tissue types characteristic of the type of stem cell). For example, preferably one of at least 20%, 30%, 40%, 50%, 60%, 70%, 80%, 90%, 95%, 96%, 97%, 98%, 99% or 100% of stem cells in the culture exhibit the multipotent or pluripotent characteristics of the parent stem cells. Preferably, HS9 acts to increase the proportion (e.g. percentage) of cells in the culture that are multipotent or pluripotent. This may be measured relative to the number of cells in the starting culture that are multipotent or pluripotent. In some aspects of the disclosure the increase in proportion of multipotent or pluripotent cells may be compared against a control culture of stem cells subject to corresponding culture conditions that differ only by lack of the presence of exogenous HS9. Stem cells cultures may optionally contain, or not contain, Vitronectin.

In yet a further aspect of the present disclosure a kit of parts is provided, the kit comprising a predetermined amount of HS9 and a predetermined amount of Vitronectin. The kit may comprise a first container containing the predetermined amount of HS9 and a second container containing the predetermined amount of Vitronectin. The kit may be provided for use in a method of cell culture.

In a further aspect of the present invention a cell culture article or container is provided having a cell culture substrate comprising an isolated heparan sulphate capable of binding a peptide or polypeptide wherein the peptide or polypeptide is in contact with the isolated heparan sulphate. In some embodiments at least a part of the cell culture surface is coated in or impregnated with the isolated heparan sulphate.

In a related aspect of the present invention a method of forming a cell culture substrate is provided, the method comprising applying an isolated heparan sulphate capable of binding a peptide or polypeptide to a cell culture support surface and contacting the isolated heparan sulphate with the peptide or polypeptide.

In a related aspect of the present invention an *in vitro* cell culture is also provided, comprising cells in contact with a cell culture substrate comprising an isolated heparan sulphate capable of binding a peptide or polypeptide wherein the peptide or polypeptide is in contact with the isolated heparan sulphate.

In a related aspect of the present invention a method of culturing cells is provided, the method comprising culturing cells *in vitro* in contact with a cell culture substrate comprising an isolated heparan sulphate capable of binding a peptide or polypeptide wherein the peptide or polypeptide is in contact with the isolated heparan sulphate.

In some parts of the disclosure the peptide or polypeptide is an extracellular matrix protein, or peptide derived therefrom.

In some embodiments the isolated heparan sulphate is obtained, identified, isolated or enriched according to the inventors' methodology described herein, which may comprise the following steps:

(i) providing a solid support having polypeptide molecules adhered to the support, wherein the polypeptide comprises or consists of a heparin-binding domain from the protein of interest;
(ii) contacting the polypeptide molecules with a mixture comprising glycosaminoglycans, preferably a heparan sulphate preparation, such that polypeptide-glycosaminoglycan complexes are allowed to form;
(iii) partitioning polypeptide-glycosaminoglycan complexes from the remainder of the mixture;
(iv) dissociating glycosaminoglycans from the polypeptide-glycosaminoglycan complexes;
(v) collecting the dissociated glycosaminoglycans.

**Description**

The inventors have used a sequence-based affinity chromatography platform to exploit the heparin-binding domain of Vitronectin (VN). This allowed the enrichment of a VN-binding heparan sulfate (HS) fraction. The binding avidity and specificity of the VN-binding HS9$^{+ve}$ for VN was confirmed using a combination of Enzyme-Linked Immunosorbant Assay (ELISA) and capillary electrophoresis. Plasma polymerization of allylamine (AA) polymers onto tissue culture-treated polystyrene (TCPS) surfaces allowed for the efficient capture of HS9$^{+ve}$. The surface combination of HS and VN supported the attachment of hESCs. Surface densities of each coating layer were confirmed by both radiolabeling and binding assays. HS compositional analysis revealed that 6O-sulfation together with N-sulfation on glucosamine residues, and lengths greater than 3 disaccharide units were critical for HS9$^{+ve}$ binding to VN. This combination substrate allows for a significant reduction in the VN surface density required for cell attachment over orthodox passive VN adsorption. The method can be easily up-scaled for the 3-dimensional culture of hESC in a cost-efficient manner.

HS9

The present invention relates to a class of heparan sulphate molecule called HS9. HS9 molecules are obtainable by methods of enriching mixtures of compounds containing one or more GAGs that bind to a polypeptide corresponding to a heparin-binding domain of Vitronectin. In particular, HS9 molecules can be obtained by enriching for heparan sulphate that binds to a heparan binding domain of Vitronectin which domain comprises, or consists of, the amino acid sequence

PRPSLAKKQRFRHRNRRKGYRSQRGHSRGRNQNSRR or
PRPSLAKKQRFRHRNRKGYRSQRGHSRGRNQNSRR. The enrichment process may be used to isolate HS9.

The present invention also relates to mixtures of compounds enriched with HS9, and methods of using such mixtures.

In addition to being obtainable by the methodology described here, HS9 can also be defined functionally and structurally.

Functionally, an HS9 is capable of binding a peptide having, or consisting of, the amino acid sequence of PRPSLAKKQRFRHRNRRKGYRSQRGHSRGRNQNSRR (SEQ ID NO:1) or PRPSLAKKQRFRHRNRKGYRSQRGHSRGRNQNSRR (SEQ ID NO:3). The peptide may contain one or more additional amino acids on one or both ends of the peptide.

Preferably, HS9 binds the peptide with a $K_D$ of less than 100$\mu$M, more preferably less than one of 50$\mu$M, 40$\mu$M, 30$\mu$M, 20$\mu$M, 10$\mu$M, 1$\mu$M, 100nM, 10nM, 1nM, or 100pM.

Preferably, HS9 also binds Vitronectin protein with a $K_D$ of less than 100$\mu$M, more preferably less than one of 50$\mu$M, 40$\mu$M, 30$\mu$M, 20$\mu$M, 10$\mu$M, 1$\mu$M, 100nM, 10nM, 1nM, or 100pM. Binding between HS9 and Vitronectin protein may be determined by the following assay method.

Vitronectin is dissolved in Blocking Solution (0.2% gelatin in SAB) at a concentration of 3 $\mu$g/ml and a dilution series from 0-3$\mu$g/ml in Blocking Solution is established. Dispensing of 200 $\mu$l of each dilution of Vitronectin into triplicate wells of Heparin/GAG Binding Plates pre-coated with heparin; incubated for 2hrs at 37°C, washed carefully three times with SAB and 200$\mu$l of 250ng/ml biotinylated anti- Vitronectin added in Blocking Solution. Incubation for one hour at 37°C, wash carefully three times with SAB, 200$\mu$l of 220ng/ml ExtrAvidin-AP added in Blocking Solution, Incubation for 30mins at 37°C, careful washing three times with SAB and tap to remove residual liquid, 200$\mu$l of Development Reagent (SigmaFAST p-Nitrophenyl phosphate) added. Incubate at room temperature for 40 minutes with absorbance reading at 405nm within one hour.

In this assay, binding may be determined by measuring absorbance and may be determined relative to controls such as Vitronectin protein in the absence of added heparan sulphate, or Vitronectin protein to which an heparan sulphate is added that does not bind Vitronectin protein.

The binding of HS9 is preferably specific, in contrast to non-specific binding and in the context that the HS9 can be selected from other heparan sulphates and/or GAGs by a method involving selection of heparan sulphates exhibiting a high affinity binding interaction with the peptide comprising PRPSLAKKQRFRHRNRRKGYRSQRGHSRGRNQNSRR, or PRPSLAKKQRFRHRNRKGYRSQRGHSRGRNQNSRR or with Vitronectin protein.

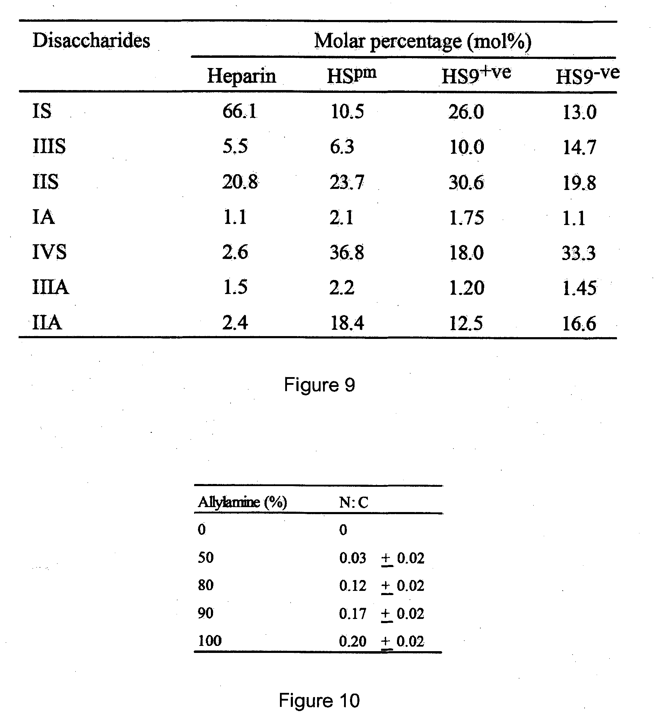

The disaccharide composition of HS9 following digestion with heparin lyases I, II and III to completion and then subjecting the resulting disaccharide fragments to capillary electrophoresis analysis is shown in Figure 9.

HS9 according to the present invention includes heparan sulphate that has a disaccharide composition within ± 10% (more preferably ± one of 9%, 8%, 7%, 6%, 5%, 4%, 3%, 2%, 1% or 0.5%) of the normalised percentage values shown for each disaccharide in Figure 9, as determined by digestion with heparin lyases I, II and III to completion and then subjecting the resulting disaccharide fragments to capillary electrophoresis analysis.

The disaccharide composition of HS9 as determined by digestion with heparin lyases I, II and III to completion and then subjecting the resulting disaccharide fragments to capillary electrophoresis analysis may have a disaccharide composition according to any one of the following:

| Disaccharide | Normalised weight percentage |
| --- | --- |
| ΔUA,2S-GlcNS,6S | 26.0±3.0 |
| ΔUA,2S-GlcNS | 10.0±2.0 |
| ΔUA-GlcNS,6S | 30.6±3.0 |
| ΔUA,2S-GlcNAc,6S | 1.75±2.0 or 1.7±2.0 |
| ΔUA-GlcNS | 18.0±3.0 |
| ΔUA,2S-GlcNAc | 1.2±0.5 |
| ΔUA-GlcNAc,6S | 12.5±3.0 |

or

| Disaccharide | Normalised weight percentage |
| --- | --- |
| ΔUA,2S-GlcNS,6S | 26.0±2.0 |
| ΔUA,2S-GlcNS | 10.0±2.0 |
| ΔUA-GlcNS,6S | 30.6±2.0 |
| ΔUA,2S-GlcNAc,6S | 1.75±2.0 or 1.7±2.0 |
| ΔUA-GlcNS | 18.0±2.0 |
| ΔUA,2S-GlcNAc | 1.2±0.5 |
| ΔUA-GlcNAc,6S | 12.5±2.0 |

or

| Disaccharide | Normalised weight percentage |
| --- | --- |
| ΔUA,2S-GlcNS,6S | 26.0±2.0 |
| ΔUA,2S-GlcNS | 10.0±1.0 |
| ΔUA-GlcNS,6S | 30.6±2.0 |
| ΔUA,2S-GlcNAc,6S | 1.75±1.0 or 1.7±1.0 |
| ΔUA-GlcNS | 18.0±2.0 |
| ΔUA,2S-GlcNAc | 1.2±0.5 |
| ΔUA-GlcNAc,6S | 12.5±2.0 |

or

| Disaccharide | Normalised weight percentage |
| --- | --- |
| ΔUA,2S-GlcNS,6S | 26.0±1.0 |

(continued)

| Disaccharide | Normalised weight percentage |
|---|---|
| ΔUA,2S-GlcNS | 10.0±0.4 |
| ΔUA-GlcNS,6S | 30.6±1.0 |
| ΔUA,2S-GlcNAc,6S | 1.75±0.6 or 1.7±0.6 |
| ΔUA-GlcNS | 18.0±3.0 |
| ΔUA,2S-GlcNAc | 1.2±0.4 |
| ΔUA-GlcNAc,6S | 12.5±1.0 |

or

| Disaccharide | Normalised weight percentage |
|---|---|
| ΔUA,2S-GlcNS,6S | 26.0±0.75 |
| ΔUA,2S-GlcNS | 10.0±0.3 |
| ΔUA-GlcNS,6S | 30.6±0.75 |
| ΔUA,2S-GlcNAc,6S | 1.75±0.45 or 1.7±0.45 |
| ΔUA-GlcNS | 18.0±2.25 |
| ΔUA,2S-GlcNAc | 1.2±0.3 |
| ΔUA-GlcNAc,6S | 12.5±0.75 |

or

| Disaccharide | Normalised weight percentage |
|---|---|
| ΔUA,2S-GlcNS,6S | 26.0±0.5 |
| ΔUA,2S-GlcNS | 10.0±0.2 |
| ΔUA-GlcNS,6S | 30.6±0.5 |
| ΔUA,2SGlcNAc,6S | 1.75±0.3 or 1.7±0.3 |
| ΔUA-GlcNS | 18.0±1.5 |
| ΔUA,2S-GlcNAc | 1.2±0.2 |
| ΔUA-GlcNAc,6S | 12.5±0.5 |

In preferred embodiments the total weight percentage of the 8 disaccharides listed is 100% (optionally ± 3.0% or less, or ± 2.0% or less, ± 1.0% or less, ± 0.5% or less). Digestion of HS9 with heparin lyases I, II and III and/or capillary electrophoresis analysis of disaccharides is preferably performed in accordance with the Examples.

Digestion of HS preparations with heparin lyase enzymes may be conducted as follows: HS preparations (1 mg) are each dissolved in 500 $\mu$L of sodium acetate buffer (100 mM containing 10 mM calcium acetate, pH 7.0) and 2.5 mU each of the three enzymes is added; the samples are incubated at 37 °C overnight (24 h) with gentle inversion (9 rpm) of the sample tubes; a further 2.5 mU each of the three enzymes is added to the samples which are incubated at 37 °C for a further 48 h with gentle inversion (9 rpm) of the sample tubes; digests are halted by heating (100 °C, 5 min) and are then lyophilized; digests are resuspended in 500 $\mu$L water and an aliquot (50 $\mu$L) is taken for analysis.

Capillary electrophoresis (CE) of disaccharides from digestion of HS preparations may be conducted as follows: capillary electrophoresis operating buffer is made by adding an aqueous solution of 20 mM $H_3PO_4$ to a solution of 20 mM $Na_2HPO_4.12H_2O$ to give pH 3.5; column wash is 100 mM NaOH (diluted from 50 % w/w NaOH); operating buffer and column wash are both filtered using a filter unit fitted with 0.2 $\mu$m cellulose acetate membrane filters; stock solutions of disaccharide Is (e.g. 12) are prepared by dissolving the disaccharides in water (1 mg/mL); calibration curves for the standards are determined by preparing a mix containing all standards containing 10 $\mu$g/100 $\mu$L of each disaccharide and a dilution series containing 10, 5, 2.5, 1.25, 0.625, 0.3125 $\mu$g/100 $\mu$L is prepared; including 2.5 $\mu$g of internal standard (ΔUA,2S-GlcNCOEt,6S). The digests of HS are diluted (50 $\mu$L/mL) with water and the same internal standard is added (2.5 $\mu$g) to each sample. The solutions are freeze-dried and re-suspended in water (1 mL). The samples are filtered using PTFE hydrophilic disposable syringe filter units.

Analyses are performed using a capillary electrophoresis instrument on an uncoated fused silica capillary tube at 25 °C using 20 mM operating buffer with a capillary voltage of 30 kV. The samples are introduced to the capillary tube using hydrodynamic injection at the cathodic (reverse polarity) end. Before each run, the capillary is flushed with 100 mM NaOH (2 min), with water (2 min) and pre-conditioned with operating buffer (5 min). A buffer replenishment system replaces the buffer in the inlet and outlet tubes to ensure consistent volumes, pH and ionic strength are maintained. Water only blanks are run at both the beginning, middle and end of the sample sequence. Absorbance is monitored at 232 nm. All data is stored in a database and is subsequently retrieved and re-processed. Duplicate or triplicate digests/analyses may be performed and the normalized percentage of the disaccharides in the HS digest is calculated as the mean average of the results for the analyses.

To identify HS9 the inventors used a method that involves enriching for glycosaminoglycan molecules that exhibit binding to particular polypeptides having a heparin-binding domain. Isolated GAG mixtures and/or molecules can then be identified and tested for their ability to modulate the growth and differentiation of cells and tissue expressing a protein containing the heparin-binding domain. This enables the controlled analysis of the effect of particular GAG saccharide sequences on the growth and differentiation of cells and tissue, both *in vitro* and *in vivo.* This methodology is described in PCT/GB2009/000469 (WO2010/030244). The inventors applied this methodology to Vitronectin in order to isolate and characterise GAGs having high binding to Vitronectin.

Accordingly, to identify HS9 the inventors provided a method of isolating glycosaminoglycans capable of binding to proteins having heparin/heparan-binding domains, the method comprising:

(i) providing a solid support having polypeptide molecules adhered to the support, wherein the polypeptide comprises a heparin-binding domain;
(ii) contacting the polypeptide molecules with a mixture comprising glycosaminoglycans such that polypeptide-glycosaminoglycan complexes are allowed to form;
(iii) partitioning polypeptide-glycosaminoglycan complexes from the remainder of the mixture;
(iv) dissociating glycosaminoglycans from the polypeptide-glycosaminoglycan complexes;
(v) collecting the dissociated glycosaminoglycans.

The inventors used this method to identify a GAG capable of binding to Vitronectin (which they called HS9), wherein the polypeptide used in the inventors' methodology comprised the heparin-binding domain of PRPSLAKKQRFRHRNRRKGYRSQRGHSRGRNQNSRR (SEQ ID NO:1) or PRPSLAKKQRFRHRNRKGYRSQRGHSRGRNQNSRR (SEQ ID NO:3).

In the inventors' methodology, the mixture comprising GAGs may contain synthetic glycosaminoglycans. However, GAGs obtained from cells or tissues are preferred. For example, the mixture may contain extracellular matrix wherein the extracellular matrix material is obtained by scraping live tissue *in situ* (i.e. directly from the tissue in the body of the human or animal from which it is obtained) or by scraping tissue (live or dead) that has been extracted from the body of the human or animal. Alternatively, the extracellular matrix material may be obtained from cells grown in culture. The extracellular matrix material may be obtained from connective tissue or connective tissue cells, e.g. bone, cartilage, muscle, fat, ligament or tendon. In one embodiment commercially available heparan sulphate from Porcine Mucosa (Celsus HS or HSpm) was used.

The GAG component may be extracted from a tissue or cell sample or extract by a series of routine separation steps (e.g. anion exchange chromatography), well known to those of skill in the art.

GAG mixtures may contain a mixture of different types of glycosaminoglycan, which may include dextran sulphates, chondroitin sulphates and heparan sulphates. Preferably, the GAG mixture contacted with the solid support is enriched for heparan sulphate. A heparan sulphate-enriched GAG fraction may be obtained by performing column chromatography on the GAG mixture, e.g. weak, medium or strong anion exchange chromatography, as well as strong high pressure liquid chromatography (SAX-HPLC), with selection of the appropriate fraction.

The collected GAGs may be subjected to further analysis in order to identify the GAG, e.g. determine GAG composition or sequence, or determine structural characteristics of the GAG. GAG structure is typically highly complex, and, taking account of currently available analytical techniques, exact determinations of GAG sequence structure are not possible in most cases.

However, the collected GAG molecules may be subjected to partial or complete saccharide digestion (e.g. chemically by nitrous acid or enzymatically with lyases such as heparinase III) to yield saccharide fragments that are both characteristic and diagnostic of the GAG. In particular, digestion to yield disaccharides (or tetrasaccharides) may be used to measure the percentage of each disaccharide obtained which will provide a characteristic disaccharide "fingerprint" of the GAG.

The pattern of sulphation of the GAG can also be determined and used to determine GAG structure. For example, for heparan sulphate the pattern of sulphation at amino sugars and at the C2, C3 and C6 positions may be used to characterise the heparan sulphate.

Disaccharide analysis, tetrasaccharide analysis and analysis of sulphation can be used in conjunction with other analytical techniques such as HPLC, mass spectrometry and NMR which can each provide unique spectra for the GAG. In combination, these techniques may provide a definitive structural characterisation of the GAG.

A high affinity binding interaction between the GAG and heparin-binding domain indicates that the GAG will contain a specific saccharide sequence that contributes to the high affinity binding interaction. A further step may comprise determination of the complete or partial saccharide sequence of the GAG, or the key portion of the GAG, involved in the binding interaction.

GAG-polypeptide complexes may be subjected to treatment with an agent that lyses glycosaminoglycan chains, e.g. a lyase. Lyase treatment may cleave portions of the bound GAG that are not taking part in the binding interaction with the polypeptide. Portions of the GAG that are taking part in the binding interaction with the polypeptide may be protected from lyase action. After removal of the lyase, e.g. following a washing step, the GAG molecule that remains bound to the polypeptide represents the specific binding partner ("GAG ligand") of the polypeptide. Owing to the lower complexity of shorter GAG molecules, following dissociation and collection of the GAG ligand, a higher degree of structural characterisation of the GAG ligand can be expected. For example, the combination of any of the saccharide sequence (i.e. the primary (linear) sequence of monosaccharides contained in the GAG ligand), sulphation pattern, disaccharide and/or tetrasaccharide digestion analysis, NMR spectra, mass spectrometry spectra and HPLC spectra may provide a high level of structural characterisation of the GAG ligand.

As used herein, the terms 'enriching', 'enrichment', 'enriched', etc. describes a process (or state) whereby the relative composition of a mixture is (or has been) altered in such a way that the fraction of that mixture given by one or more of those entities is increased, while the fraction of that mixture given by one or more different entities is decreased. GAGs isolated by enrichment may be pure, i.e. contain substantially only one type of GAG, or may continue to be a mixture of different types of GAG, the mixture having a higher proportion of particular GAGs that bind to the heparin-binding domain relative to the starting mixture.

As used herein, the process of 'contacting' involves the bringing into close physical proximity of two or more discrete entities. The process of 'contacting' involves the bringing into close proximity of two or more discrete entities for a time, and under conditions, sufficient to allow a portion of those two or more discrete entities to interact on a molecular level. Preferably, as used herein, the process of 'contacting' involves the bringing into close proximity of the mixture of compounds possessing one or more GAGs and the polypeptide corresponding to the heparin-binding domain of a heparin-binding factor. Examples of 'contacting' processes include mixing, dissolving, swelling, washing. In preferred embodiments 'contact' of the GAG mixture and polypeptide is sufficient for complexes, which may be covalent but are preferably non-covalent, to form between GAGs and polypeptides that exhibit high affinity for each other.

The polypeptide may comprise the full length or near full length primary amino acid sequence of a selected protein having a heparin-binding domain. Due to folding that may occur in longer polypeptides leading to possible masking of the heparin-binding domain from the GAG mixture, it is preferred for the polypeptide to be short. Preferably, the polypeptide will have an amino acid sequence that includes the heparin-binding domain and optionally including one or more amino acids at one or each of the N- and C-terminals of the peptides. These additional amino acids may enable the addition of linker or attachment molecules to the polypeptide that are required to attach the polypeptide to the solid support.

In preferred embodiments of the inventors' methodology, in addition to the number of amino acids in the heparin-binding domain the polypeptide contains 1-20, more preferably 1-10, still more preferably 1-5 additional amino acids. In some embodiments the amino acid sequence of the heparin-binding domain accounts for at least 80% of the amino acids of the polypeptide, more preferably at least 90%, still more preferably at least 95%. In order to adhere polypeptides to the surface of a solid support the polypeptides are preferably modified to include a molecular tag, and the surface of the solid support is modified to incorporate a corresponding molecular probe having high affinity for the molecular tag, i.e. the molecular tag and probe form a binding pair. The tag and/or probe may be chosen from any one of: an antibody, a cell receptor, a ligand, biotin, any fragment or derivative of these structures, any combination of the foregoing, or any other structure with which a probe can be designed or configured to bind or otherwise associate with specificity. A preferred binding pair suitable for use as tag and probe is biotin and avidin.

The polypeptide is derived from the protein of interest, which in the present case is Vitronectin. By "derived from" is meant that the polypeptide is chosen, selected or prepared because it contains the amino acid sequence of a heparin-binding domain that is present in the protein of interest. The amino acid sequence of the heparin-binding domain may be modified from that appearing in the protein of interest, e.g. to investigate the effect of changes in the heparin-binding domain sequence on GAG binding.

In this specification the protein is Vitronectin. The amino acid sequences of the preferred heparin-binding domains is PRPSLAKKQRFRHRNRRKGYRSQRGHSRGRNQNSRR (SEQ ID NO:1) or PRPSLAKKQRFRHRNRKGYRSQRGHSRGRNQNSRR (SEQ ID NO:3).

It is understood by those skilled in the art that small variations in the amino acid sequence of a particular polypeptide may allow the inherent functionality of that portion to be maintained. It is also understood that the substitution of certain amino acid residues within a peptide with other amino acid residues that are isosteric and/or isoelectric may either maintain or improve certain properties of the unsubstituted peptide. These variations are also encompassed within the scope of the present invention. For example, the amino acid alanine may sometimes be substituted for the amino acid glycine (and vice versa) whilst maintaining one or more of the properties of the peptide. The term 'isosteric' refers to a spatial similarity between two entities. Two examples of moieties that are isosteric at moderately elevated temperatures are the iso-propyl and tert-butyl groups. The term 'isoelectronic' refers to an electronic similarity between two entities, an example being the case where two entities possess a functionality of the same, or similar, pKa.

The polypeptide corresponding to the heparin-binding domain may be synthetic or recombinant.

The solid support may be any substrate having a surface to which molecules may be attached, directly or indirectly, through either covalent or non-covalent bonds. The solid support may include any substrate material that is capable of providing physical support for the probes that are attached to the surface. It may be a matrix support. The material is generally capable of enduring conditions related to the attachment of the probes to the surface and any subsequent treatment, handling, or processing encountered during the performance of an assay. The materials may be naturally occurring, synthetic, or a modification of a naturally occurring material. The solid support may be a plastics material (including polymers such as, e.g., poly(vinyl chloride), cyclo-olefin copolymers, polyacrylamide, polyacrylate, polyethylene, polypropylene, poly(4-methylbutene), polystyrene, polymethacrylate, poly(ethylene terephthalate), polytetrafluoroethylene (PTFE or Teflon®), nylon, poly(vinyl butyrate)), etc., either used by themselves or in conjunction with other materials. Additional rigid materials may be considered, such as glass, which includes silica and further includes, for example, glass that is available as Bioglass. Other materials that may be employed include porous materials, such as, for example, controlled pore glass beads. Any other materials known in the art that are capable of having one or more functional groups, such as any of an amino, carboxyl, thiol, or hydroxyl functional group, for example, incorporated on its surface, are also contemplated.

Preferred solid supports include columns having a polypeptide immobilized on a surface of the column. The surface may be a wall of the column, and/or may be provided by beads packed into the central space of the column.

The polypeptide may be immobilised on the solid support. Examples of methods of immobilisation include: adsorption, covalent binding, entrapment and membrane confinement. In a preferred embodiment of the present invention the interaction between the polypeptide and the matrix is substantially permanent. In a further preferred embodiment of the present invention, the interaction between the peptide and the matrix is suitably inert to ion-exchange chromatography. In a preferred arrangement, the polypeptide is attached to the surface of the solid support. It is understood that a person skilled in the art would have a large array of options to choose from to chemically and/or physically attach two entities to each other. These options are all encompassed within the scope of the present invention. In a preferred arrangement, the polypeptide is adsorbed to a solid support through the interaction of biotin with streptavidin. In a representative example of this arrangement, a molecule of biotin is bonded covalently to the polypeptide, whereupon the biotin-polypeptide conjugate binds to streptavidin, which in turn has been covalently bonded to a solid support. In another arrangement, a spacer or linker moiety may be used to connect the molecule of biotin with the polypeptide, and/or the streptavidin with the matrix.

By contacting the GAG mixture with the solid support GAG-polypeptide complexes are allowed to form. These are partitioned from the remainder of the mixture by removing the remainder of the mixture from the solid support, e.g. by washing the solid support to elute non-bound materials. Where a column is used as the solid support non-binding components of the GAG mixture can be eluted from the column leaving the GAG-polypeptide complexes bound to the column.

It is understood that certain oligosaccharides may interact in a non-specific manner with the polypeptide. In certain embodiments, oligosaccharide which interacts with the polypeptide in a non-specific manner may be included in, or excluded from the mixture of compounds enriched with one or more GAGs that modulate the effect of a heparin-binding factor. An example of a non-specific interaction is the temporary confinement within a pocket of a suitably sized and/or shaped molecule. Further it is understood that these oligosaccharides may elute more slowly than those oligosaccharides that display no interaction with the peptide at all. Furthermore it is understood that the compounds that bind non-specifically may not require the input of the same external stimulus to make them elute as for those compounds that bind in a specific manner (for example through an ionic interaction). The inventors' methodology is capable of separating a mixture of oligosaccharides into those components of that mixture that: bind in a specific manner to the polypeptide; those that bind in a non-specific manner to the polypeptide; and those that do not bind to the polypeptide. These designations are defined operationally for each GAG-peptide pair.

By varying the conditions (e.g. salt concentration) present at the surface of the solid support where binding of the GAG and polypeptide occurs those GAGs having the highest affinity and/or specificity for the heparin-binding domain can be selected.

GAGs may accordingly be obtained that have a high binding affinity for a protein of interest and/or the heparin-binding domain of the protein of interest. The binding affinity ($K_d$) may be chosen from one of: less than $10 \mu M$, less than $1 \mu M$, less than 100nM, less than 10nM, less than 1nM, less than 100pM.

GAGs obtained by the methods described may be useful in a range of applications, *in vitro* and/or *in vivo.*

The GAGs may be provided as a formulation for such purposes. For example, culture media may be provided comprising a GAG obtained by the method described, i.e. comprising HS9.

Cells or tissues obtained from *in vitro* cell or tissue culture in the presence of HS9 may be collected and implanted into a human or animal patient in need of treatment. A method of implantation of cells and/or tissues may therefore be provided, the method comprising the steps of:

(a) culturing cells and/or tissues *in vitro* in contact with HS9;
(b) collecting the cells and/or tissues;
(c) implanting the cells and/or tissues into a human or animal subject in need of treatment.

The cells may be cultured in part (a) in contact with HS9 for a period of time sufficient to allow growth, proliferation or differentiation of the cells or tissues. For example, the period of time may be chosen from: at least 5 days, at least 10 days, at least 20 days, at least 30 days or at least 40 days.

In another embodiment the HS9 may be formulated for use in a method of medical treatment, including the prevention or treatment of disease. A pharmaceutical composition or medicament may be provided comprising HS9 and a pharmaceutically acceptable diluent, carrier or adjuvant. Such pharmaceutical compositions or medicaments may be provided for the prevention or treatment of disease. The use of HS9 in the manufacture of a medicament for the prevention or treatment of disease is also provided. Optionally, pharmaceutical compositions and medicaments according to the present invention may also contain the protein of interest (i.e. Vitronectin) having the heparin-binding domain to which the GAG binds.

Pharmaceutical compositions and medicaments according to the present invention may therefore comprise one of:

(a) HS9;
(b) HS9 in combination with a protein containing the heparin-binding domain bound by HS9 (e.g. SEQ ID NO:1 or SEQ ID NO:3);

In another aspect, the present invention provides a biological scaffold comprising HS9. In some embodiments, the biological scaffolds of the present invention may be used in orthopaedic, vascular, prosthetic, skin and corneal applications. The biological scaffolds provided by the present invention include extended-release drug delivery devices, tissue valves, tissue valve leaflets, drug-eluting stents, vascular grafts, and orthopaedic prostheses such as bone, ligament, tendon, cartilage and muscle. In a preferred embodiment of the present disclosure, the biological scaffold is a catheter wherein the inner (and/or outer) surface comprises one or more GAG compounds (including HS9) attached to the catheter.

The compounds of the present invention can be administered to a subject as a pharmaceutically acceptable salt thereof. For example, base salts of the compounds of the enriched mixtures of the present invention include, but are not limited to, those formed with pharmaceutically acceptable cations, such as sodium, potassium, lithium, calcium, magnesium, ammonium and alkylammonium. The present invention includes within its scope cationic salts, for example the sodium or potassium salts.

It will be appreciated that the compounds of the enriched mixtures of the present invention which bear a carboxylic acid group may be delivered in the form of an administrable prodrug, wherein the acid moiety is esterified (to have the form -CO2R'). The term "pro-drug" specifically relates to the conversion of the -OR' group to a -OH group, or carboxylate anion therefrom, *in vivo*. Accordingly, the prodrugs of the present invention may act to enhance drug adsorption and/or drug delivery into cells. The *in vivo* conversion of the prodrug may be facilitated either by cellular enzymes such as lipases and esterases or by chemical cleavage such as *in vivo* ester hydrolysis.

Medicaments and pharmaceutical compositions according to aspects of the present invention may be formulated for administration by a number of routes, including but not limited to, injection at the site of disease or injury. The medicaments and compositions may be formulated in fluid or solid form. Fluid formulations may be formulated for administration by injection to a selected region of the human or animal body.

Administration is preferably in a "therapeutically effective amount", this being sufficient to show benefit to the individual. The actual amount administered, and rate and time-course of administration, will depend on the nature and severity of the injury or disease being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins.

Stem cells

The stem cells cultured and described herein may be stem cells of any kind. They may be totipotent, pluripotent or multipotent. Pluripotent stem cells may be embryonic stem cells or induced pluripotent stem cells.

In this specification, by stem cell is meant any cell type that has the ability to divide (i.e. self-renew) and respectively remain totipotent, pluripotent or multipotent and give rise to specialized cells.

Stem cells cultured in the present disclosure may be obtained or derived from existing cultures or cell lines or directly from any adult, embryonic or fetal tissue, including blood, bone marrow, skin, epithelia or umbilical cord (a tissue that is normally discarded).

The multipotency of stem cells may be determined by use of suitable assays. Such assays may comprise detecting one or more markers of pluripotency, e.g. alkaline phosphatase activity, detection of RUNX2, osterix, collagen I, II, IV, VII, X, osteopontin, Osteocalcin, BSPII, SOX9, Aggrecan, ALBP, CCAAT/enhancer binding protein-a (C/EBP$\alpha$), adipocyte lipid binding protein (ALBP), alkaline phosphatase (ALP), bone sialoprotein 2, (BSPII), Collagen2a1 (Coll2a) and SOX9.

The stem cells according to the disclosure may be obtained from any animal or human, e.g. non-human animals, e.g. rabbit, guinea pig, rat, mouse or other rodent (including cells from any animal in the order Rodentia), cat, dog, pig, sheep, goat, cattle, horse, non-human primate or other non-human vertebrate organism; and/or non-human mammalian animals; and/or human. Preferably they are human. Optionally they are non-human. Optionally they are non-embryonic stem cells. Optionally they are not totipotent. Optionally they are not pluripotent.

In yet a further aspect of the present disclosure, a pharmaceutical composition comprising stem cells or other cells generated by any of the methods of the present disclosure, or fragments or products thereof, is provided. The pharmaceutical composition may be useful in a method of medical treatment. Suitable pharmaceutical compositions may further comprise a pharmaceutically acceptable carrier, adjuvant or diluent.

In another aspect of the present disclosure, stem cells or other cells generated by any of the methods of the present disclosure may be used in a method of medical treatment, preferably, a method of medical treatment is provided comprising administering to an individual in need of treatment a therapeutically effective amount of said medicament or pharmaceutical composition.

Stem cells and other cells obtained through culture methods and techniques according to this disclosure may be used to differentiate into another cell type for use in a method of medical treatment. Thus, the differentiated cell type may be derived from, and may be considered as a product of, a stem cell obtained by the culture methods and techniques described which has subsequently been permitted to differentiate. Pharmaceutical compositions may be provided comprising such differentiated cells, optionally together with a pharmaceutically acceptable carrier, adjuvant or diluent. Such pharmaceutical composition may be useful in a method of medical treatment.

Sources of Pluripotent Cells

Some aspects and embodiments of the present disclosure are concerned with the use of pluripotent cells. Embryonic stem cells and induced pluripotent stem cells are described as examples of such cells.

Embryonic stem cells have traditionally been derived from the inner cell mass (ICM) of blastocyst stage embryos (Evans, M.J., and Kaufman, M.H. (1981). Establishment in culture of pluripotential cells from mouse embryos. Nature 292, 154-156. Martin, G.R. (1981).

Isolation of a pluripotent cell line from early mouse embryos cultured in medium conditioned by teratocarcinoma stem cells. Proc. Natl. Acad. Sci. USA 78, 7634-7638. Thomson, J.A., Itskovitz-Eldor, J., Shapiro, S.S., Waknitz, M.A., Swiergiel, J.J., Marshall, V.S., and Jones, J.M. (1998). Embryonic stem cell lines derived from human blastocysts. Science 282, 1145-1147). In isolating embryonic stem cells these methods may cause the destruction of the embryo.

Several methods have now been provided for the isolation of pluripotent stem cells that do not lead to the destruction of an embryo, e.g. by transforming adult somatic cells or germ cells. These methods include:

1. Reprogramming by nuclear transfer. This technique involves the transfer of a nucleus from a somatic cell into an oocyte or zygote. In some situations this may lead to the creation of an animal-human hybrid cell. For example, cells may be created by the fusion of a human somatic cell with an animal oocyte or zygote or fusion of a human oocyte or zygote with an animal somatic cell.

2. Reprogramming by fusion with embryonic stem cells. This technique involves the fusion of a somatic cell with an embryonic stem cell. This technique may also lead to the creation of animal-human hybrid cells, as in 1 above.

3. Spontaneous re-programming by culture. This technique involves the generation of pluripotent cells from non-pluripotent cells after long term culture. For example, pluripotent embryonic germ (EG) cells have been generated by long-term culture of primordial germ cells (PGC) (Matsui et al., Derivation of pluripotential embryonic stem cells from murine primordial germ cells in culture. Cell 70, 841-847, 1992). The development of pluripotent stem cells after prolonged culture of bone marrow-derived cells has also been reported (Jiang et al., Pluripotency of mesenchymal stem cells derived from adult marrow. Nature 418, 41-49, 2002). They designated these cells multipotent adult progenitor cells (MAPCs). Shinohara et al also demonstrated that pluripotent stem cells can be generated during the course of culture of germline stem (GS) cells from neonate mouse testes, which they designated multipotent germline stem (mGS) cells (Kanatsu-Shinohara et al., Generation of pluripotent stem cells from neonatal mouse testis. Cell 119, 1001-1012, 2004).

4. Reprogramming by defined factors. For example the generation of iPS cells by the retrovirus-mediated introduction of transcription factors (such as Oct-3/4, Sox2, c-Myc, and KLF4) into mouse embryonic or adult fibroblasts, e.g. as described above. Kaji et al (Virus-free induction of pluripotency and subsequent excision of reprogramming factors. Nature. Online publication 1 March 2009) also describe the non-viral transfection of a single multiprotein expression vector, which comprises the coding sequences of c-*Myc*, *Klf4*, *Oct4* and Sox2 linked with 2A peptides, that can reprogram both mouse and human fibroblasts. iPS cells produced with this non-viral vector show robust expression of pluripotency markers, indicating a reprogrammed state confirmed functionally by *in vitro* differentiation assays and formation of adult chimaeric mice. They succeeded in establishing reprogrammed human cell lines from embryonic fibroblasts with robust expression of pluripotency markers.

Methods 1-4 are described and discussed by Shinya Yamanaka in Strategies and New Developments in the Generation of Patient-Specific Pluripotent Stem Cells (Cell Stem Cell 1, July 2007 a2007 Elsevier Inc).

5. Derivation of hESC lines from single blastomeres or biopsied blastomeres. See Klimanskaya I, Chung Y, Becker S, Lu SJ, Lanza R. Human embryonic stem cell lines derived from single blastomeres. Nature 2006; 444:512, Lei et al Xeno-free derivation and culture of human embryonic stem cells: current status, problems and challenges. Cell Research (2007) 17:682-688, Chung Y, Klimanskaya I, Becker S, et al. Embryonic and extraembryonic stem cell lines derived from single mouse blastomeres. Nature. 2006;439:216-219. Klimanskaya I, Chung Y, Becker S, et al. Human embryonic stem cell lines derived from single blastomeres. Nature. 2006;444:481-485. Chung Y, Klimanskaya I, Becker S, et al. Human embryonic stem cell lines generated without embryo destruction. Cell Stem Cell. 2008;2:113-117 and Dusko Ilic et al (Derivation of human embryonic stem cell lines from biopsied blastomeres dn human feeders with a minimal exposure to xenomaterials. Stem Cells And Development - paper in pre-publication),

6. hESC lines obtained from arrested embryos which stopped cleavage and failed to develop to morula and blastocysts *in vitro.* See Zhang X, Stojkovic P, Przyborski S, et al. Derivation of human embryonic stem cells from developing and arrested embryos. Stem Cells 2006; 24:2669-2676 and Lei et al Xeno-free derivation and culture of human embryonic stem cells: current status, problems and challenges. Cell Research (2007) 17:682-688,

7. Parthogenesis (or Parthenogenesis). This technique involves chemical or electrical stimulation of an unfertilised egg so as to cause it to develop into a blastomere from which embryonic stem cells may be derived. For example, see Lin et al. Multilineage potential of homozygous stem cells derived from metaphase II oocytes. Stem Cells. 2003;21(2):152-61 who employed the chemical activation of nonfertilized metaphase II oocytes to produce stem cells.

8. Stem cells of fetal origin. These cells lie between embryonic and adult stem cells in terms of potentiality and may be used to derive pluripotent or multipotent cells. Human umbilical-cord-derived fetal mesenchymal stem cells (UC fMSCs) expressing markers of pluripotency (including Nanog, Oct-4, Sox-2, Rex-1, SSEA-3, SSEA-4, Tra-1-60, and Tra-1-81, minimal evidence of senescence as shown by β-galactosidase staining, and the consistent expression of telomerase activity) have been successfully derived by Chris H. Jo et al (Fetal mesenchymal stem cells derived from human umbilical cord sustain primitive characteristics during extensive expansion. Cell Tissue Res (2008) 334:423-433). Winston Costa Pereira et al (Reproducible methodology for the isolation of mesenchymal stem cells from human umbilical cord and its potential for cardiomyocyte generation J Tissue Eng Regen Med 2008; 2: 394-399. isolated a pure population of mesenchymal stem cells from Wharton's jelly of the human umbilical cord. Mesenchymal stem cells derived from Wharton's jelly are also reviewed in Troyer & Weiss (Concise Review: Wharton's Jelly-Derived Cells Are a primitive Stromal Cell Population. Stem Cells 2008:26:591-599). Kim et al (Ex vivo characteristics of human amniotic membrane-derived stem cells. Cloning Stem Cells 2007 Winter;9(4):581-94) succeeded in isolating human amniotic membrane-derived mesenchymal cells from human amniotic membranes. Umbilical cord is a tissue that is normally discarded and stem cells derived from this tissue have tended not to attract moral or ethical objection.

9. Chung et al. [(2008) Human Embryonic Stem Cell Lines Generated without Embryo Destruction. Cell Stem Cell. 2(2) 113-117. Epub 2008 Jan 10] describes the generation of human embryonic setm cell lines with the destruction of an embryo.

Induced pluripotent stem cells have the advantage that they can be obtained by a method that does not cause the destruction of an embryo, more particularly by a method that does not cause the destruction of a human or mammalian embryo. The method described by Chung et al (item 9 above) also permits obtaining of human embryonic stem cells by a method that does not cause the destruction of a human embryo.

The present disclosure includes the use of pluripotent or multipotent stem cells obtained from any of these sources or created by any of these methods. In some embodiments, the pluripotent or multipotent cells used in the methods of the present invention have been obtained by a method that does not cause the destruction of an embryo. More preferably in some embodiments, the pluripotent or multipotent cells used in the methods of the present invention have been obtained by a method that does not cause the destruction of a human or mammalian embryo. As such, methods of the invention may be performed using cells that have not been prepared exclusively by a method which necessarily involves the destruction of human embryos from which those cells may be derived. This optional limitation is specifically intended to take account of Decision G0002/06 of 25 November 2008 of the Enlarged Board of Appeal of the European Patent Office.

Induced Pluripotent Stem Cells

The methods and compositions described here may be used for the propagation of induced pluripotent stem cells.

Induced pluripotent stem cells, commonly abbreviated as iPS cells or iPSCs, are a type of pluripotent stem cell artificially derived from a non-pluripotent cell, typically an adult somatic cell, by inserting certain genes. iPS cells are reviewed and discussed in Takahashi, K. & Yamanaka (2006), Yamanaka S, et. al. (2007), Wernig M, et. al. (2007), Maherali N, et. al. (2007) and Thomson JA, Yu J, et al. (2007) and Takahashi et al., (2007).

iPS cells are typically derived by transfection of certain stem cell-associated genes into non-pluripotent cells, such as adult fibroblasts. Transfection is typically achieved through viral vectors, such as retroviruses. Transfected genes include the master transcriptional regulators Oct-3/4 (Pouf51) and Sox2, although it is suggested that other genes enhance the efficiency of induction. After 3-4 weeks, small numbers of transfected cells begin to become morphologically and biochemically similar to pluripotent stem cells, and are typically isolated through morphological selection, doubling time, or through a reporter gene and antibiotic infection.

Maintenance of Stem Cell Characteristics

Propagated stem cells may retain at least one characteristic of a parent stem cell. The stem cells may retain the characteristic after one or more passages. They may do so after a plurality of passages.

The characteristic may comprise a morphological characteristic, immunohistochemical characteristic, a molecular biological characteristic, etc. The characteristic may comprise a biological activity.

*Stem Cell Characteristics*

The stem cells propagated by our methods may display any of the following stem cell characteristics.

Stem cells may display increased expression of Oct4 and/or SSEA-1. Expression of any one or more of Flk-1, Tie-2 and c-kit may be decreased. Stem cells which are self-renewing may display a shortened cell cycle compared to stem cells which are not self-renewing.

Stem cells may display defined morphology. For example, in the two dimensions of a standard microscopic image, human embryonic stem cells display high nuclear/cytoplasmic ratios in the plane of the image, prominent nucleoli, and compact colony formation with poorly discernable cell junctions.

Stem cells may also be characterized by expressed cell markers as described in further detail below.

*Expression of Pluripotency Markers*

The biological activity that is retained may comprise expression of one or more pluripotency markers.

Stage-specific embryonic antigens (SSEA) are characteristic of certain embryonic cell types. Antibodies for SSEA markers are available from the Developmental Studies Hybridoma Bank (Bethesda Md.). Other useful markers are detectable using antibodies designated Tra-1-60 and Tra-1-81 (Andrews et al., Cell Linesfrom Human Germ Cell Tumors, in E. J. Robertson, 1987, supra). Human embryonic stem cells are typically SSEA-1 negative and SSEA-4 positive. hEG cells are typically SSEA-1 positive. Differentiation of pPS cells in vitro results in the loss of SSEA-4, Tra-1-60, and Tra-1-81 expression and increased expression of SSEA-1. pPS cells can also be characterized by the presence of alkaline phosphatase activity, which can be detected by fixing the cells with 4% paraformaldehyde, and then developing with Vector Red as a substrate, as described by the manufacturer (Vector Laboratories, Burlingame Calif.).

Embryonic stem cells are also typically telomerase positive and OCT-4 positive. Telomerase activity can be determined using TRAP activity assay (Kim et al., Science 266:2011, 1997), using a commercially available kit (TRAPeze.RTM. XK Telomerase Detection Kit, Cat. s7707; Intergen Co., Purchase N.Y.; or TeloTAGGG.TM. Telomerase PCR ELISA plus, Cat. 2,013,89; Roche Diagnostics, Indianapolis). hTERT expression can also be evaluated at the mRNA level by RT-PCR. The LightCycler TeloTAGGG.TM. hTERT quantification kit (Cat. 3,012,344; Roche Diagnostics) is available commercially for research purposes.

Any one or more of these pluripotency markers, including FOXD3, PODXL, alkaline phosphatase, OCT-4, SSEA-4 and TRA-1-60, etc, may be retained by the propagated stem cells.

Detection of markers may be achieved through any means known in the art, for example immunologically. Histochemical staining, flow cytometry (FACs), Western Blot, enzyme-linked immunoassay (ELISA), etc may be used.

Flow immunocytochemistry may be used to detect cell-surface markers. immunohistochemistry (for example, of fixed cells or tissue sections) may be used for intracellular or cell-surface markers. Western blot analysis may be conducted on cellular extracts. Enzyme-linked immunoassay may be used for cellular extracts or products secreted into the medium.

For this purpose, antibodies to the pluripotency markers as available from commercial sources may be used.

Antibodies for the identification of stem cell markers including the Stage-Specific Embryonic Antigens 1 and 4 (SSEA-1 and SSEA-4) and Tumor Rejection Antigen 1-60 and 1-81 (TRA-1-60, TRA-1-81) may be obtained commercially, for example from Chemicon International, Inc (Temecula, CA, USA). The immunological detection of these antigens using monoclonal antibodies has been widely used to characterize pluripotent stem cells (Shamblott M.J. et. al. (1998) PNAS 95: 13726-13731; Schuldiner M. et. al. (2000). PNAS 97: 11307 - 11312; Thomson J.A. et. al. (1998). Science 282: 1145-1147; Reubinoff B.E. et. al. (2000). Nature Biotechnology 18: 399-404; Henderson J.K. et. al. (2002). Stem Cells 20: 329-337; Pera M. et. al. (2000). J. Cell Science 113: 5-10.).

The expression of tissue-specific gene products can also be detected at the mRNA level by Northern blot analysis, dot-blot hybridization analysis, or by reverse transcriptase initiated polymerase chain reaction (RT-PCR) using sequence-specific primers in standard amplification methods. Sequence data for the particular markers listed in this disclosure can be obtained from public databases such as GenBank . See U.S. Pat. No. 5,843,780 for further details.

Substantially all of the propagated cells, or a substantial portion of them, may express the marker(s). For example, the percentage of cells that express the marker or markers may be 50% or more, 60% or more, 70% or more, 80% or more, 90% or more, 93% or more, 95% or more, 97% or more, 98% or more, 99% or more, or substantially 100%.

*Cell Viability*

The biological activity may comprise cell viability after the stated number of passages. Cell viability may be assayed in various ways, for example by Trypan Blue exclusion. A protocol for vital staining follows. Place a suitable volume of a cell suspension (20-200 $\mu$L) in appropriate tube add an equal volume of 0.4% Trypan blue and gently mix, let stand for 5 minutes at room temperature. Place 10 $\mu$l of stained cells in a hemocytometer and count the number of viable (unstained) and dead (stained) cells. Calculate the average number of unstained cells in each quadrant, and multiply by $2 \times 10^4$ to find cells/ml. The percentage of viable cells is the number of viable cells divided by the number of dead and viable cells.

The viability of cells may be 50% or more, 60% or more, 70% or more, 80% or more, 90% or more, 93% or more, 95% or more, 97% or more, 98% or more, 99% or more, or substantially 100%.

*Karyotype*

The propagated stem cells may retain a normal karyotype during or after propagation. A "normal" karyotype is a karyotype that is identical, similar or substantially similar to a karyotype of a parent stem cell from which the propagule is derived, or one which varies from it but not in any substantial manner. For example, there should not be any gross anomalies such as translocations, loss of chromosomes, deletions, etc.

Karyotype may be assessed by a number of methods, for example visually under optical microscopy. Karyotypes may be prepared and analyzed as described in McWhir et al. (2006), Hewitt et al. (2007), and Gallimore and Richardson (1973). Cells may also be karyotyped using a standard G-banding technique (available at many clinical diagnostics labs that provides routine karyotyping services, such as the Cytogenetics Lab at Oakland Calif.) and compared to published stem cell karyotypes.

All or a substantial portion of propagated cells may retain a normal karyotype. This proportion may be 50% or more, 60% or more, 70% or more, 80% or more, 90% or more, 93% or more, 95% or more, 97% or more, 98% or more, 99% or more, or substantially 100%.

*Pluripotency*

The propagated stem cells may retain the capacity to differentiate into all three cellular lineages, i.e., endoderm, ectoderm and mesoderm. Methods of induction of stem cells to differentiate each of these lineages are known in the art and may be used to assay the capability of the propagated stem cells. All or a substantial portion of propagated cells may retain this ability. This may be 50% or more, 60% or more, 70% or more, 80% or more, 90% or more, 93% or more, 95% or more, 97% or more, 98% or more, 99% or more, or substantially 100% of the propagated stem cells.

Glycosaminglycans

As used herein, the terms 'glycosaminoglycan' and 'GAG' are used interchangeably and are understood to refer to the large collection of molecules comprising an oligosaccharide, wherein one or more of those conjoined saccharides possess an amino substituent, or a derivative thereof. Examples of GAGs are chondroitin sulfate, keratan sulfate, heparin, dermatan sulfate, hyaluronate and heparan sulfate.

As used herein, the term 'GAG' also extends to encompass those molecules that are GAG conjugates. An example of a GAG conjugate is a proteoglycosaminoglycan (PGAG, proteoglycan) wherein a peptide component is covalently bound to an oligosaccharide component.

Heparan sulphate (HS)

Heparan sulfate proteoglycans (HSPGs) represent a highly diverse subgroup of proteoglycans and are composed of heparan sulfate glycosaminoglycan side chains covalently attached to a protein backbone. The core protein exists in three major forms: a secreted form known as perlecan, a form anchored in the plasma membrane known as glypican, and a transmembrane form known as syndecan. They are ubiquitous constituents of mammalian cell surfaces and most extracellular matrices. There are other proteins such as agrin, or the amyloid precursor protein, in which an HS chain may be attached to less commonly found cores.

"Heparan Sulphate" ("Heparan sulfate" or "HS") is initially synthesised in the Golgi apparatus as polysaccharides consisting of tandem repeats of D-glucuronic acid (GlcA) and N-acetyl-D-glucosamine (GlcNAc). The nascent polysaccharides may be subsequently modified in a series of steps: N-deacetylation/N-sulfation of GlcNAc, C5 epimerisation of GlcA to iduronic acid (IdoA), O-sulphation at C2 of IdoA and GlcA, O-sulphation at C6 of N-sulphoglucosamine (GlcNS) and occasional O-sulphation at C3 of GlcNS. N-deacetylation/N-sulphation, 2-O-, 6-O- and 3-O-sulphation of HS are mediated by the specific action of HS N-deacetylase/N-sulfotransferase (HSNDST), HS 2-O-sulfotransferase (HS2ST), HS 6-O-sulfotransferase (HS6ST) and HS 3-O-sulfotransferase, respectively. At each of the modification steps, only a fraction of the potential substrates are modified, resulting in considerable sequence diversity. This structural complexity of HS has made it difficult to determine its sequence and to understand the relationship between HS structure and function.

Heparan sulfate side chains consist of alternately arranged D-glucuronic acid or L-iduronic acid and D-glucosamine, linked via (1 -> 4) glycosidic bonds. The glucosamine is often N-acetylated or N-sulfated and both the uronic acid and the glucosamine may be additionally O-sulfated. The specificity of a particular HSPG for a particular binding partner is created by the specific pattern of carboxyl, acetyl and sulfate groups attached to the glucosamine and the uronic acid. In contrast to heparin, heparan sulfate contains less N- and O-sulfate groups and more N-acetyl groups. The heparan sulfate side chains are linked to a serine residue of the core protein through a tetrasaccharide linkage (-glucuronosyl-$\beta$-(1→3)-galactosyl-$\beta$-(1→3)-galactosyl-$\beta$-(1→4)-xylosyl-$\beta$-1-O-(Serine)) region.

Both heparan sulfate chains and core protein may undergo a series of modifications that may ultimately influence their biological activity. Complexity of HS has been considered to surpass that of nucleic acids (Lindahl et al, 1998, J. Biol. Chem. 273, 24979; Sugahara and Kitagawa, 2000, Curr. Opin. Struct. Biol. 10, 518). Variation in HS species arises from the synthesis of non-random, highly sulfated sequences of sugar residues which are separated by unsulfated regions of disaccharides containing N- acetylated glucosamine. The initial conversion of N-acetylglucosamine to N-sulfoglucosamine creates a focus for other modifications, including epimerization of glucuronic acid to iduronic acid and a complex pattern of O-sulfations on glucosamine or iduronic acids. In addition, within the non-modified, low sulfated, N-acetylated sequences, the hexuronate residues remain as glucuronate, whereas in the highly sulfated N-sulfated regions, the C-5 epimer iduronate predominates. This limits the number of potential disaccharide variants possible in any given chain but not the abundance of each. Most modifications occur in the N-sulfated domains, or directly adjacent to them, so that in the mature chain there are regions of high sulfation separated by domains of low sulfation (Brickman et al. (1998), J. Biol. Chem. 273(8), 4350 -4359).

It is hypothesized that the highly variable heparan sulfate chains play key roles in the modulation of the action of a large number of extracellular ligands, including regulation and presentation of growth and adhesion factors to the cell, via a complicated combination of autocrine, juxtacrine and paracrine feedback loops, so controlling intracellular signaling and thereby the differentiation of stem cells. For example, even though heparan sulfate glycosaminoglycans may be genetically described (Alberts et al. (1989) Garland Publishing, Inc, New York & London, pp. 804 and 805), heparan sulfate glycosaminoglycan species isolated from a single source may differ in biological activity. As shown in Brickman et al, 1998, Glycobiology 8, 463, two separate pools of heparan sulfate glycosaminoglycans obtained from neuroepithelial cells could specifically activate either FGF-1 or FGF-2, depending on mitogenic status. Similarly, the capability of a heparan sulfate (HS) to interact with either FGF-1 or FGF-2 is described in WO 96/23003. According to this patent application, a respective HS capable of interacting with FGF-1 is obtainable from murine cells at embryonic day from about 11 to about 13, whereas a HS capable of interacting with FGF-2 is obtainable at embryonic day from about 8 to about 10.

As stated above HS structure is highly complex and variable between HS. Indeed, the variation in HS structure is considered to play an important part in contributing toward the different activity of each HS in promoting cell growth and directing cell differentiation. The structural complexity is considered to surpass that of nucleic acids and although HS structure may be characterised as a sequence of repeating disaccharide units having specific and unique sulfation patterns at the present time no standard sequencing technique equivalent to those available for nucleic acid sequencing is available for determining HS sequence structure. In the absence of simple methods for determining a definitive HS sequence structure HS molecules are positively identified and structurally characterised by skilled workers in the field by a number of analytical techniques. These include one or a combination of disaccharide analysis, tetrasaccharide analysis, HPLC, capillary electrophoresis and molecular weight determination. These analytical techniques are well known to and used by those of skill in the art.

Two techniques for production of di- and tetra- saccharides from HS include nitrous acid digestion and lyase digestion. A description of one way of performing these digestion techniques is provided below, purely by way of example, such description not limiting the scope of the present invention.

*Nitrous acid digestion*

Nitrous acid based depolymerisation of heparan sulphate leads to the eventual degradation of the carbohydrate chain into its individual disaccharide components when taken to completion.

For example, nitrous acid may be prepared by chilling 250 $\mu$l of 0.5 M $H_2SO_4$ and 0.5 M $Ba(NO_2)_2$ separately on ice for 15 min. After cooling, the $Ba(NO_2)_2$ is combined with the $H_2SO_4$ and vortexed before being centrifuged to remove the barium sulphate precipitate. 125 $\mu$l of $HNO_2$ was added to GAG samples resuspended in 20 $\mu$l of $H_2O$, and vortexed before being incubated for 15 min at 25°C with occasional mixing. After incubation, 1 M $Na_2CO_2$ was added to the sample to bring it to pH 6. Next, 100 $\mu$l of 0.25 M $NaBH_4$ in 0.1 M NaOH is added to the sample and the mixture heated to 50°C for 20 min. The mixture is then cooled to 25°C and acidified glacial acetic acid added to bring the sample to pH 3. The mixture is then neutralised with 10 M NaOH and the volume decreased by freeze drying. Final samples are run on a Bio-Gel P-2 column to separate di- and tetrasaccharides to verify the degree of degradation.

*Lyase digestion*

Heparinise III cleaves sugar chains at glucuronidic linkages. The series of Heparinase enzymes (I, II and III) each display relatively specific activity by depolymerising certain heparan sulphate sequences at particular sulfation recognition sites. Heparinase I cleaves HS chains with NS regions along the HS chain. This leads to disruption of the sulphated domains. Heparinase III depolymerises HS with the NA domains, resulting in the separation of the carbohydrate chain into individual sulphated domains. Heparinase II primarily cleaves in the NA/NS "shoulder" domains of HS chains, where varying sulfation patterns are found. Note: The repeating disaccharide backbone of the heparan polymer is a uronic acid connected to the amino sugar glucosamine. "NS" means the amino sugar is carrying a sulfate on the amino group enabling sulfation of other groups at C2, C6 and C3. "NA" indicates that the amino group is not sulphated and remains acetylated.

For example, for depolymerisation in the NA regions using Heparinase III both enzyme and lyophilised HS samples are prepared in a buffer containing 20 mM Tris-HCL, 0.1 mg/ml BSA and 4 mM $CaCl_2$ at pH 7.5. Purely by way of example, Heparinase III may be added at 5 mU per 1$\mu$g of HS and incubated at 37°C for 16 h before stopping the reaction by heating to 70°C for 5 min.

Di- and tetrasaccharides may be eluted by column chromatography.

Biomaterials

Pharmaceutical compositions and medicaments of the invention may take the form of a biomaterial that is coated and/or impregnated with HS9. An implant or prosthesis may be formed from the biomaterial. Such implants or prostheses may be surgically implanted to assist in transplantion of cells.

HS9 may be applied to implants or prostheses to accelerate new tissue formation at a desired location. It will be appreciated that heparan sulphates, unlike proteins, are particularly robust and have a much better ability to withstand the solvents required for the manufacture of synthetic bioscaffolds and application to implants and prostheses.

The biomaterial may be coated or impregnated with HS9. Impregnation may comprise forming the biomaterial by mixing HS9 with the constitutive components of the biomaterial, e.g. during polymerisation, or absorbing HS9 into the biomaterial. Coating may comprise adsorbing the HS9 onto the surface of the biomaterial.

The biomaterial should allow the coated or impregnated HS9 to be released from the biomaterial when administered to or implanted in the subject. Biomaterial release kinetics may be altered by altering the structure, e.g. porosity, of the biomaterial.

In addition to coating or impregnating a biomaterial with HS9, one or more biologically active molecules may be impregnated or coated on the biomaterial. For example, at least one chosen from the group consisting of: BMP-2, BMP-4, OP-1, FGF-1, FGF-2, TGF-β1, TGF-β2, TGF-β3; VEGF; collagen; laminin; fibronectin; vitronectin. In addition or alternatively to the above bioactive molecules, one or more bisphosphonates may be impregnated or coated onto the biomaterial along with HS9. Examples of useful bisphosphonates may include at least one chosen from the group consisting of: etidronate; clodronate; alendronate; pamidronate; risedronate; zoledronate.

The biomaterial provides a scaffold or matrix support. The biomaterial may be suitable for implantation in tissue, or may be suitable for administration (e.g. as microcapsules in solution).

The implant or prosthesis should be biocompatible, e.g. non-toxic and of low immunogenicity (most preferably non-immunogenic). The biomaterial may be biodegradable such that the biomaterial degrades. Alternatively a non-biodegradable biomaterial may be used with surgical removal of the biomaterial being an optional requirement.

Biomaterials may be soft and/or flexible, e.g. hydrogels, fibrin web or mesh, or collagen sponges. A "hydrogel" is a substance formed when an organic polymer, which can be natural or synthetic, is set or solidified to create a three-dimensional open-lattice structure that entraps molecules of water or other solutions to form a gel. Solidification can occur by aggregation, coagulation, hydrophobic interactions or cross-linking.

Alternatively biomaterials may be relatively rigid structures, e.g. formed from solid materials such as plastics or biologically inert metals such as titanium.

The biomaterial may have a porous matrix structure which may be provided by a crosslinked polymer. The matrix is preferably permeable to nutrients and growth factors required for bone growth.

Matrix structures may be formed by crosslinking fibres, e.g. fibrin or collagen, or of liquid films of sodium alginate, chitosan, or other polysaccharides with suitable crosslinkers, e.g. calcium salts, polyacrylic acid, heparin. Alternatively scaffolds may be formed as a gel, fabricated by collagen or alginates, crosslinked using well established methods known to those skilled in the art.

Suitable polymer materials for matrix formation include, but are not limited by, biodegradable/bioresorbable polymers which may be chosen from the group of: agarose, collagen, fibrin, chitosan, polycaprolactone, poly(DL-lactide-co-caprolactone), poly(L-lactide-co-caprolactone-co-glycolide), polyglycolide, polylactide, polyhydroxyalcanoates, co-polymers thereof, or non-biodegradable polymers which may be chosen from the group of: cellulose acetate; cellulose butyrate, alginate, polysulfone, polyurethane, polyacrylonitrile, sulfonated polysulfone, polyamide, polyacrylonitrile, polymethylmethacrylate, co-polymers thereof.

Collagen is a promising material for matrix construction owing to its biocompatibility and favourable property of supporting cell attachment and function (U.S. Pat. No. 5,019,087; Tanaka, S.; Takigawa, T.; Ichihara, S. & Nakamura, T. Mechanical properties of the bioabsorbable polyglycolic acid-collagen nerve guide tube Polymer Engineering & Science 2006, 46, 1461-1467). Clinically acceptable collagen sponges are one example of a matrix and are well known in the art (e.g. from Integra Life Sciences).

Fibrin scaffolds (e.g. fibrin glue) provide an alternative matrix material. Fibrin glue enjoys widespread clinical application as a wound sealant, a reservoir to deliver growth factors and as an aid in the placement and securing of biological implants (Rajesh Vasita, Dhirendra S Katti. Growth factor delivery systems for tissue engineering: a materials perspective. Expert Reviews in Medical Devices. 2006; 3(1): 29-47; Wong C, Inman E, Spaethe R, Helgerson S. Thromb.Haemost. 2003 89(3): 573-582; Pandit AS, Wilson DJ, Feldman DS. Fibrin scaffold as an effective vehicle for the delivery of acidic growth factor (FGF-1). J. Biomaterials Applications. 2000; 14(3); 229-242; DeBlois Cote MF. Doillon CJ. Heparin-fibroblast growth factor fibrin complex: in vitro and in vivo applications to collagen based materials. Biomaterials. 1994; 15(9): 665-672.).

Luong-Van et al (In vitro biocompatibility and bioactivity of microencapsulated heparan sulphate Biomaterials 28 (2007) 2127-2136) describes prolonged localised delivery of HS from polycaprolactone microcapsules.

A further example of a biomaterial is a polymer that incorporates hydroxyapatite or hyaluronic acid.

Other suitable biomaterials include ceramic or metal (e.g. titanium), hydroxyapatite, tricalcium phosphate, demineralised bone matrix (DBM), autografts (i.e. grafts derived from the patient's tissue), or allografts (grafts derived from the tissue of an animal that is not the patient). Biomaterials may be synthetic (e.g. metal, fibrin, ceramic) or biological (e.g. carrier materials made from animal tissue, e.g. non-human mammals (e.g. cow, pig), or human).

The biomaterial can be supplemented with additional cells. For example, one can "seed" the biomaterial (or co-synthesise it) with stem cells.

In one aspect the biomaterial may comprise be coated or impregnated with HS9, and further comprise vitronection (e.g. as a further coating or impregnated component) and cells, e.g. stem cells, adhered to the biomaterial.

The subject to be treated may be any animal or human. The subject is preferably mammalian, more preferably human. The subject may be a non-human mammal (e.g. rabbit, guinea pig, rat, mouse or other rodent (including cells from any animal in the order Rodentia), cat, dog, pig, sheep, goat, cattle (including cows, e.g. dairy cows, or any animal in the order *Bos*), horse (including any animal in the order *Equidae*), donkey, and non-human primate). The non-human mammal may be a domestic pet, or animal kept for commercial purposes, e.g. a race horse, or farming livestock such as pigs, sheep or cattle. The subject may be male or female. The subject may be a patient.

Methods according to the present disclosure may be performed *in vitro* or *in vivo,* as indicated. The term "*in vitro*" is intended to encompass procedures with cells in culture whereas the term "*in vivo*" is intended to encompass procedures with intact multi-cellular organisms.

Culture Media

Culture media comprising HS9 (preferably isolated HS9) may be of any kind but is preferably liquid or gel and may contain other nutrients and growth factors (e.g. Vitronectin). Culture media may be prepared in dried form, e.g. powered form, for reconstitution in to liquid or gel. HS9 will preferably be present in non-trace amounts. For example, the concentration of HS9 in the culture media may range between about 1 ng/ml culture media to about 1000 ng/ml culture media. Preferably, the concentration of HS9 in the culture media is about 500 ng/ml or less, more preferably one of 250 ng/ml or less, 100 ng/ml or less, 90 ng/ml or less, 80 ng/ml or less, 70 ng/ml or less, 60 ng/ml or less, 50 ng/ml or less, 40 ng/ml or less, 30 ng/ml or less, 20 ng/ml or less, 10 ng/ml or less, or 5 ng/ml or less.

Cell Culture Substrate

The inventors' methodology allows for the isolation of an HS that binds to any selected protein. This enables the isolation an HS that binds to proteins useful as cell culture substrates, such as mammalian or human extracellular matrix proteins.

As such, a cell culture substrate may be provided, the substrate comprising an isolated HS that binds a peptide or protein of interest, e.g. an extracellular matrix protein, or Vitronectin. The HS may be HS9. The HS may be in contact with a cell culture support surface, which may be in the form of a culture dish, plate, bottle, flask, sheet, tissue culture plastic, tissue culture polystyrene or other conventional cell culture support material, article or container. A cell culture support surface may also be provided as a three-dimensional scaffold or matrix formed from a material capable of supporting cell culture and/or from a biomaterial, as described herein. The cell culture support may form all or part of an implant or prosthesis as described herein.

As such a cell culture article or container may be provided in which at least a part of the cell culture surface is coated in the isolated HS. The HS may be covalently bonded to the culture support surface or non-covalently in contact with the support surface. In some embodiments the culture support surface may have been treated by allylamine plasma polymerisation prior to coating with the HS.

In some embodiments the substrate further comprises the extracellular matrix protein or Vitronectin, preferably in contact with the HS. The substrate may be formed by a layer of HS, which may be in contact with comprises the extracellular matrix protein or Vitronectin. The the extracellular matrix protein or Vitronectin may be provided as an adjacent layer. In some embodiments the HS is bound to the extracellular matrix protein or Vitronectin. A method of forming a cell culture substrate is provided, comprising the steps of applying the HS to a cell culture support surface. The HS may be coated onto the support surface, e.g. by painting, spraying or pouring HS onto the support surface. In some embodiments prior to applying HS to the support surface the support surface is treated to facilitate or enhance attachment of HS to the support surface. Such treatment may involve plasma treatment, e.g. plasma polymerisation or allylamine plasma polymerisation, or chemical treatment.

Culture of Cells on Cell Culture Substrate

The cell culture substrate described herein may be used in methods of culturing cells, e.g. stem cells.

As such, a cell culture is provided comprising cells in *in vitro* culture, wherein the cells are in contact with a cell culture substrate, as described herein.

A method of culturing cells, e.g. stem cells, is also provided. The method comprising culturing cells *in vitro* in contact with a cell culture substrate, as described herein.

Dosages of Heparan Sulphate

In both *in vitro* and *in vivo* uses, HS9 may be used in concentrations or dosages of about 500 ng/ml or less, more preferably one of 250 ng/ml or less, 100 ng/ml or less, 90 ng/ml or less, 80 ng/ml or less, 70 ng/ml or less, 60 ng/ml or less, 50 ng/ml or less, 40 ng/ml or less, 30 ng/ml or less, 20 ng/ml or less, 10 ng/ml or less, 5 ng/ml or less; or of about 100 mg or less, 50 mg or less, 40 mg or less, 30 mg or less, 20 mg or less, 10 mg or less, 5 mg or less, 4 mg or less, 3 mg or less, 2 mg or less, or 1 mg or less; or about between 0.3-5$\mu$g/ml, 0.3-4, 0.3-3, 0.3-2.5, 0.3-2, 0.3-1.5, 0.3-1.0, 0.3-0.9, 0.3-0.8, 0.3-0.7, 0.3-0.6, 0.3-0.5, 0.3-0.4, 1-2, 1-1.75, 1-1.5, 1-1.25, 1.25-2, 1.5-2, or 1.75-2 $\mu$g/ml.

Vitronectin

Vitronectin is a glycoprotein found in the extracellular matrix.

The amino acid sequence of Vitronectin from Homo sapiens (SEQ ID NO:2) is shown below (the heparin binding domain of SEQ ID NO:1 and SEQ ID NO:3 is underlined). This sequence is available in Genbank under Accession no. AAH05046.1 GI:13477169.

```
  1     MAPLRPLLIL  ALLAWVALAD  QESCKGRCTE  GFNVDKKCQC  DELCSYYQSC  CTDYTAECKP



 61     QVTRGDVFTM  PEDEYTVYDD  GEEKNNATVH  EQVGGPSLTS  DLQAQSKGNP  EQTPVLKPEE
121     EAPAPEVGAS  KPEGIDSRPE  TLHPGRPQPP  AEEELCSGKP  FDAFTDLKNG  SLFAFRGQYC
181     YELDEKAVRP  GYPKLIRDVW  GIEGPIDAAF  TRINCQGKTY  LFKGSQYWRF  EDGVLDPDYP
241     RNISDGFDGI  PDNVDAALAL  PAHSYSGRER  VYFFKGKQYW  EYQFQHQPSQ  EECEGSSLSA
301     VFEHFAMMQR  DSWEDIFELL  FWGRTSAGTR  QPQFISRDWH  GVPGQVDAAM  AGRIYISGMA
361     PRPSLAKKQR  FRHRNRKGYR  SQRGHSRGRN  QNSRRPSRAM  WLSLFSSEES  NLGANNYDDY
421     RMDWLVPATC  EPIQSVFFFS  GDKYYRVNLR  TRRVDTVDPP  YPRSIAQYWL  GCPAPGHL
```

In this specification "Vitronectin" includes proteins having at least 70%, more preferably one of 75%, 80%, 85%, 90%, 95%, 96%, 97%, 98%, 99% or 100% sequence identity with the amino acid sequence of Vitronectin illustrated above.

The term "Vitronectin" also includes fragments of such proteins. A fragment may comprise at least, i.e. have a minimum length of, 10, 11, 12, 13, 14, 15, 16, 17, 18, 19, 20, 25, 30, 40, 50, 60, 70, 80, 85, 90, 95, 96, 97, 98 or 99% of the corresponding full length sequence. The fragment may have a maximum length, i.e. be no longer than, 10, 11, 12, 13, 14, 15, 16, 17, 18, 19, 20, 25, 30, 40, 50, 60, 70, 80, 85, 90, 95, 96, 97, 98 or 99% of the corresponding full length sequence. The fragment may comprise at least, i.e. have a minimum length of 5 amino acids or one of at least 10, 15, 20, 25, 30, 40, 50, 100, 150, 200, 300, or 400 amino acids. The fragment may have a maximum length of, i.e. be no longer than, 10 amino acids, or one of less than 15, 20, 25, 30, 40, 50, 100, 150, 200, 300, or 400 amino acids. The fragment may have a length anywhere between the said minimum and maximum length.

The Vitronectin protein preferably also includes a heparin binding domain having the amino acid sequence of SEQ ID NO:1 or SEQ ID NO:3, or an amino acid sequence having one of 75%, 80%, 85%, 90%, 95%, 96%, 97%, 98%, or 99% sequence identity to SEQ ID NO:1 or SEQ ID NO:3.

The Vitronectin protein may be from, or derived from, any animal or human, e.g. non-human animals, e.g. rabbit, guinea pig, rat, mouse or other rodent (including from any animal in the order *Rodentia*), cat, dog, pig, sheep, goat, cattle (including cows, e.g. dairy cows, or any animal in the order Bos), horse (including any animal in the order *Equidae*), donkey, and non-human primate or other non-human vertebrate organism; and/or non-human mammalian animal; and/or human.

Dosages of Vitronectin

In both *in vitro* and *in vivo* uses, Vitronectin may be used in combination with HS9. In some cell culture methods of the present invention exogenous HS9 is added to the culture.

Suitable concentrations or dosages of Vitronectin include about 500 ng/ml or less, more preferably one of 250 ng/ml or less, 100 ng/ml or less, 90 ng/ml or less, 80 ng/ml or less, 70 ng/ml or less, 60 ng/ml or less, 50 ng/ml or less, 40 ng/ml or less, 30 ng/ml or less, 20 ng/ml or less, 10 ng/ml or less, 5 ng/ml or less; or of about 100 mg or less, 50 mg or less, 40 mg or less, 30 mg or less, 20 mg or less, 10 mg or less, 5 mg or less, 4 mg or less, 3 mg or less, 2 mg or less, or 1 mg or less; or between about range 0.1-5 ng/ml, 0.1-0.2, 0.1-0.3, 0.1-0.4, 0.1-0.5, 0.1-0.6, 0.1-0.7, 0.1-0.8, 0.1-0.9, 0.1-1.0, 0.1-1.5, 0.1-0.2.0, 0.1-2.5, 0.1-3.0, 0.1-3.5, 0.1-4.0, 0.1-4.5, 0.1-5.0 ng/ml.

In some embodiments, in vitro and in vivo uses of HS9 exclude the addition of exogenous Vitronectin. For example, in some cell culture methods of the present invention exogenous Vitronectin is not added to the culture.

The disclosure includes the combination of the aspects and preferred features described except where such a combination is clearly impermissible or expressly avoided.

The section headings used herein are for organizational purposes only and are not to be construed as limiting the subject matter described.

Aspects of the present disclosure will now be illustrated, by way of example, with reference to the accompanying figures. Further aspects and embodiments will be apparent to those skilled in the art.

## Brief Description of the Figures

Embodiments and experiments illustrating the principles of the invention will now be discussed with reference to the accompanying figures.

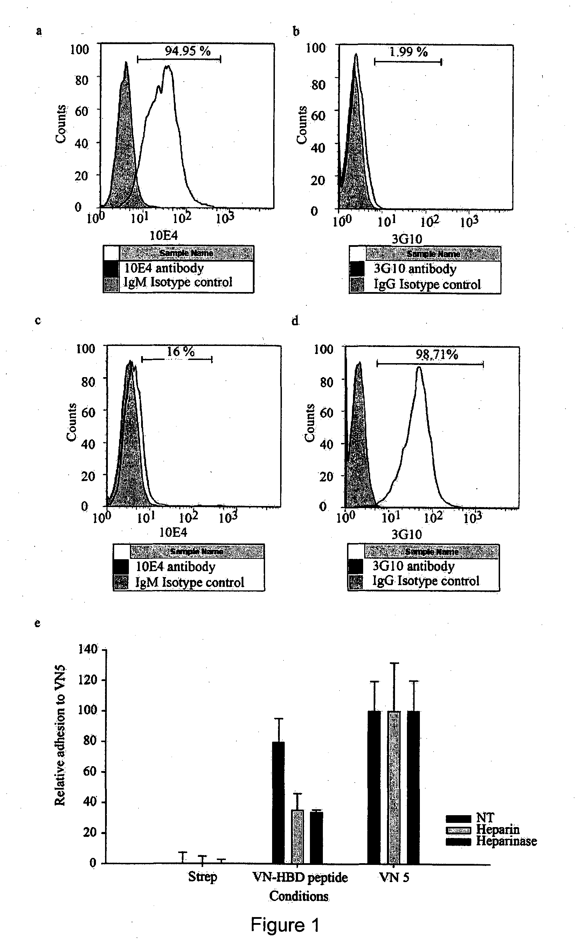

**Figure 1.** FACS analysis of HES-3 cells before and after heparinase I, II and III digestion. (a) 10E4 (b) 3G10 antibody staining of cells before enzyme digestion. Cells expressed high levels of intact HS chains, and low levels of digested HS chains. (c) 10E4 and (d) 3G10 antibody staining of cells after enzyme digestion. Cells expressed low levels of intact HS chains and high levels of digested HS chains. (e) Cell adhesion assay of intact cells, cells pre-incubated with heparin, and heparinase-digested cells. Cells were seeded onto streptavidin control surfaces, VN-HBD peptide surfaces or VN5 surfaces. The adhesion to VN-HBD peptide was reduced by ~ 40 % after heparin and heparinase treatment, while the cells' ability to bind VN5 was not affected. This suggests that cell surface HS is important for the binding of hESCs to the VN-HBD.

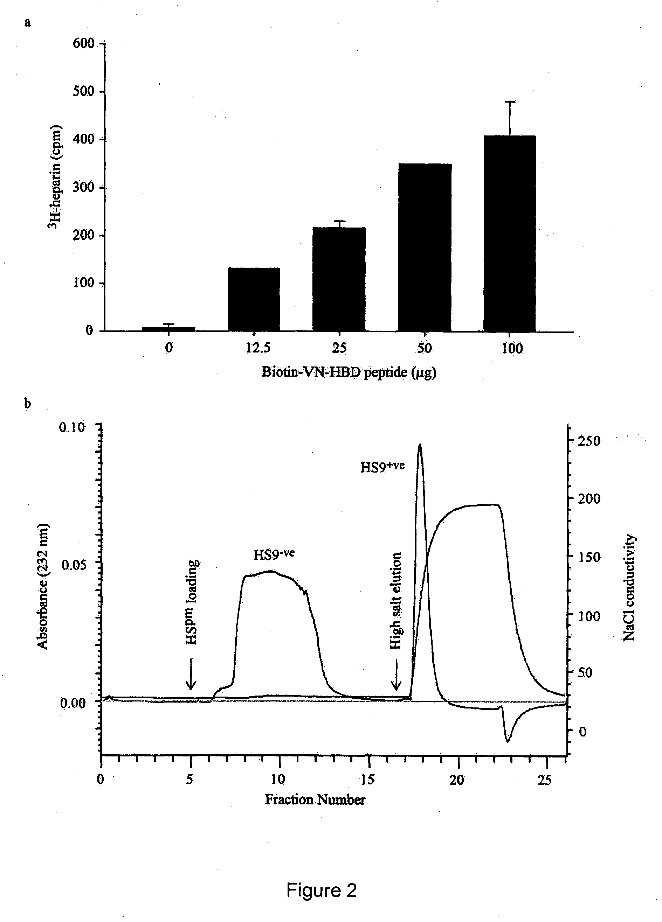

**Figure 2.** (a) Binding ability of VN-HBD to $^3$H-heparin. VN-HBD peptides were spotted onto nitrocellulose membranes and incubated with $^3$H-heparin. Bound $^3$H-heparin was determined by liquid scintillation. A concentration-dependent increase in $^3$H-heparin binding to VN-HBD peptide confirming the sequences on the peptide are indeed the heparin binding domain. (b) Chromatogram depicting HS9$^{+ve}$ isolation. HS9$^{+ve}$ variant were isolated from the starting HS$^{pm}$ mixture using streptavidin column pre-bound with VN-HBD peptide. The flow-through unbound HS9$^{-ve}$ variant and later with a high salt wash (1.5 M) (red trace) released the HS9$^{+ve}$ variant (blue trace). Both variants are collected and desalted before further analysis.

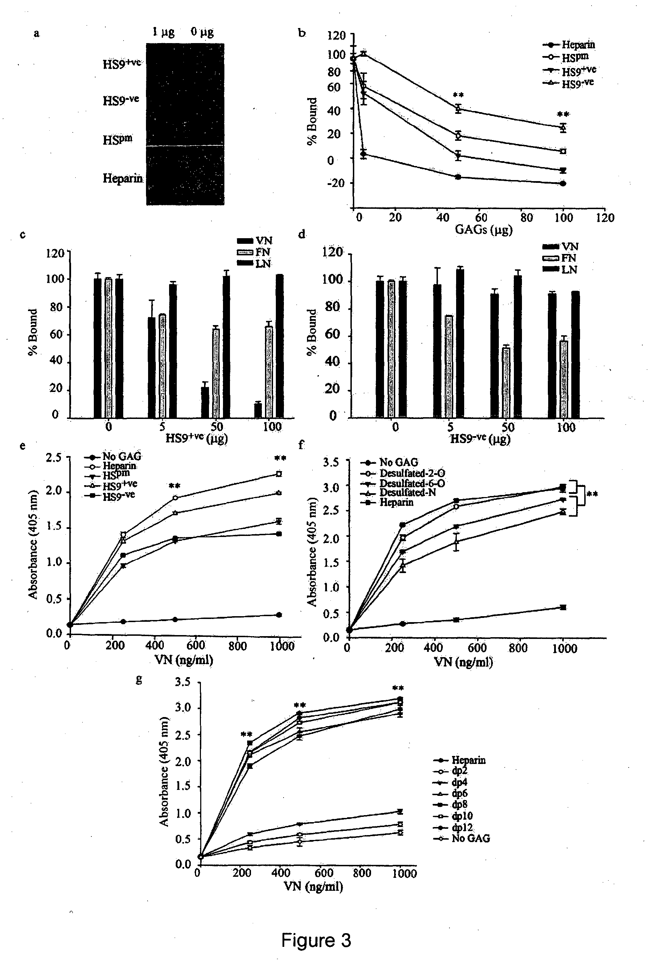

**Figure 3.** (a) Dot blots binding profile of the different HS variants. VN was spotted onto the membrane and incubated with different GAGs (HS9$^{+ve}$, HS9$^{-ve}$, HS$^{pm}$ and heparin). HS9$^{+ve}$ variants have a higher binding capacity for heparin than the HS9$^{-ve}$ variants; HS$^{pm}$ has only intermediate binding ability. (b) The competition assay was performed with soluble heparin, or the HS variants. Inhibitory effects of the various HS variants on the binding of VN to heparin beads were observed. Soluble heparin binds most avidly to VN, followed by HS9$^{+ve}$, HS$^{pm}$ and HS9$^{-ve}$. (c) Inhibitory effect of HS9$^{+ve}$ variant on the binding of ECM proteins (VN, FN and LN) to heparin beads. HS9$^{+ve}$ variants inhibited VN rather than to FN and LN in binding to heparin beads. On the other hand, (d) HS9$^{-ve}$ variant had higher affinity to FN than VN and LN in competition beads assay. (e) Binding profile of various GAGs to VN by GAG-ELISA. HS9$^{+ve}$ variants had a significantly higher affinity for VN than the HS$^{pm}$ and HS9$^{-ve}$ variants. (f) Binding profile of various de-sulfated heparin to VN by GAG-ELISA. Variants without 6-O and N sulfation had significantly reduced binding, and variants without 2-O sulfation had no effect on VN binding. (g) Binding profile of various length of heparin to VN by GAG-ELISA. GAGs of dp2 and dp4 were not able to bind VN, but dp6 units and longer were able to bind to VN. Heparin serves as a positive control. ** = P < 0.05

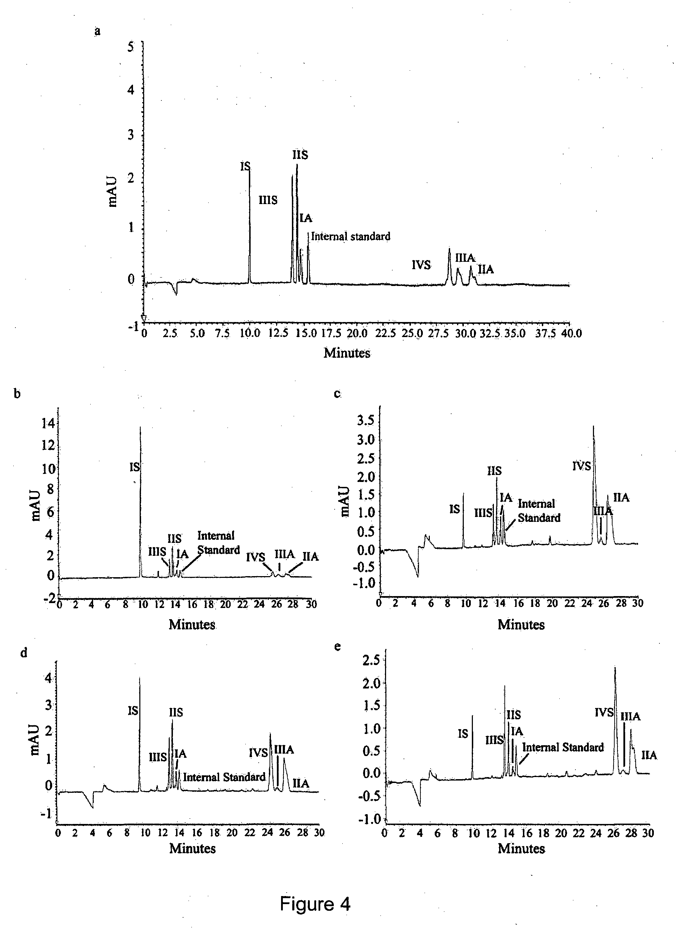

**Figure 4.** (a) Electropherogram of A-disaccharide standards using CE. Standards were individually separated with distinct peaks. Electropherograms of the depolymerized samples. (b) Heparin, (c) HS$^{pm}$, (d) HS9$^{+ve}$ and (e) HS9$^{-ve}$. IS: ΔUA2S(1→4)-D-GlcNS6S (2S, NS, 6S); IIIS: ΔUA2S(1→4)-D-GlcNS (2S, NS); IIS: ΔUA(1→4)-D-GlcNS6S (NS, 6S); IA: ΔUA2S(1→4)-D-GlcNAc6S (2S, 6S); IVS: ΔUA(1→4)-D-GlcNS (NS); IIIA: ΔUA2S(1→4)-D-GlcNAc (2S); IIA: ΔUA(1→4)-D-GlcNAc6S (6S) Internal standard helped in identifying each peak.

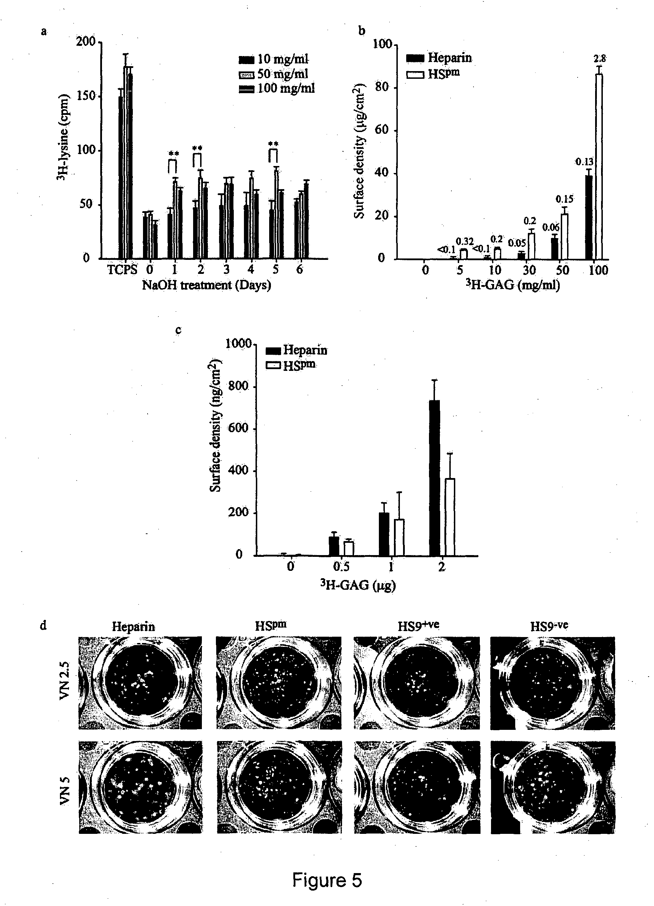

**Figure 5**. (a) Optimizations of surface and EDC concentration for covalent grafting. $^3$H-lysine was used as a read-out for the EDC grafting ability on the different surfaces. NaOH was used to etch the PS surface for 6 days to produce different densities of carboxyl groups. TCPS gives the highest grafting ability as compared to NaOH-treated surfaces, regardless of how many days etching was carried out. The optimized surface was TCPS and EDC concentration was 50 mg/ml. ** = P < 0.05. (b) Surface density of heparin and HS$^{pm}$ on EDC grafted surfaces. Concentration-dependent increase in $^3$H-GAG grafting onto surfaces. The numbers above the bars (in %) represent the grafting efficiency. This low efficiency is not feasible for further studies. (c) Surface density of heparin and HS$^{pm}$ on PLL surfaces. Increasing surface density was observed with increasing solution concentrations. The surface density of $^3$H-heparin and $^3$H-HS$^{pm}$ after exposure to 2 mg of coating solution was $\sim$ 800 ng/cm$^2$ and 400 ng/cm$^2$ respectively. (d) HES-3 cell images after 7 days of culture on PLL+GAG+VN surfaces. Cells did not spread, or reach confluence. Scale bar = 0.3 mm.

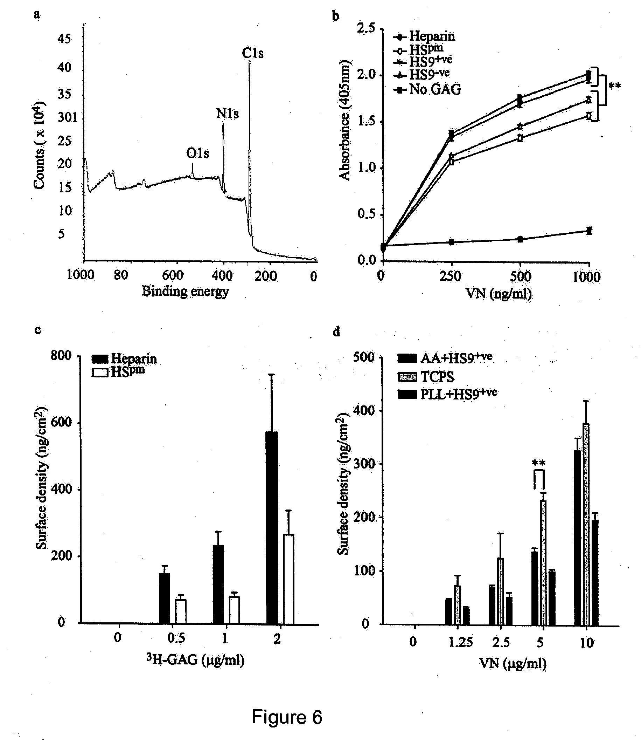

**Figure 6**. (a) XPS binding energy profile of 100% AA plates. The 100% AA surface has C (79.2 %), N (16.4 %) and O (4.34 %). (b) VN binding profile on the 100% AA surface using GAG-ELISA. The HS9$^{+ve}$ variants bind VN significantly better than the HS9$^{-ve}$ variants. Uncoated wells and heparin-coated wells served as the negative and positive control respectively. (c) Surface densities of heparin and HS$^{pm}$ on the 100% AA surface. GAG binds to AA surface in a concentration-dependent manner with the heparin density higher than HS$^{pm}$. $^3$H-GAG (1 mg) was used for coating; the final surface density of $^3$H-heparin was $\sim$250 ng/cm$^2$ and of the $^3$H-HS$^{pm}$ $\sim$ 100 ng/cm$^2$. (d) $^{125}$I-VN surface density on TCPS-, AA+HS9$^{+ve}$- and PLL+HS9$^{+ve}$- coated surfaces. The highest VN density was measured on TCPS, followed by the AA+HS9$^{+ve}$ surface, with the lowest density on PLL+ HS9$^{+ve}$ across all the VN concentrations used. ** = P < 0.05.

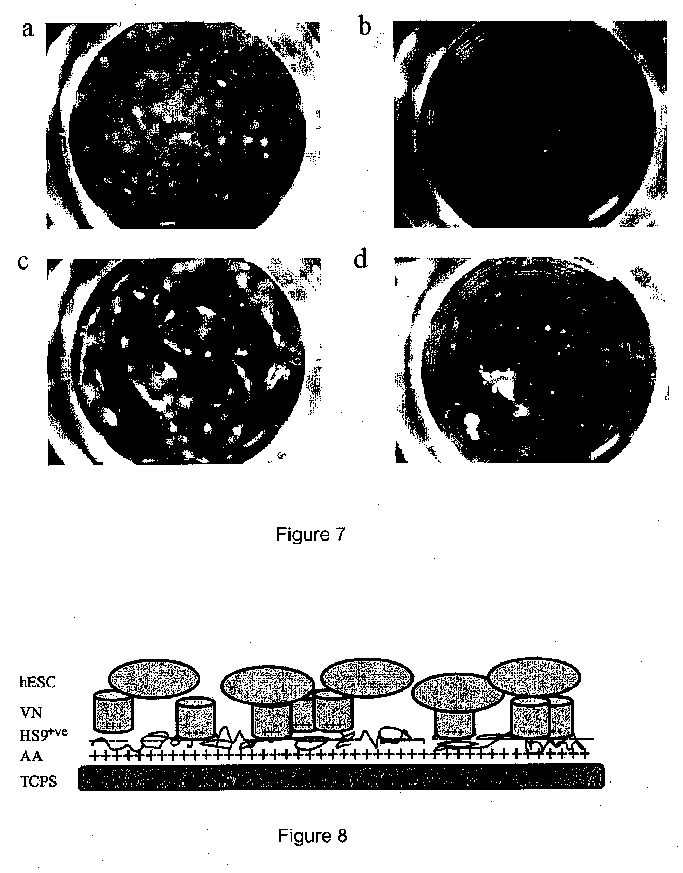

**Figure 7.** Photomicrographs of HES-3 cells on AA+GAG+VN2 surfaces after 1 week. (a) AA+Heparin+VN2, (b) AA+HS$^{pm}$+VN2, (c) AA+HS9$^{+ve}$+VN2 and (d) AA+HS9$^{-ve}$+VN2. Cells remained attached to AA+Heparin+VN2 and AA+HS9$^{+ve}$+VN2 substrates but detached on AA+HS$^{pm}$+VN2 and AA+HS9$^{-ve}$+VN2 substrates. This showed that there are sufficient immobilized VN on heparin and HS9+ve underlying substrates for the attachment and proliferation of HES-3 cells. Scale bar = 1 mm

**Figure 8.** Summary of novel substrate for hESC culture. TCPS surfaces were first polymerized with positively-charged AA, than coated with negatively-charged HS9$^{+ve}$ variants and VN for hESC culture.

**Figure 9.** Table 1 Comparison of the different A-disaccharides composition of depolymerized GAG samples.

**Figure 10.** Table 2 Summary of the N: C ratios of 0, 50, 80, 90 and 100% AA surfaces.

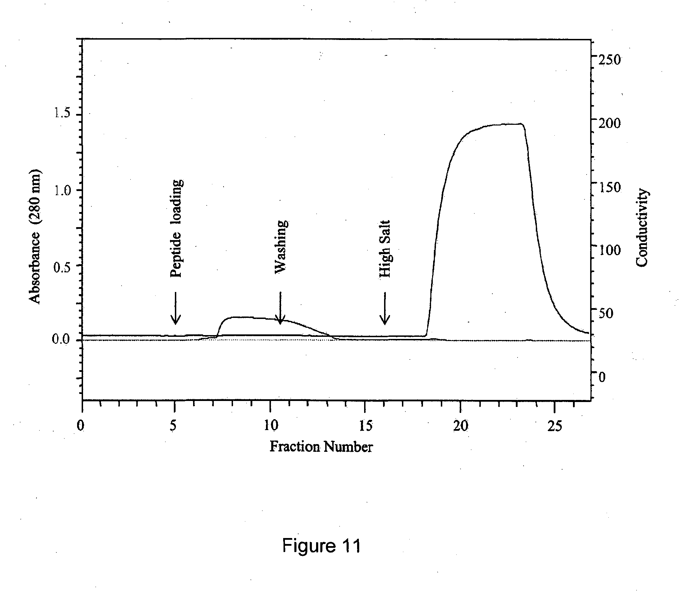

**Figure 11.** Chromatogram of biotinylated VN-HBD peptide loading. Peptide was loaded into the column and excess peptide that flows out of the column was monitored at 280 nm. Column was washed with 1.5 M high salt buffer to ensure peptide is tightly bound to the column.

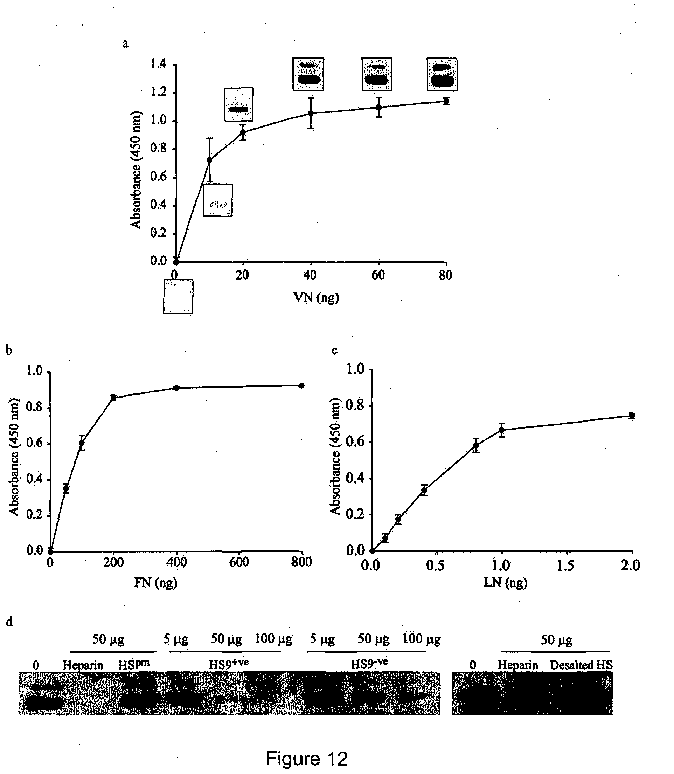

**Figure 12.** (a) VN binding profile on heparin beads. Beads were incubated with different amounts of VN, and visualized with HRP. To prevent non-specific binding, sub-optimal amounts of VN were used as probes. The small insert shows the immunoblot images of the respective amounts of VN. (d) Immunoblot images of the VN left on the beads after heparin beads competition assay. Note that desalted HS$^{pm}$ binds to VN better than NaCl containing HS$^{pm}$. Binding profiles of (b) FN and (c) LN to heparin beads. Beads bound to increasing amount of protein and reached saturation at 200 ng for FN and 1 mg for LN.

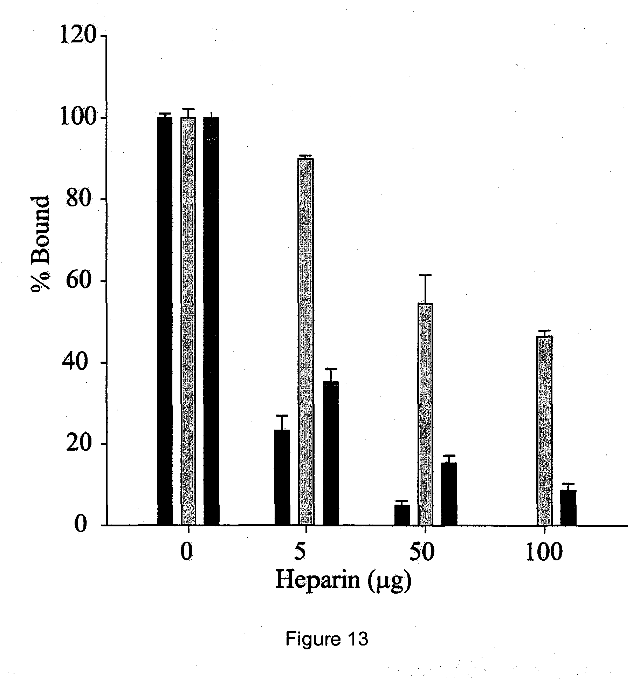

**Figure 13.** Competition heparin beads assay to evaluate the inhibitory effect of heparin on the binding of VN, FN and LN to beads. Heparin was used as positive control. Heparin was able to bind VN, FN and LN in a concentration-dependent manner with varying affinities.

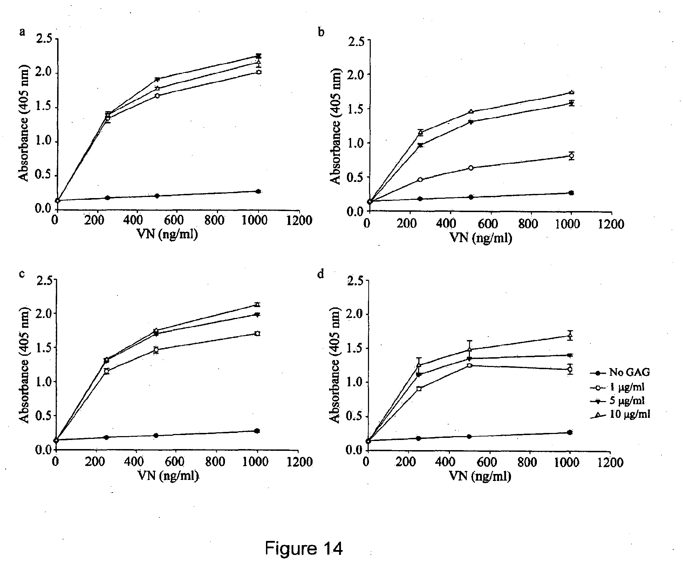

**Figure 14.** Determination of saturating amounts of GAGs with GAG ELISA. Differing concentrations of (a) heparin, (b) HS$^{pm}$, (c) HS9$^{+ve}$ and (d) HS9$^{-ve}$ were coated onto the wells and their ability to bind VN analyzed. No significant difference was observed in either 5 or 10 mg/ml; therefore 5 mg/ml was the saturating concentration. Uncoated wells served as the negative control.

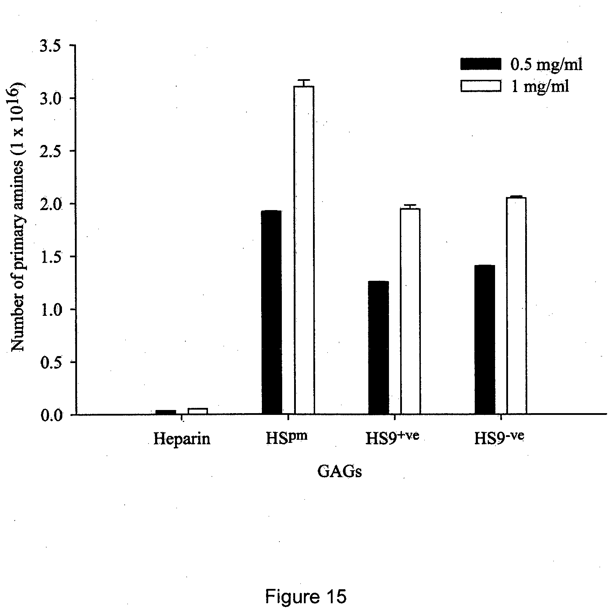

**Figure 15.** Number of primary amines in GAGs by fluorescamine protein assay. Increase in number of amines from 0.5 mg/ml to 1 mg/ml GAGs. A > 60 % difference in the number of primary amines at 1 mg/ml of HS and heparin.

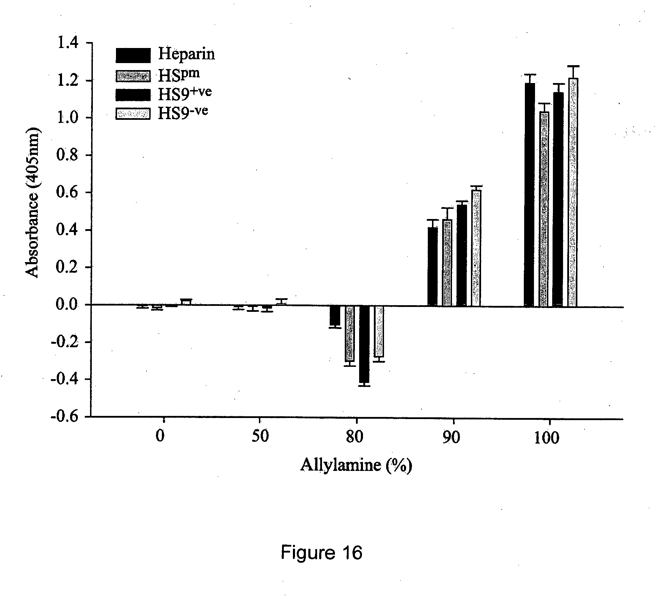

**Figure 16.** GAG binding profile on the different allylamine surface by ELISA. GAG (5 mg/ml) was coated onto the different AA density surfaces (0, 50, 80, 90 and 100%) followed by binding of 500 ng/ml of VN. The AA surface at 100 % density binds the highest amount of VN while densities < 100 % no longer bind the optimal amount of GAG.

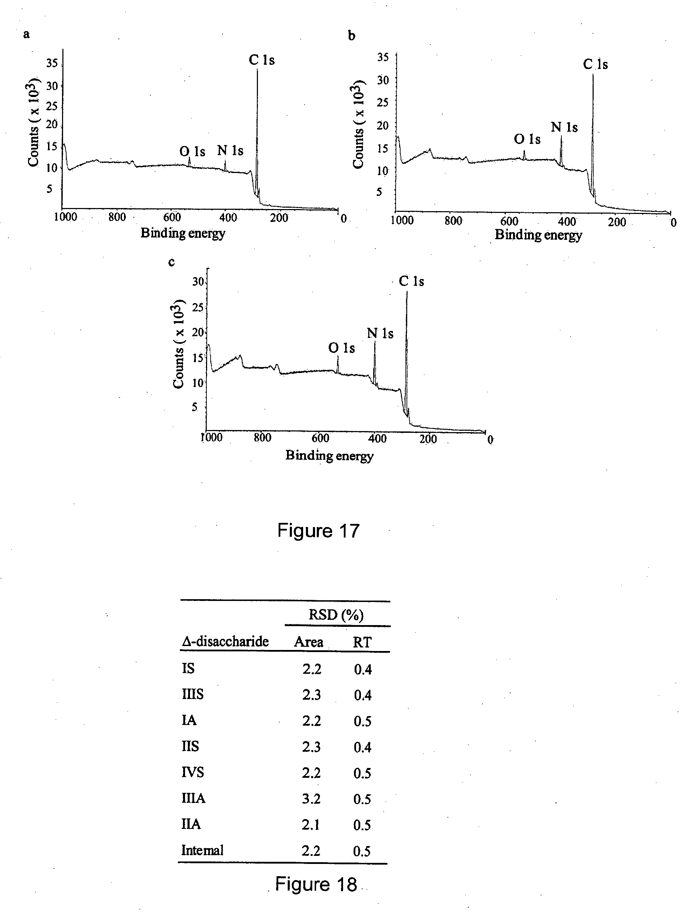

**Figure 17.** Representative XPS binding energy profile of 50-100% AA plates. Results were analyzed with CasaXPS software to determine the area of the peaks and N: C ratio was calculated. (a) 50% AA: 50% octa-1, 7-diene (b) 80% AA: 20% octa-1, 7-diene (c) 90% AA: 10% octa-1, 7-diene. Higher amounts of nitrogen atoms were calculated from the higher % of AA utilised.

**Figure 18.** Relative standard deviation (R.S.D.) of Δ-disaccharide standards. RT represents retention time. When area RSD were < 5 %, and migration time RSD of < 1 % were considered as good reproducible results.

**Examples**

Materials and methods. All examples dealing with HES cells are illustrative only.

*Preparation of VN-HBD peptide surfaces*

Following the VN5 platform in our earlier work [18], this study exploited an N-terminal biotinylated VN-HBD peptide (Biotin-PRPSLAKKQRFRHRNRRKGYRSQRGHSRGRNQNSRR) [48] synthesized by China Peptides Co. Ltd, China. This peptide, which lacks an RGD motif, was first immobilized onto streptavidin-coated surfaces to assess the attachment efficiencies of overlayed, heparinase-treated hESCs [15]. The streptavidin (Genescipt) was first reconstituted in PBS to 1 mg/ml stock and subsequently prepared as a 20 μg/ml working concentration. Bacterial grade 24-well plates (Beckon Dickinson) were coated with 625 μl of the working streptavidin concentration and incubated overnight at 4 °C. Wells were then washed twice with PBS and 10 μM of VN-HBD peptide incubated for 2 h at room temperature, after which wells were washed again and 1 ml of mTeSR™1 media supplemented with 10 μM Rock inhibitor (Y27632) (Calbiochem) [49] added according to the method of Klim *et al.* [15]. Coated plates were used immediately for crystal violet cell adhesion assays as previously described [18]..

*Heparinase digestion and heparin-inhibition of HES-3 cells*

To efficiently remove cell surface HS for the study of cell attachment, heparinase I, II and III were employed. Heparinase I (E.C. 4.2.2.7), heparinase II (no E.C. number) and heparinase III (E.C. 4.2.2.8) (Sigma Aldrich) were resuspended in digestion buffer (20 mM Tris-HCL, 50 mM NaCl, 4 mM $CaCl_2$ and 0.01 % BSA, pH 7.5) and used within 1 freeze-thaw cycle. HES-3 single cells at $1 \times 10^6$ cells per well were resuspended in 100 μl of DMEM/F12 media (Invitrogen) containing heparinase I (10 milli international units (mIU)), heparinase II (5 mIU) and heparinase III (10 mIU). Cell suspensions were incubated at 37 °C for 1 h, with mixing every 10 min. The cells were then spun at 12,000 rpm and surface HS expression analyzed by FACS. Soluble heparin (Sigma Aldrich) was preincubated with cells and served as a competitor to the surface-bound HS with the peptide surface. Similarly, cells ($1 \times 10^6$) were resuspended in 100 μl DMEM/F12 media (Invitrogen), incubated with soluble heparin at 500 μg/ml concentrations for 1 h, with mixing after every 10 min. Cells were then analysed for binding to VN-HBD surfaces with the crystal violet assay. Both the heparinase-digested cells and cells incubated with soluble heparin were seeded onto the prepared 10 μM of VN-HBD peptide surface or a VN5 positive control [18] in 96-well plates. Plates were left to incubate for 45 min to allow attachment, and crystal violet cell adhesion assays performed. Streptavidin-coated surfaces served as a negative control and data was normalized to the absorbance of VN5.

*FACS analysis of heparinase-digested cells*

To ensure enzymatic digestion effectively removed exogenous HS chains from HES-3 cells, FACS analysis using both the 10E4 [50] and 3G10 [51] anti-HS antibodies was performed. Digested cells at a density of $2.5 \times 10^5$ were washed with 1 % BSA and 200 μl of either 10E4 or the 3G10 (1:100) antibodies (Seikagaku) added for 1 h against the respective isotype controls (10 μg/ml) (DAKO). Cells were then washed and stained with FITC-conjugated goat anti-mouse secondary antibody (DAKO) (1:500) for 30 min. Exogenous HS expression was then determined using a FACS Calibur (Becton Dickinson) and the results analyzed with FlowJo software. Gating was done at the point of intersection between the isotype control and 10E4/3G10 expression.

*Peptide binding assay with ³H-heparin*

To confirm the binding ability of the synthesized VN-HBD peptide to heparin, the ³H-heparin binding assay was employed in the manner of Baird *et al.* [52]. Briefly, biotinylated VN-HBD peptide was serially diluted in PBS (0, 12.5, 25, 50 and 100 μg peptide) and individually spotted onto 0.2 μM nitrocellulose membranes (Biorad). Peptide was left to dry on the membrane and heated to 80 °C for 30 min. The membrane was then washed three times with PBS, whereupon 1 ml of 0.1 μCi of ³H-heparin (Perkin Elmer) in 4 % BSA (Sigma Aldrich) was added, and the membrane incubated overnight. Next day, the membrane was washed three times and 1 ml of Ultima Gold scintillation cocktail (Perkin Elmer) added with analysis in a liquid scintillation Tri-carb 2800TR counter (Perkin Elmer) for 1 min.

*Affinity chromatography*

Saturating amounts of biotinylated VN-HBD peptide (3 mg) were coupled to a 1 ml streptavidin column (GE Healthcare) as assessed by the detection of unbound peptide at $A_{280}$ nm in the flowthrough. To ensure peptide was firmly bound to the column, a 1.5 M high salt buffer wash was performed. When no HS trace ($A_{280}$ nm) was detected, the column was equilibrated with low salt buffer prior to HS loading.

Crude HS (HS$^{pm}$) (Celsus Laboratories) was dissolved in low salt buffer (20 mM phosphate buffer, 150 mM NaCl, pH 7.2) at 1 mg/ml. A total of 100 mg HS$^{pm}$ solution was loaded in a total of 30 separate injections in low-salt buffer (Biologic-Duoflow chromatography system; Bio-Rad) at 0.2 ml/min, and the column washed with the same buffer until the baseline reached zero. The bound HS was eluted with a one step gradient of 1.5 M high salt (20 mM phosphate buffer, 1.5 M NaCl), the bound and unbound variants collected (monitored at $A_{232}$ nm), and the column re-equilibrated with low-salt buffer. The eluent (HS9$^{+ve}$) and flow-through (HS9$^{-ve}$) peak samples were collected separately, freeze-dried, and stored at -20 °C. Both the HS9$^{+ve}$ and the HS9$^{-ve}$ variants were then separately dissolved in 10 ml of HPLC grade water (Sigma Aldrich) and desalted once on a HiPrep 26/10 desalting column (Amersham Biosciences). The different HS variants were then collected, freeze-dried, and stored at - 20 °C.

*Dot Blotting*

To analyze the different HS variants for binding affinity to VN, nitrocellulose membranes were rinsed with TBST and VN (1 μg) added into the wells. The membrane was blocked with 5 % BSA for 1 h. The GAGs (2 mg) were initially biotinylated using biotin-LC-hydrazide (60 μl of a 2 mg/ml solution) (Thermo Scientific) dissolved in 1 ml of 0.1 M MES buffer, pH 5.5. Briefly, EDC (1.5 mg) was added to the mixture and incubated for 2 h before the addition of another 1.5 mg of EDC after which unincorporated biotin was removed with a Fast Desalting (PD 10) Column (GE Healthcare). These biotinylated GAGs (1 μg) were added into the wells of the dot blot apparatus, left for 10 min and then aspirated off with a pipette and washed with TBST. Streptavidin-HRP (2 ml) was added for 10 min, washed, exposed to LumiGLO chemiluminescent substrate (Kirkegaard & Perry Laboratories) and exposed to X-ray film (Amersham).

*Heparin-Sepharose bead competition assay*

Heparin-Sepharose bead competition assays were performed to investigate the binding affinity of each desalted HS variant (Heparin, HS$^{pm}$, HS9$^{+ve}$ and HS9$^{-ve}$) to VN and other ECM proteins according to Ono *et al.* [53]. Briefly, assays were done at room temperature and 20 μl of heparin-Sepharose beads (Sigma Aldrich) with 20 μl of Biogel P10 (Biorad) per reaction used. A "master mix" of bead slurry was prepared to reduce error. The master mix was washed 3 times with 1 ml of 1% BSA. Aliquots (40 μl) of bead slurry were separated into individual 1.5 ml Eppendorf tubes for binding experiments.

Varying concentrations of ECM proteins (VN, LN, and FN) were added to the beads in 100 μl volume. The suspension was incubated for 30 min under constant rotation, after which the beads were spun (2000 rpm) for 1 min, and washed twice with 1 ml of 1% BSA and with 1 ml of 0.02 % Tween20 (Sigma Aldrich) in PBS. The corresponding anti-ECM antibody (100 μl) (Millipore) in PBS (250 ng/ml anti-VN, 5 μg/ml anti-LN, 2 μg/ml anti-FN), was added for another 30 min. The beads were washed and 100 μl of the HRP-conjugated goat anti-mouse antibodies (1:10,000) in PBS was added for 30 min. Finally, the beads were washed, 100 μl of TMB substrate (Thermo Scientific) added and colour developed. After 30 min, 50 μl of 2 M $H_2SO_4$ was added.

The binding affinities of the GAGs to ECM proteins were next investigated by competition assay [53]. Different concentrations (0, 5, 50 and 100 μg) of GAGs were pre-incubated with the individual ECM protein in 100 μl for 30 min with rotation. The reaction was then added into the washed 40 μl of bead slurry. Results were expressed as "percentage bound" by normalizing to readings from control (uncompleted) beads. To confirm the results from the competition assay, immunoblotting was performed. After the competition, the beads were washed and boiled at 95 °C with 30 μl of Laemmli buffer (Sigma Aldrich). The beads were spun and the supernatant loaded into SDS-PAGE gels (Invitrogen) at 180 V for 40 min and transferred to nitrocellulose membranes. The membrane was then probed with the corresponding primary antibody in 5 % BSA at 4 °C overnight. Then, HRP-conjugated goat anti-mouse secondary antibody (Jackson Immunoresearch) (1:10000) in 5 % BSA was incubated for 1 h at room temperature. Membranes were finally washed and exposed to LumiGLO Reserve™ chemiluminescent substrate (Kirkegaard & Perry Laboratories) to visualise the bands.

*Glycosaminoglycan ELISA*

This assay was based on the immobilization of HS variants onto the glycosaminoglycan-binding 96-well plates (Iduron) and the VN binding ability assessed via antibodies as per manufacturer's recommendations. Wells were incubated overnight at room temperature with 200 μl of 5 □g/ml GAGs (Heparin, HS$^{pm}$, HS9$^{+ve}$ and HS9$^{-ve}$), heparin disaccharide standards (dp2 to dp12) or selectively desulfated heparin standards prepared in standard assay buffer (SAB; 100 mM NaCl, 50 mM NaAc, (v/v) 0.2 % Tween 20, pH 7.2). Wells were then washed with 200 μl of SAB three times and blocked (0.4 % (w/v) fish gelatin in SAB), for 1 h at 37 °C. Wells were washed with SAB three times and VN at different concentrations (0 - 1 μg/ml, 200 μl each) added into the wells and incubated at 37 °C for 2 h. Wells were again washed and 200 μl of anti-VN antibodies (250 ng/ml) (Millipore) added at 37 °C for 1 h. Wells were washed to remove unbound antibody and 200 μl of 250 ng/ml goat anti-mouse biotinylated antibody (Sigma Aldrich) added for 1 h at 37 °C. After incubation, wells were washed and ExtrAvidin (200 μl of 220 ng/ml) (Sigma Aldrich) added for 30 min for 37 °C. Wells were finally washed (3 times) and 200 μl of SIGMA*FAST*™ p-Nitrophenyl phosphate (Sigma Aldrich) added for 40 min. Colourimetric absorbance was read at 405 nm with a Victor multiplate reader (Perkin Elmer).

*Capillary electrophoresis*

Heparin, Hs[pm], HS9[+ve] and HS9[-ve] variants (200 μg) were all digested (50 mM NaAc, 1 mM calcium acetate and 100 μg/ml BSA, pH 7) with heparinase I, II and III (Iduron) to yield 2 mg/ml stock. Heparin was first digested with 4 mIU of heparinase I and II each for 3 h at 30 °C, then 1 mIU of heparinase II for another 60 min. HS samples were digested with 4 mIU heparinase I for 30 min and 4 mIU heparinases II and III for another 3.5 h at 30 °C. Absorbances at 232 nm were measured throughout the digestion process to ensure complete digestion. Reactions were terminated by denaturing at 95 °C for 1 min.

Quantification of disaccharides in the depolymerized samples was completed by diluting the stock (2 mg/ml) to 1 mg/ml with MilliQ water and 25 μl of a 1 mg/ml internal standard (4UA-2S ® GlcNCOEt-6S) (Iduron) Δ-disaccharide added. The depolymerized sample was then subjected to CE and the area-under-the-peak then compared to a standard curve. Molar percentages of each sample were then calculated from the molecular weight of each standard. To generate the standard profile for comparison, heparin disaccharide (Δ-disaccharide) standards (Iduron) were separated at 250 μg/ml.

CE was performed on a P/ACE MDQ instrument equipped with a diode array detector (Beckman) at 25 °C. Membrane-filtered (0.22 μm) 60 mM formic acid solution (pH 3.4) (Sigma Aldrich) was used as the running buffer. Separations were carried out in uncoated fused-silica capillaries, with a length of 60 cm and a 75 μm internal diameter (Beckman). The cycles were programmed for 5 min water rinses, 3 min 1M NaOH, 5 min buffer washes and 5 sec sample injection (0.5 pound force per square inch (p.s.i.), reverse polarity of -15 kV). Disaccharides were separated for 40 min and individual peaks were detected at 232 nm.

*Sodium hydroxide etching of polystyrene surfaces*

To immobilize HS chemically, NaOH was employed for the etching of polystyrene surfaces to expose the maximal number of carboxyl groups in white-wall, transparent-bottomed 96-well TCPS plates (Corning) [54]. A solution of 4: 1 (v/v) NaOH (4 N): methanol was prepared and 250 μl added into each well at the nominated time points (day 0 to day 7) at 37 °C. After 7 days, the wells were washed with 10 % citric acid (Sigma Aldrich) for 1 h and used for subsequent $^3$H-GAG grafting assays.

*Fluorescamine Assay*

To compare primary amine content of the various GAGs (heparin, HS[pm], HS9[+ve] and HS9[-ve]), a fluorescamine protein dye assay was utilised. Fresh stocks of fluorescamine (Sigma Aldrich) (3 mg/ml) were prepared with dimethyl sulfoxide (DMSO) in an amber tube with vigorous mixing. Two GAG concentrations (0.5 and 1 mg/ml) were utilized. GAG solutions (90 μl) were added to 30 μl of the stock fluorescamine solution and mixed by pipeting. The reaction was allowed to proceed for 15 min, after which the reaction was thoroughly mixed and read using an Infinite® 200 Multimode Microplate Reader (Tecan). Standard curves were generated from the two-fold dilutions of BSA (from 500 μg/ml) standards. The 90 μl standards were mixed with 30 μl of dye at room temperature for 15 min and read. Concentrations of primary amines were quantified by comparison to the standard curve.

*Covalent binding of GAG to TCPS with EDC*

To physically link HS onto NaOH-etched PS and TCPS surfaces, covalent immobilization was explored. Here 1-ethyl-3-[3-dimethylaminopropyl] carbodiimide hydrochloride (EDC) (Sigma Aldrich) was optimized for the crosslinking of amines in $^3$H-lysine (Perkin Elmer) to a working concentration of 50 mg/ml. This was dissolved in 0.1 M MES buffer, pH 6, and 200 μl of this solution added into white-wall, transparent-bottomed 96-well TCPS plates to react for 1 h at room temperature with agitation. After 60 min, the plate was first washed with 0.1 M MES buffer and then with water, twice each. Different concentrations of $^3$H-heparin and $^3$H-HS (0 to 100 mg/ml) in PBS (100 μl) were added into triplicate wells and incubated at room temperature for 2 h with agitation. Finally, the wells were washed twice with 10 x PBS (10 min each), once with water (10 min) and three more times with water (5 min each). Scintillation liquid (200 μl) was added into each well and radioactive counts (1 min) monitored on a MicroBeta counter (Perkin Elmer).

*Poly-L-lysine coating*

A simple method of creating a positively charged surface is by coating with poly-L-lysine (PLL). A 0.01 % PLL solution (Sigma Aldrich) (50 μl) was added into each well of the white-wall, transparent-bottomed 96-well TCPS plates or 300 μl into each well of 24-well plates for 5 min at room temperature with agitation. Excess unbound PLL solution was removed and the surface washed twice with PBS. The plate was air-dried for 3 h. The poly-L-lysine coated positive charged surfaces were then utilized for the immobilization of GAGs (5 μg/ml) and subsequent cell culture.

*Screening of PLL surface with HES-3 ESCs*

To ensure the suitability of PLL surfaces for ESC culture, a cell attachment assay was performed with HES-3. PLL-pre-coated 24-well plates were coated with 400 μl of GAG (heparin, HS[pm], HS9[+ve], HS9[-ve]) (5 □g/ml) in PBS for 2 h at room temperature. Wells were washed with PBS and subsequently coated with VN2.5 or VN5 (300 μl per well) in PBS overnight at 4 °C. HES-3 cells were routinely maintained on TCPS coated with VN5 in mTeSR™1 media (Stem Cell Technologies). Differentiated cells were removed by manual pipetting and the rest dissociated mechanically. Cells $(3 \times 10^5)$ were seeded into each test well and cell growth assessed at the end of day 7.

*Allylamine plasma polymerization and analysis*

To robustly generate positively charged TCPS surfaces for electrostatic immobilization of negative charged GAGs, plasma polymerization using allylamine monomer (Sigma Aldrich) was employed [47]. Polystyrene plates (96-well or 24-well) (SARSTEDT) and an aluminium foil were placed in the plasma chamber under vacuum overnight to remove any air prior to the next day's plasma coating. The reactor was evacuated to a base pressure of less than $5 \times 10^{-4}$ mbar.

Allylamine monomer was degassed over several freeze-thaw cycles to remove dissolved gases before use. A monomer flow rate of ∼ 5 standard cubic centimetres per minute (sccm) [46] was tuned with a needle valve and allowed to stabilise before deposition. To achieve the different percentages of the AA-coated plates, the flow was mixed with different ratios of allylamine and octa-1, 7-diene. The monomer ratios used were 100% allylamine, 90% allylamine: 10% octa-1, 7-diene, 80% allylamine: 20% octa-1, 7-diene, 50% allylamine: 50% octa-1, 7-diene and 100% octa-1, 7-diene. The flow rate was calculated by formula [55]:

$$\text{(Pressure after isolating the chamber at the end of 30 sec – pressure before isolating the chamber)/ 30 *1251}$$

Plasma was then ignited with a radio frequency generator (Coaxial Power System Ltd) at 13.56 MHz and 5 Watts. The plasma was turned on for 40 min after which the chamber was pumped back to base pressure. The plates were removed and lids were replaced to maintain sterility. These sterile plates were then used for the experiments involving immobilization of GAGs for cell culture. The aluminium foil in the chamber was read by a XPS equipped with an aluminium anode and wide-scan analysis to confirm the density of amines deposited onto the plates. Results were analyzed with CasaXPS software. The nitrogen: carbon (N: C) ratios were calculated from the area underneath the N and C peaks to obtained the relative amount of AA on the surface. Immobilized GAGs on the various percentages of AA-coated 96-well plates were analysed for their binding ability to VN using the GAG ELISA assay method.

*Quantification of $^3H$-heparin and $^3H$-HS$^{pm}$*

To determine the amount of GAG bound to the 100 % AA-polymerized 96-well plate, binding assays using $^3H$-heparin (Perkin Elmer) and $^3H$-HS$^{pm}$ (radiolabelled by RC TRITEC, Switzerland) were employed. Ascending concentrations (0, 1.25, 2.5 and 5 μg/ml) of mixtures of 90 % unlabelled heparin or HS$^{pm}$ : 10% of $^3H$-heparin and $^3H$-HS$^{pm}$ radioactive solution were prepared in PBS. Each GAG solution (200 μl) was incubated on the surfaces to be tested (TCPS, PLL, AA) overnight at room temperature. The next day, wells were washed with PBS, 200 μl of scintillation

cocktail added, and the plates read three times in a MicroBeta liquid scintillation counter for 1 min each. A standard curve was generated with known amounts of $^3$H-GAG and the surface densities of heparin and HS$^{pm}$ determined.

*$^{125}$I-VN quantification on surfaces*

To quantify the amount of VN bound to the different 96-well plates (TCPS, PLL+HS9$^{+ve}$ and 100 % AA polymerized surfaces coated with HS9$^{+ve}$) AA+HS9$^{+ve}$ radioactive-labelled VN was employed. Surfaces were coated with 5 μg/ml of HS9$^{+ve}$ at 300 μl per well. VN was labelled with $^{125}$I isotope (Perkin Elmer) using Iodination Beads (Thermo Scientific) and quantified with a MicroBeta scintillation counter (Perkin Elmer).

*Statistical analysis*

All data values are all reported in triplicates as $\pm$ standard error. One-way ANOVA was performed to compare differences across the groups, and $P < 0.05$ was considered as significant. A two-tailed student's t-test was performed to determine the differences between two sample groups. Graphs were plotted and data transformed using Sigma plot software.

Results

*Surface heparan sulfate is important for attachment to VN-HBD substrate*

As endogenous GAGs might have been part of the attachment complex with VN, we first sought to investigate the role of surface HS in cell attachment. Braam *et al.* have previously shown that $\alpha$V$\beta$5 integrin is required for hESCs to adhere to full-length VN [12]. A study by Klim *et al.* also demonstrated that surface GAG is important for hESCs to be able to bind the VN-HBD, which lacks an RGD motif, after digestion with chrondroitinase ABC. However, chrondroitinase ABC digests primarily chondroitin sulfate (CS), rather than the most abundant GAG on stem cell surfaces, HS. Therefore, to investigate the functional role of surface HS on cell attachment to VN-coated TCPS, the enzymes heparinase I, II and III were first used to specifically digest the endogenous cell surface GAG. When used in combination, the enzymes can remove > 90 % of endogenous HS [56]. After digestion, the cells were analyzed by FACS to confirm the absence of surface HS with both the 10E4 and 3G10 antibodies (Fig. 1). The 10E4 antibody binds to intact HS and 3G10 antibody binds to depolymerized HS chains. Before digestion, the cells expressed high levels (> 90 %) of intact 10E4-reactive HS and low levels (< 2 %) of digested 3G10-reactive HS. After heparinase digestion, intact HS chains were removed, so revealing > 95 % 3G10-reactive de-saturated uronic acid residues on cell surfaces (Fig. 1a - d). This confirmed the absence of endogenous HS after heparinase digestion.

The digested cells were then seeded onto VN or VN-HBD surfaces and their attachment assessed. The data clearly showed that the binding ability to this peptide surface was reduced for digested cells but not affected for untreated cells (Fig. 1e). Pre-incubation of cells with soluble heparin also reduce its binding ($\sim$ 40 %), suggesting that heparin was able to compete with endogenous HS in binding to peptide. Taken together, this demonstrated that cells bind to VN-HBD peptide via endogenous HS. In contrast, cells after either treatment did not have their binding reduced on the VN5 surface, similar to untreated cells. This suggested that surface HS was not critical for the binding to full length VN, the binding for which cells mainly utilize $\alpha$V$\beta$5 integrin.

*Isolation of HS9$^{+ve}$ variant*

We next isolated an HS variant with increased VN-binding affinity. To confirm the heparin-binding ability of synthesized biotinylated VN-HBD peptide, a $^3$H-heparin binding assay was first performed. [52]. The data showed that the peptides were able to bind to $^3$H-heparin in a concentration-dependent manner (Fig. 2a), suggesting that this sequence in VN is indeed heparin-binding, and could be used as an affinity chromatography ligand. The column was first saturated with biotin-peptide until excess peptide (green trace) was observed in the flow-through at 280 nm (Fig. 11). The column was equilibrated with low salt buffer in readiness for the loading of the commercial preparation of HS$^{pm}$ (Fig. 2b). Flow-through HS that did not bind to the peptide (blue trace) was designated HS9$^{-ve}$. Bound variants were eluted from the column using a one-step 1.5 M NaCl elution (red trace) and collected as HS9$^{+ve}$ (blue trace) and desalted. From the 100 mg of starting HS$^{pm}$, 19.6 mg (19.6 %) of HS9$^{+ve}$ and 43.4 mg (43.4 %) of HS9$^{-ve}$ were isolated; the rest of the weight was constituted by NaCl. An interesting observation was the presence of a lag time to reach maximum elution of HS9$^{+ve}$ during the high salt wash. This suggested that there is a heterogeneous population of high- to low-binding affinity species comprising HS9$^{+ve}$. The binding ability of the HS9$^{+ve}$ and HS9$^{-ve}$ variants to VN was next determined.

*HS variant characterization*

To further investigate the binding affinity of heparin, starting material (HS$^{pm}$), and the bound and unbound variants, dot blotting, heparin-Sepharose bead assays and GAG microtiter plate assays were conducted. These assays used different strategies to assess their VN binding affinity, either by immobilizing VN or GAG, or a competition for VN binding to heparin beads.

*Dot blotting*

Immunoblotting using nitrocellulose-immobilized VN, followed by biotinylated GAG, was used to verify the binding ability of each HS variant. No non-specific binding of the GAGs was detected, as shown by the absence of spots in the negative control (Fig. 3a). In the positive control, heparin was found to bind strongly to VN, producing an intense spot on the film. HS9$^{+ve}$ bound to VN better than the HS9$^{-ve}$ variant, and HS$^{pm}$ was found to have an intermediate binding ability. This was expected, as we surmised HS$^{pm}$ contained mixtures of positive and negative HS, and thus highly variable degrees of binding ability.

*Heparin-Sepharose bead competition assay*

This assay measures the ability of the HS variants to inhibit the binding of VN to heparin beads. Heparin, having an extreme negative charge, binds to VN with the highest affinity. Thus, the binding ability of heparin for VN was challenged with the different HS variants. To confirm that VN did bind to the heparin beads, and to determine a suitable working concentration, various amounts of VN were utilized (0 - 80 ng) and detected using this ELISA method. The VN saturation curve revealed that it bound in a concentration-dependent manner, with maximal binding occurring at 40 ng. The sub-optimal VN amount identified from the curve was 20 ng (Supplementary Fig. 2a). Absorbance from the VN (20 ng) was then used as the 100 % bound level.

Further assays were done by pre-incubation of VN with soluble heparin (as a positive control) or the HS$^{pm}$, HS9$^{+ve}$ or HS9$^{-ve}$ variants, each at 5, 50 and 100 μg. Results were normalized to the absorbance of 100 % VN-bound beads as measured previously, and plotted as % bound to beads (Fig. 3b). The HS variants that specifically bind to VN competitively inhibit the binding of the VN to the heparin beads. Soluble heparin (100 μg) could almost completely inhibit VN binding (< 10 % binding) to the beads as confirmed by the lack of detection of bound VN. The HS9$^{-ve}$ variant had the weakest binding affinity for VN as shown by the high absorbances detected. Increasing amounts of HS9$^{-ve}$, even to 100 μg, could not inhibit the interactions between VN and the heparin beads. In contrast, with increasing amounts of HS9$^{+ve}$, a concentration-dependent inhibition of VN binding to the beads was observed (~ 10 % bound at 100 μg VN). A moderate binding affinity was detected from HS$^{pm}$, as suggested by the intermediary inhibition (~ 30 % bound at 100 μg VN). Together, these findings suggested that the binding affinity of the HS9$^{+ve}$ variant is higher than the HS9$^{-ve}$ variant, and that HS$^{pm}$ has an intermediate affinity.

To further understand the requirements of HS-VN binding, the ionic strength of the binding buffer was also systemically varied. Immunoblotting results revealed that desalted HS$^{pm}$ could inhibit VN-bead binding, as indicated by the decrease in band intensity. However, NaCl-containing HS$^{pm}$ could not bind to VN, as confirmed by the strong band intensity (Fig. 12d). Thus only physiological buffers such as PBS were used for subsequent binding experiments.

In order to confirm the relative specificity of HS9$^{+ve}$ for VN, its affinity for other ECM proteins (Fibronectin (FN) and Laminin (LN)) was also investigated. The suboptimal working amount of each ECM protein was first predetermined. The saturating binding profiles of FN and LN showed that the sub-optimal amounts were 200 ng and 1 μg respectively (Fig. 12b and c). These amounts were utilized for the subsequent competition assays and for the normalization of competition data to achieve % bound on beads.

Increasing concentrations of soluble HS9$^{+ve}$ variant were used to competitively inhibit the binding of the different ECM proteins to the heparin beads, and the amount of protein left on the beads measured with their respective antibody (Fig. 3c). The HS9$^{+ve}$ variant dose-dependently inhibited the binding of VN to the beads, leading to a lower level of VN detected, but did not inhibit the binding of FN and LN. This was indicated by the lack of dose-dependent decrease in protein bound even at 100 μg of HS9$^{+ve}$, again demonstrating that HS9$^{+ve}$ has a relative specificity for VN. Concurrently, the HS9$^{-ve}$ variant was also used to inhibit VN binding to heparin beads (Fig. 3d). The data showed that HS9$^{-ve}$ variant was able to inhibit FN binding to the beads better than VN or LN. This suggested that that the HS9$^{-ve}$ variant was enriched for FN-binding sequences.

To further confirm that this competitive inhibition, soluble heparin was used as the competitor (Fig. 13). Heparin inhibited all three ECM proteins to varying degrees, with VN inhibition better than FN and LN in binding to heparin beads. Collectively these findings clearly show that the HS9 variants have a graded binding affinity to VN whereby heparin > HS9$^{+ve}$ > HS$^{pm}$ > HS9$^{-ve}$.

*Glycosaminoglycan ELISA*

To provide further validation of HS9 variant binding affinity, an ELISA based on GAG binding to VN was employed; initial experiments were designed to optimize the concentrations of heparin, HS$^{pm}$, HS9$^{+ve}$ and HS9$^{-ve}$ needed to completely saturate the plate surface. Wells were coated with 1, 5 and 10 $\mu$g/ml of each GAG and the binding affinity for VN explored (Fig. 14). The data revealed that 5 $\mu$g/ml of a GAG solution was sufficient to completely saturate the wells; this saturating concentration was therefore used for the rest of the experiments.

GAGs were then coated overnight and investigated for their binding affinity to VN (Fig. 3e). The binding curve showed that, irrespective of GAG, there was a dose-dependent increase in VN binding. HS9$^{+ve}$ had a significantly higher affinity for VN than starting material HS$^{pm}$ and flow-through HS9$^{-ve}$. Heparin, being the most negatively charged variant, was bound to VN at significantly higher levels than the rest. This reinforced the results of the heparin bead competition assay.

Because HS binding is in part the result of negatively charged sulfate residues along the disaccharide chain, understanding the structural composition of HS variants that interact with VN is key. Selectively desulfated heparin standards were immobilized onto ELISA surfaces (Fig. 3f). Heparin that had been selectively de-N-sulfated or de-6-O-sulfated was found to have significantly reduced levels of binding to VN. In contrast, chains lacking 2-O sulfation were unaffected, and strongly bound to VN in a manner comparable to that of intact heparin. In analogous experiments, heparin standards of varying length, from 2 disaccharides (degree of polymerization (dp2)) to 12 disaccharides (dp12), were used to look for the minimum size needed for VN binding (Fig. 3g). There was a significant lack of binding to the dp2 and dp4 chains. For chains with more than 3 repeating units, there was a generally similar binding affinity for VN. These results strongly suggest therefore that N- and 6-O-sulfation on chains of at least 3 repeating disaccharide units are necessary for VN binding to heparin. This conclusion may also be valid for HS.

*Capillary electrophoresis*

This technique separates individual disaccharides based on size and charge. The separation of 7 heparin disaccharide standards was completed, and is depicted in Figure 4a. A-disaccharide standard IS designation represents $\Delta$ UA2S(1→4)-D-GlcNS6S containing 2O-, 6O- and N-sulfation; IIS represents $\Delta$ UA(1→44)-D-GlcNS6S containing 6O- and N-sulfation; IIIS represents $\Delta$ UA2S(1→4)-D-GlcNS containing 2O- and N-sulfation; IVS represents $\Delta$ UA(1→4)-D-GlcNS containing only N-sulfation; IA represents $\Delta$ UA2S(1→4)-D-GlcNAc6S containing 2O- and 6O- sulfation; IIA represents $\Delta$ UA(1→4)-D-GlcNAc6S containing only 6O-sulfation and IIIA represents $\Delta$ UA2S(1→4)-O-GlcNAc containing only 2O-sulfation. Elemental structure is represented in Ruiz-Calero *et al* [57].

The last peak (IVA) could not be detected, even when the recommended 0.5 per square inch (p.s.i.) pressure gradient was applied for a further 30 min after completion of the run. Thus IVA is omitted from this study. The observed differences in the detection times from Ruiz-Calero et *al.* might be due to the different equipment used, or the inability to achieve 0.5 p.s.i.; nevertheless the peaks were well separated from each other. An internal standard was added to assist in the identification of the various peaks in the digested samples.

To achieve reproducible results and account for the variations in the samples, 5 replicates of the A-disaccharide standards were separated. The migration time and peak areas were expressed as relative standard deviation (R.S.D.) (Fig.18). R.S.D. was considered reliable when area standard deviations were < 5 %, and migration time standard deviations of < 1 % were achieved. The measurement standard (R.S.D.) allowed for the generation of calibration curves (goodness-of-fit ($R^2$) > 0.99) for each $\Delta$-disaccharide standard from the individual peak area.

Electropherogram profiles of depolymerized heparin, HS$^{pm}$, HS9$^{+ve}$ or HS9$^{-ve}$ variants were next generated. Before CE analysis, the depolymerization of each HS variant was monitored at 232 nm. The undigested samples had an absorbance of 0.01 and increased to ∼ 1.1, and when no further increase was seen, the depolymerization was be deemed to be complete. Electropherograms showing each sample profile are shown in Figure 4b to e. Three replicates were run and average areas-under-the-peak calculated and compared to the standard curve. The identity of each peak was determined from both the relative shift from the internal standard added into the mixture, and the migration time of each peak. Due to the almost undetectable peak for IIIA, its identity was confirmed by adding IIIA $\Delta$-disaccharide standard into the depolymerized HS sample.

A comparison of the disaccharide composition of each digested HS variant is shown in the Table in Figure 9. The results revealed that the major units in heparin are the trisulfated (2S, 6S and NS) IS and disulfated (6S and NS) IIS at 66.1% and 20.8 % respectively. In contrast, the major units in HS$^{pm}$ are the monosulfated (NS) IVS and disulfated (6S and NS) IIS (36.8 % and 23.7 % respectively). The large decrease in trisulfated (2S, 6S and NS) IS observed in HS$^{pm}$ is likely because of the lower sulfation of HS. It was also observed that, after the affinity chromatography step, the HS9$^{+ve}$ variant was enriched for trisulfated (2S, 6S and NS) IS and disulfated (6S and NS) IIS (26.0 % and 30.6 % each), whereas the HS9$^{-ve}$ variant most prominently possessed monosulfated (NS) IVS (33.3 %).

Clearly the most notable point is the presence of the higher proportions of the trisulfated IS and disulfated IIS A-motifs in HS9$^{+ve}$ than in HS9$^{-ve}$. This clearly suggests that a combination of 6O- and N-sulfation is very important for HS binding to VN, a result also supported by the GAG-ELISA (Fig. 3f). Lack of enrichment of monosulfated (NS) IVS and monosulfated (6S) IIA in HS9$^{+ve}$ variants as compared to starting HS$^{pm}$ material suggests that neither domain alone are sufficient for VN binding. In contrast, 2O-sulfation does not seem so essential for the binding of HS to VN, as evidenced by both the previous GAG ELISA, and the lack of enrichment of the monosulfated (2S) IIIA, disulfated (2S, 6S) IA and disulfated (2S and NS) IIIS Δ-disaccharide units in HS9$^{+ve}$.

Collectively, the results in this section demonstrate that the highly 6O- and N-sulfated HS9$^{+ve}$ variant isolated by VN affinity chromatography has a higher capacity for VN than either the HS$^{pm}$ starting material or the HS9$^{-ve}$ flow-through. It also has a greater capacity to bind VN than either LN or FN, suggesting that the specificity of the HS9$^{+ve}$ has increased.

*Immobilization of HS9 variants*

Strategies to immobilize HS9$^{+ve}$ efficiently onto surfaces for the presentation of unmodified VN for cell culture using covalent and electrostatic methods were next explored.

*Covalent EDC chemistry*

NaOH was next utilized to etch surfaces to create free carboxyl groups on low carboxylated polystyrene (PS) surfaces. The coupling of the primary amine groups in HS onto surfaces was accomplished with EDC chemistry (Fig. 5a). It was previously shown by Plante *et al.* that EDC chemistry could be used to tether disaccharide units through their free amine ends to surface carboxyl groups [42].

Optimization of EDC concentrations (10, 50 and 100 mg/ml) was first required. $^{3}$H-lysine was used, because every molecule contains 2 primary amines for coupling, so that the amine will not be the limiting factor. The results demonstrated that 24 h of NaOH treatment was sufficient to etch the maximum levels of the carboxyl groups on PS surfaces. However, TCPS yielded better grafting than NaOH-treated PS; thus TCPS was used for the subsequent reactions. A concentration-dependent increase in grafting of $^{3}$H-lysine was observed with 10 and 50 mg/ml. However, no significant increase from 50 to 100 mg/ml of EDC was observed, suggesting that the grafting concentration saturated at 50 mg/ml.

Another parameter that required consideration was the amount of primary amines in heparin and HS$^{pm}$. To confirm these levels, a fluorescamine protein assay was employed (Fig. 15) with two concentrations of each GAG (0.5 and 1 mg/ml). There are more (> 60 %) primary amines present in HS$^{pm}$ than in heparin. By comparing 1 mg/ml heparin and the HS$^{pm}$ variant, it was shown that there were $3 \times 10^{16}$ amines present in heparin and $5 \times 10^{14}$ amines present in HS$^{pm}$, a difference of 60 %. This difference was less (40 %) in heparin compared to HS9 variants ($2 \times 10^{16}$ amines), suggesting some compositional differences in both of them after affinity chromatography, confirming the data obtained previously with CE. Thus, the number of amines in GAGs directly affects the EDC grafting efficiency. $^{3}$H-heparin and $^{3}$H-Hs$^{pm}$ were then grafted onto TCPS surfaces (Fig. 5b). Concentration-dependent increases in $^{3}$H-GAG binding were observed, with the surface density of $^{3}$H-HS$^{pm}$ notably higher than $^{3}$H-heparin at all concentrations tested. This was expected, because heparin has > 80 % of its amino groups as N-sulfates and the number of free amines is lower than in HS [23].

Although this method does immobilize GAGs onto surfaces, the overall grafting is inefficient. With the use of 30 mg/ml of $^{3}$H-GAG at 3 mg per well (0.32 cm$^2$), only ~ 1.5 μg of $^{3}$H-heparin and ~ 6 μg of $^{3}$H-HS$^{pm}$ were detectable on the surfaces, which equates to a 0.05 % and 0.2 % grafting efficiency respectively. Moreover, a study by Roy *et al.* has shown that covalently binding GAGs to surfaces carries significant disadvantages. In particular, utilizing the N-domains for coupling compromises the biological activities of GAGs [58]. Therefore, better and more efficient strategies were sought.

*Poly-L-lysine coating*

Strategies exploiting the electrostatic interactions between the negatively charged HS and positively charged surfaces were next employed. Poly-L-lysine (PLL) has been used successfully as a substrate for many types of stem cells, but not for hESCs on TCPS [59-61]. Therefore to test such surfaces, PLL-coated TCPS plates were subjected to overnight coating with different concentrations of $^{3}$H-heparin and $^{3}$H-HS$^{pm}$ (Fig. 5c). The surface density of each GAG was then determined: 1 μg of $^{3}$H-GAG solution yielded a surface density of ~ 200 ng/cm$^2$, with density increasing to ~ 800 ng/cm$^2$ and 400 ng/cm$^2$ respectively at 2 μg of $^{3}$H-heparin and $^{3}$H-HS$^{pm}$. The higher density of $^{3}$H-heparin observed was due to its higher overall negative charge. Interestingly, differences in surface density were observed in heparin and HS$^{pm}$ only at 2 μg, suggesting an inferior binding of GAGs onto PLL surfaces at lower GAG concentrations. Therefore, 2 μg was subsequently used for the immobilization of GAGs on PLL surfaces.

To further analyze the utility of the PLL surface for cell culture, hESCs (HES-3) were screened for cell proliferation on PLL-coated GAG (PLL+GAG) surfaces (Fig. 5d). VN concentrations of 2.5 µg/ml (VN2.5) and 5 µg/ml (VN5) were coated onto PLL+GAG surfaces, and HES-3 cells seeded and allowed to grow for 7 days. Photomicrographs after a week revealed that the cells had not spread well on these surfaces, with the exception of those on PLL+heparin+VN5. This suggested that PLL-coated surfaces are not optimal for hESC culture.

Allylamine polymerization

As the previous two methods failed to address key requirements (cost, simplicity, safety and efficacy) essential to an engineered substrate, another surface was clearly needed to immobilize GAGs for long-term cell propagation. We have previously reported that plasma polymerization of allylamine (AA) monomers onto plastic surfaces is able to immobilize GAG effectively [47]. The AA monomer is positively charged, and, when polymerized onto surfaces, gives the surface a net positive charge that persists over time [62].

To assess the optimal AA density for immobilizing HS on surfaces, different percentages of AA surfaces were generated to determine which density has the highest functional binding ability for GAGs, as assessed by ELISA. Surface densities were controlled with a neutral octa-1, 7-diene monomer. Density varied from 0% AA: 100% octa-1, 7-diene, 50% AA: 50% octa-1, 7-diene, 80% AA: 20% octa-1, 7-diene, 90% AA: 10% octa-1, 7-diene to 100% AA: 0% octa-1, 7-dlene. The results revealed that the 100% AA surfaces bound the highest amount of functional GAG, followed by the 90% AA surface (Fig. 16). It was interesting to note that the GAGs bound to 80 % AA surface no longer retained their capability to bind VN, instead having a negative effect. This was almost certainly due to the high background observed in the blank wells, which may have been because of the ineffective blocking of the fish gelatin. The 50% AA surface could not trigger any VN binding, suggesting that insufficient GAG was immobilized by it onto the surface. Thus, the 100% AA polymerized surface gave by far the best binding, and was used for all further analysis.

In order to confirm the presence of AA polymer on the surface, an XPS analysis was performed to determine the oxygen (O), nitrogen (N) and carbon (C) content of the AA polymerized aluminium foil surfaces placed together in the plasma reactor chamber. The readings on the foil reflected the amount of each atom on the plate surface [47]. The 50% AA surface gave readings of C (92.5%), N (4.56 %) and O (2.93 %); the 80% AA surface produced C (85.2 %), N (12.14 %) and O (2.7 %); the 90 % AA surface produced C (80.7 %), N (15.3 %) and O (4 %) (Fig. 17); the 100% AA surface had C (79.2 %), N (16.4 %) and O (4.34 %) (Fig. 6a). A consistent nitrogen: carbon (N: C) ratio of 0.18 to 0.22 was observed for several batches of the 100% AA plates (Fig. 10). This all demonstrated the reproducibility of the plasma polymerization reaction, which was also consistent with our previous studies [46, 47]. Thus an N: C ratio of 0.18 to 0.22 was optimal for functional HS9 binding, and was used subsequently for immobilization of unmodified VN.

Following the success of HS immobilization, the ability of each GAG variant to bind VN was assessed by ELISA (Fig. 6b). Irrespective of the GAG, there was a concentration-dependent increase in absorbance. Wells without GAG served as the negative control, indicating no non-specific interactions. Binding data revealed that the heparin and HS9[+ve] variants had the highest affinity for VN, and that the flowthrough HS9[-ve] variant and starting HS[pm] had the weakest. Moreover, the HS9[+ve] variant produced a higher absorbance than the HS9[-ve] variant, indicating a significant higher affinity for VN. This result is similar to that which employed the GAG-ELISA (Fig. 3e), suggesting the higher binding affinity of the HS9[+ve].

To confirm the presence of GAGs on the AA surfaces, the surface density of $^3$H-heparin and $^3$H-HS[pm] was determined. $^3$H-GAG was immobilized onto the surface overnight, washed and read in a scintillation counter (Fig. 6c). There was a concentration-dependent increase in surface density, with a higher density seen for heparin than for HS[pm]. When 1 µg of GAG was used for coating, the $^3$H-heparin yielded a surface density of ~ 250 ng/cm$^2$; $^3$H-HS[pm] yielded only ~ 100 ng/cm$^2$. This corresponds to immobilization efficiencies of ~ 8 % and ~ 3.2 % respectively, which was a significant increase over the use of covalent EDC immobilization chemistry. It was expected that heparin would bind better to the surfaces because of its higher density of negative charge per unit length. Thus, this simple and robust AA+GAG surface was used for all further immobilization of VN.

*$^{125}$I-VN surface density*

Our previous study [18] demonstrated that the threshold density for successful maintenance of hESCs on VN-adsorbed TCPS surfaces is 250 ng/cm$^2$. The question now became whether an HS with tuned affinity for VN can be used as a substrate to capture and present VN in an efficient way. The surface densities of VN on TCPS, AA+HS9[+ve] and PLL+HS9[+ve] substrates were measured and compared. Increasing concentrations (0, 1.25, 2.5 and 5 µg/ml) of $^{125}$I-VN were incubated on the different surfaces, and the amounts measured by scintillation and compared to a standard curve (Fig. 6d). All surfaces showed a concentration-dependent increase in binding of the $^{125}$I-VN, with the lowest VN surface density recorded on the PLL+HS9[+ve] surface and the highest on TCPS surface. There was insufficient VN on the surface for cells to attach and proliferate on the PLL+HS9[+ve] surface, presumably explaining the HES-3 cell response seen in Figure 5d.

*hESC culture*

To determine the suitability of the substrate coating that consisted of AA+GAG+VN for serial culturing, hESC (HES-3) growth was assessed over a 7 day period. Surfaces that were pre-coated with AA and GAGs (heparin, HS$^{pm}$, HS9$^{+ve}$ and HS9$^{-ve}$) were used to immobilize 2 $\mu$g/ml of VN solution concentration (VN2). Photomicrographs taken after 7 days revealed that cell attachment and proliferation on VN2 was only supported with the heparin and HS9$^{+ve}$ pre-coatings (Fig 7a and c). Clearly, higher amounts of VN were adsorbed on the heparin and HS9$^{+ve}$ substrates as compared to the HS$^{pm}$ and HS9$^{-ve}$ substrates (Fig. 7b and d). This bioassay confirmed that the affinity chromatography was able to separate 'tune' HS with a higher VN-binding affinity from low binding HS9$^{-ve}$ and medium binding HS$^{pm}$. As heparin binds strongly to a wide range of ECM proteins, it was utilized as a positive control for the experiments. Although heparin can support HES-3 cells at low VN concentration (VN2), it is not a suitable pre-coating for future therapy because of its adverse clinical side effects [23].

According to the VN surface densities identified with $^{125}$I-VN (Fig. 6d), the densities on AA+Heparin+VN2 and AA+HS9$^{+ve}$+VN2 were much lower than the VN threshold (250 ng/cm$^2$) that we had reported previously. This suggested that with the pre-coating of HS9$^{+ve}$ fractions, we are able to reduce the VN density needed for hESC proliferation. However, we observed that cells attached on AA+HS9$^{+ve}$+VN2 substrate showed signs of weak attachment, as revealed by the rolling of cells at the edges of colonies. Therefore a follow-up study to evaluate the proper surface density of VN on AA+HS9$^{+ve}$ substrate that can robustly support long-term hESCs culture is needed.

A schematic representation of this layer-by-layer model was depicted in Figure 8. In conclusion, the cell culture results reiterate the contention that this novel engineered AA+HS9$^{+ve}$+VN substrate is able to capture sufficient VN for the culture of hESC.

Discussion

The isolation of a VN-binding HS variant via VN-HBD peptide affinity chromatography has allowed us to engineer a novel hESC culture platform. The binding ability of the different variants was compared using a combination of dot blot, ELISA and heparin bead competition assays. To check the apparent specificity of the HS9$^{+ve}$ variant for VN binding, it was compared to the binding of LN and FN. Together these results confirmed that the HS9$^{+ve}$ variant binds VN with relative specificity and certainly better than the HS$^{pm}$ and HS9$^{-ve}$ variants. This result suggests the possibility of isolating a library of HS variants tuned to other ECM proteins for generating a range of specific substrates. A modification to the elution buffer during affinity chromatography, involving a step-wise elution with a different molarity buffer to separate the different species in HS9$^{+ve}$ is a future possibility.

Glycosaminoglycans are an important structural and functional component of the ECM [23]. One of most abundant GAGs on stem cell surfaces is HS, with CS predominant in the ECM of mature bone, cartilage and heart valve [23]. Previously studies have shown that cell surface HSPGs are crucial for cell adhesion to a FN heparin-binding peptide, as shown after soluble heparin and heparinase treatment [22, 63]. Thus, to extend these findings, heparinase digestion of surface HS was employed to determine its importance for cell binding to VN-HBD. The results showed that cells could bind to VN-HBD peptide via surface HS, but that they are not critical if the RGD motif is present. Several studies have also shown that heparin and HS are able to support hESC self-renewal and growth [7, 64]. Following this, we aimed to isolate a high affinity VN binding HS variant from a mixture, to present VN efficiently.

Affinity-separated heparin variants have been isolated before, but by employing whole native protein during the affinity step. Thus, heparin variants with high affinity to fibronectin [21], heparin co-factor II [65], tissue plasminogen activator [66], FGF-1 [33, 67] and anti-thrombin III [68, 69] have been isolated. However, the use of whole protein is both impractical and costly, especially at this scale. McCaffrey and colleagues subsequently demonstrated that two variants, with high and low TGF-$\beta$ binding affinity, could be isolated from heparin mixtures with a synthetic peptide that mimicked the TGF-$\beta$ HBD [70]. Their study used heparin as the starting material, a compound not suitable for tissue regeneration. Understanding the composition of the variants is clearly important for the future design of a synthetic HS9 mimic. As evidenced by the low R.S.D. values with all the depolymerized samples, the compositional analysis of heparin and HS can be considered reliable. The percentages of disaccharides in heparin were consistent with those published by other groups [32, 57, 71-74]. The molar percentages of the major disaccharides in porcine intestinal mucosa heparin, IS and IIS, range from 50 % to 68 % and from 10 % to 20 % respectively. No compositional studies have been done with the porcine mucosa HS; however, a previous comparison between HS from other sources such as bovine kidney revealed a similar trend, with the monosulfated IVS disaccharide unit having a higher molar percent ratio compared to the other units [34, 71].

The elucidation of the anti-coagulant pentasaccharide sequence in heparin revealed that 3O-sulfation is essential for its anti-coagulant effects [75]; in a similar manner, FGF-2 binding to HS requires 2O-sulfation [76, 77] whereas 6O- and N- sulfation tend to impair binding [78]; FGF-1 interaction with HS in contrast requires the 6O-sulfate group [79]. FGF-4 requires both 2O- and 6O-sulfation for binding and signalling [80, 81] and hepatocyte growth factor requires 6O-sulfation [82]. Falcone *et al*. and Mahalingam *et al.* demonstrated that an avid heparin-binding variant selective for FN appeared particularly highly charged, with 7 to 8 N-sulfated disaccharides being required, and a larger domain (> 14 residues) than unfractionated heparin [21, 22]. Ling *et al*. have also shown that N-sulfation in heparin contributes to the binding and activity of Wnt3a ligands for its osteogenic activity [83]. These studies support the idea that differentially modified HS motifs confer distinct protein recognition properties on HS. The GAG ELISA and CE analysis both directly and indirectly revealed that the 6O- and N-sulfation of the glucosamine residue, and that at least 3 disaccharide units, are required for VN binding to HS. Removal of N-sulfation from the heparin standards also reduced the level of binding to VN, indicating that N-sulfation together with 60-sulfation is crucial. This is the first report of an essential sulfation motif within HS important for VN binding.

In the search for an effective and efficient way to immobilize HS9$^{+ve}$ variants onto TCPS, the results demonstrated that the low proportion of primary amines in HS renders the EDC coupling method inefficient. This also accounts for the lack of proliferation on VN-coated PLL surfaces revealed in the $^{125}$I-VN binding assay. The low VN surface density on PLL surfaces was also not sufficient to support robust culture of hESCs. As shown previously, the minimum VN surface density required on TCPS is at least 250 ng/cm$^2$ [18]. Building upon our previous report of using a simple non-covalent method (AA plasma polymerization) to immobilize GAGs [47], we then selected this method for the immobilization of HS9$^{+ve}$ variant. AA-polymerized cell culture surfaces have been studied by Punzon-Quijorna *et al*. and Schroder *et al*. to help culture mesenchymal stem cells on both polycaprolactone (PCL) [84] and titanium surfaces [85]. Although AA surfaces have been used for cell culture, this study is particularly interested in the culture of hESCs, which are known to be very demanding in their choice of substrate.

Concerning VN contains a similar number of negatively (66) and positively (56) charged residues (calculated from data on the ExPASy website, www.expasy.com), which might explain why VN binds to both the negatively- and positively- charged surfaces. Thus we posit that the hydrophilic, net negatively charged TCPS surface is favourable for VN binding, while the highly positively charged AA surface supports more VN binding than PLL. PLL might not uniformly coat the surface, so that less positive charge is deposited, explaining the lower VN density. Such patchy coating might also translate into an inability of stem cells to proliferate on the PLL+HS9$^{+ve}$ surfaces.

Heparin has been recognised for playing roles in regulating self-renewal of hESC and is an important component of the microenvironment [39]. Importantly however, by using a less sulphated, VN- tuned HS, we are able to better support hESC attachment and proliferation at a lower VN density compared to 250 ng/cm$^2$ reported previously [18].

This technology, of first obtaining a 'tuned' HS variant, isolated from a heterogeneous population of HS by affinity binding to a HBD-VN peptide, clearly demonstrates its applicability for the isolation of other 'tuned' HS variants aimed at specific ECM components. These can then be used to study the mechanisms that are responsible for the proliferation and differentiation of hESCs in a compliant environment.

The aim of this research was to develop a substrate capable of binding unmodified VN based through its affinity for heparan glycosaminoglycans. An avid VN-binding variant (HS9$^{+ve}$) derived from HS$^{pm}$ was isolated using affinity chromatography. Comparison of the HS9$^{+ve}$, HS9$^{-ve}$ and HS$^{pm}$ variants revealed that the HS9$^{+ve}$ variant had a higher binding propensity for VN, suggesting that affinity chromatography is a powerful technique for the separation of active GAG variants tuned to specific adhesive proteins. Compositional analysis with CE confirmed an enrichment of trisulfated (2S, 6S and NS) IS and disulfated (6S and NS) IIS disaccharides in the HS9$^{+ve}$ variant. Together with the GAG-ELISA results, it can thus be concluded that 6O-sulfation together with N-sulfation on glucosamine residue and at least 3 disaccharide units are critical for HS9$^{+ve}$ binding to VN. AA-plasma polymerized surfaces were able to bind to functional GAG, which in turn was able to bind sufficient amounts of VN for successful cell culture. In summary, this research established the process development of a robust, simple and cost effective substrate not only for the presentation of unmodified VN, but for any ECM component with an HBD, for cell culture.

## References

[1] Boston CG. High potential diagnosis in stem cell research. http://wwwslidesharenet/MedicineAndHealth14/stem-cells-sweden. 2001.

[2] Summit Sc. http://www.stemcellsummit.com/2007/stem-cell-fact-sheet.pdf. 2007.

[3] Goldring CEP, Duffy PA, Benvenisty N, Andrews PW, Ben-David U, Eakins R, et al. Assessing the Safety of Stem Cell Therapeutics. Cell Stem Cell. 2011;8:618-28.

[4] Alper J. Geron gets green light for human trial of ES cell-derived product. Nat Biotechnol. 2009;27:213-4.

[5] Schwartz SD, Hubschman JP, Heilwell G, Franco-Cardenas V, Pan CK, Ostrick RM, et al. Embryonic stem cell trials for macular degeneration: a preliminary report. Lancet. 2012;379:713-20.

[6] Li Y, Powell S, Brunette E, Lebkowski J, Mandalam R. Expansion of human embryonic stem cells in defined serum-free medium devoid of animal-derived products. Biotechnol Bioeng. 2005;91:688-98.

[7] Furue MK, Na J, Jackson JP, Okamoto T, Jones M, Baker D, et al. Heparin promotes the growth of human embryonic stem cells in a defined serum-free medium. Proc Natl Acad Sci U S A. 2008;105:13409-14.

[8] Ludwig TE, Levenstein ME, Jones JM, Berggren WT, Mitchen ER, Frane JL, et al. Derivation of human embryonic stem cells in defined conditions. Nat Biotechnol. 2006;24:185-7.

[9] Wang L, Schuiz TC, Sherrer ES, Dauphin DS, Shin S, Nelson AM, et al. Self-renewal of human embryonic stem cells requires insuhn-like growth factor-1 receptor and ERBB2 receptor signaling. Blood. 2007;110:4111-9.

[10] Rodin S, Domogatskaya A, Strom S, Hansson EM, Chien KR, Inzunza J, et al. Long-term self-renewal of human pluripotent stem cells on human recombinant laminin-511. Nat Biotechnol. 2010;28:611-U102.

[11] Xu C, Inokuma MS, Denham J, Golds K, Kundu P, Gold JD, et al. Feeder-free growth of undifferentiated human embryonic stem cells. Nat Biotechnol. 2001;19:971-4.

[12] Braam SR, Zeinstra L, Litjens S, Ward-van Oostwaard D, van den Brink S, van Laake L, et al. Recombinant vitronectin is a functionally defined substrate that supports human embryonic stem cell self-renewal via alphavbeta5 integrin. Stem Cells. 2008;26:2257-65.

[13] Amit M, Margulets V, Segev H, Shariki K, Laevsky I, Coleman R, et al. Human feeder layers for human embryonic stem cells. Biol Reprod. 2003;68:2150-6.

[14] Nagaoka M, Si-Tayeb K, Akaike T, Duncan SA. Culture of human pluripotent stem cells using completely defined conditions on a recombinant E-cadherin substratum. BMC Dev Biol. 2010;10:12.

[15] Klim JR, Li LY, Wrighton PJ, Piekarczyk MS, Kiessling LL. A defined glycosaminoglycan-binding substratum for human pluripotent stem cells. Nat Methods. 2010;7:989-U72.

[16] Melkoumian Z, Weber JL, Weber DM, Fadeev AG, Zhou Y, Dolley-Sonneville P, et al. Synthetic peptide-acrylate surfaces for long-term self-renewal and cardiomyocyte differentiation of human embryonic stem cells. Nat Biotechnol. 2010;28:606-U95.

[17] Villa-Diaz LG, Nandivada H, Ding J, Nogueira-De-Souza NC, Krebsbach PH, O'Shea KS, et al. Synthetic polymer coatings for long-term growth of human embryonic stem cells. Nat Biotechnol. 2010;28:581-3.

[18] Yap LYW, Li J, Phang IY, Ong LT, Ow JaZ-E, Goh JCH, et al. Defining a Threshold Surface Density of Vitronectin for the Stable Expansion of Human Embryonic Stem Cells. Tissue Engineering Part C-Methods. 2011;17:193-207.

[19] Marson A, Rock MJ, Cain SA, Freeman LJ, Morgan A, Mellody K, et al. Homotypic fibrillin-1 interactions in microfibril assembly. J Biol Chem. 2005;280:5013-21.

[20] Lindahl U, Backstrom G, Hook M, Thunberg L, Fransson LA, Linker A. Structure of the antithrombin-binding site in heparin Proc Natl Acad Sci U S A. 1979;76:3198-202.

[21] Falcone DJ, Salisbury BGJ. Fibronectin stimulates macrophage uptake of low-density lipoprotein-heparin-collagen complexes Arteriosclerosis. 1988;8:263-73.

[22] Mahalingam Y, Gallagher JT, Couchman JR. Cellular adhesion responses to the heparin-binding (HepII) domain of fibronectin require heparan sulfate with specific properties. J Biol Chem. 2007;282:3221-30.

[23] Gandhi NS, Mancera RL. The Structure of Glycosaminoglycans and their Interactions with Proteins. Chem Biol Drug Des. 2008;72:455-82.

[24] Venkataraman G, Shriver Z, Raman R, Sasisekharan R. Sequencing complex polysaccharides. Science. 1999;286:537-42.

[25] Desai UR, Wang HM, Linhardt RJ. Specificity studies on the heparin lyases from Flavobacterium-heparinum Biochemistry. 1993;32:8140-5.

[26] Shriver Z, Sundaram M, Venkataraman G, Fareed J, Linhardt R, Biemann K, et al. Cleavage of the antithrombin III binding site in heparin by heparinases and its implication in the generation of low molecular weight heparin. Proc Natl Acad Sci U S A. 2000;97:10365-70.

[27] Karamanos NK, Vanky P, Tzanakakis GN, Tsegenidis T, Hjerpe A. Ion-pair high-performance liquid chromatography for determining disaccharide composition in heparin and heparan sulphate. J Chromatogr A. 1997;765:169-79.

[28] Vynios DH, Karamanos NK, Tsiganos CP. Advances in analysis of glycosaminoglycans: its application for the assessment of physiological and pathological states of connective tissues. J Chromatogr B. 2002;781:21-38.

[29] Hampson IN, Gallagher JT. Separation of radiolabeled glycosaminoglycan oligosaccharides by polyacrylamide-gel electrophoresis Biochem J. 1984;221:697-705.

[30] Skidmore M AA, Yates E and Turnbull JE. Labelling heparan sulfate saccharides with chromophore, fluorescence and mass tag for HPLC and MS separations. Methods in Molecular biology. 2009;534:157-69.

[31] Lamari F, Militsopoulou M, Gioldassi X, Karamanos NK. Capillary electrophoresis: a superior miniaturized tool for analysis of the mono-, di-, and oligosaccharide constituents of glycan moieties in proteoglycans. Fresenius J Anal Chem. 2001;371:157-67.

[32] Karamanos NK, Vanky P, Tzanakakis GN, Hjerpe A. High performance capillary electrophoresis method to characterize heparin and heparan sulfate disaccharides. Electrophoresis. 1996;17:391-5.

[33] Sudhalter J, Folkman J, Svahn CM, Bergendal K, Damore PA. Importance of size, sulfation, and anticoagulant activity in the potentiation of acidic fibroblast growth-factor by heparin J Biol Chem. 1989;264:6892-7.

[34] Militsopoulou M, Lamari FN, Hjerpe A, Karamanos NK. Determination of twelve heparin- and heparan sulfate-derived disaccharides as 2-aminoacridone derivatives by capillary zone electrophoresis using ultraviolet and laser-induced fluorescence detection. Electrophoresis. 2002;23:1104-9.

[35] Bramono DS, Rider DA, Murali S, Nurcombe V, Cool SM. The Effect of Human Bone Marrow Stroma-Derived Heparan Sulfate on the Ex Vivo Expansion of Human Cord Blood Hematopoietic Stem Cells. Pharm Res. 2011;28:1385-94.

[36] Levenstein ME, Berggren WT, Lee JE, Conard KR, Llanas RA, Wagner RJ, et al. Secreted Proteoglycans Directly Mediate Human Embryonic Stem Cell-Basic Fibroblast Growth Factor 2 Interactions Critical for Proliferation. Stem Cells. 2008;26:3099-107.

[37] Nurcombe V, Cool SM. Heparan sulfate control of proliferation and differentiation in the stem cell niche. Crit Rev Eukaryot Gene Expr. 2007;17:159-71.

[38] Helledie T, Dombrowski C, Rai B, Lim ZX, Hin IL, Rider DA, et al. Heparan sulfate enhances the self-renewal and therapeutic potential of mesenchymal stem cells from human adult bone marrow. Stem Cells Dev. 2012;21:1897-910.

[39] Uygun BE, Stojsih SE, Matthew HWT. Effects of Immobilized Glycosaminoglycans on the Proliferation and Differentiation of Mesenchymal Stem Cells. Tissue Eng Part A. 2009;15:3499-512.

[40] Nishioka S, Aikawa J, Ida M, Matsumoto I, Street M, Tsujimoto M, et al. Ligand-binding activity and expression profile of annexins in Caenorhabditis elegans. J Biochem. 2007;141:47-55.

[41] Feng WY, Zhao LH, Wang KY. Interaction of polysaccharides with interferon-gamma using an improved ELISA approach. Carbohydrate Polymers. 2004;58:89-94.

[42] Plante OJ, Palmacci ER, Seeberger PH. Automated solid-phase synthesis of oligosaccharides. Science. 2001;291:1523-7.

[43] Foxall C, Holme KR, Liang WH, Wei Z. An enzyme-linked-immunosorbent-assay using biotinylated heparan-sulfate to evaluate the interactions of heparin-like molecules and basic fibroblast growth-factor Anal Biochem. 1995;231:366-73.

[44] Hasan M, Najjam S, Gordon MY, Gibbs RV, Rider CC. IL-12 is a heparin-binding cytokine. J Immunol. 1999;162:1064-70.

[45] Parkar AA, Day AJ. Overlapping sites on the Link module of human TSG-6 mediate binding to hyaluronan and chondroitin-4-sulphate. FEBS Lett. 1997;410:413-7.

[46] Mahoney DJ, Whittle JD, Milner CM, Clark SJ, Mulloy B, Buttle DJ, et al. A method for the non-covalent immobilization of heparin to surfaces. Anal Biochem. 2004;330:123-9.

[47] Robinson DE, Buttle DJ, Short RD, McArthur SL, Steele DA, Whittle JD. Glycosaminoglycan (GAG) binding surfaces for characterizing GAG-protein interactions. Biomaterials. 2012;33:1007-16.

[48] Suzuki S, Pierschbacher MD, Hayman EG, Nguyen K, Ohgren Y, Ruoslahti E. Domain-structure of vitronectin - Alignment of active-sites J Biol Chem. 1984;259:5307-14.

[49] Watanabe K, Ueno M, Kamiya D, Nishiyama A, Matsumura M, Wataya T, et al. A ROCK inhibitor permits survival of dissociated human embryonic stem cells. Nat Biotechnol. 2007;25:681-6.

[50] van den Born J, Salmivirta K, Henttinen T, Ostman N, Ishimaru T, Miyaura S, et al. Novel heparan sulfate structures revealed by monoclonal antibodies. J Biol Chem. 2005;280:20516-23.

[51] David G, Bai XM, Vanderschueren B, Cassiman JJ, Vandenberghe H. Developmental-changes in heparan-sulfate expression - insitu detection with mAbs J Cell Biol. 1992;119:961-75.

[52] Baird A, Schubert D, Ling N, Guillemin R. Receptor-binding and heparin-binding domains of basic fibroblast growth-factor Proc Natl Acad Sci U S A. 1988;85:2324-8.

[53] Ono K, Hattori H, Takeshita S, Kurita A, Ishihara M. Structural features in heparin that interact with VEGF(165) and modulate its biological activity. Glycobiology. 1999;9:705-11.

[54] Hambardzumyan A, Biltresse S, Dufrene Y, Marchand-Brynaert J. An unprecedented surface oxidation of polystyrene substrates by wet chemistry under basic conditions. J Colloid Interface Sci. 2002;252:443-9.

[55] Robinson DE, Marson A, Short RD, Buttle DJ, Day AJ, Parry KL, et al. Surface gradient of functional heparin. Advanced Materials. 2008;20:1166-+.

[56] Manton KJ, Leong DF, Cool SM, Nurcombe V. Disruption of heparan and chondroitin sulfate signaling enhances mesenchymal stem cell-derived osteogenic differentiation via bone morphogenetic protein signaling pathways. Stem Cells. 2007;25:2845-54.

[57] Ruiz-Calero V, Puignou L, Galceran MT. Use of reversed polarity and a pressure gradient in the analysis of disaccharide composition of heparin by capillary electrophoresis. J Chromatogr A. 1998;828:497-508.

[58] Roy S, Lai HC, Zouaoui R, Duffner J, Zhou H, Jayaraman LP, et al. Bioactivity screening of partially desulfated low-molecular-weight heparins: A structure/activity relationship study. Glycobiology. 2011;21:1194-205.

[59] Cai L, Lu J, Sheen V, Wang SF. Optimal Poly(L-lysine) Grafting Density in Hydrogels for Promoting Neural Progenitor Cell Functions. Biomacromolecules. 2012;13:1663-74.

[60] Galli D, Benedetti L, Bongio M, Maliardi V, Silvani G, Ceccarelli G, et al. In vitro osteoblastic differentiation of human mesenchymal stem cells and human dental pulp stem cells on poly-L-lysine-treated titanium-6-aluminium-4-vanadium. J Biomed Mater Res Part A. 2011 ;97A:118-26.

[61] Cai L, Lu J, Sheen V, Wang SF. Promoting Nerve Cell Functions on Hydrogels Grafted with Poly(L-lysine). Biomacromolecules. 2012;13:342-9.

[62] Whittle JD, Short RD, Douglas CWI, Davies J. Differences in the aging of allyl alcohol, acrylic acid, allylamine, and octa-1,7-diene plasma polymers as studied by X-ray photoelectron spectroscopy. Chem Mat. 2000;12:2664-71.

[63] Drake SL, Klein DJ, Mickelson DJ, Oegema TR, Furcht LT, McCarthy JB. Cell-surface phosphatidylinositol-anchored heparan-sulfate proteoglycan initiates mouse melanoma cell-adhesion to a fibronectin-derived, heparin-binding synthetic peptide J Cell Biol. 1992;117:1331-41.

[64] Sasaki N, Okishio K, Ui-Tei K, Saigo K, Kinoshita-Toyoda A, Toyoda H, et al. Heparan sulfate regulates self-renewal and pluripotency of embryonic stem cells. J Biol Chem. 2008;283:3594-606.

[65] Kim YS, Linhardt RJ. Structural features of heparin and their effect of heparin cofactor-II mediated inhibition of thrombin Thromb Res. 1989;53:55-71.

[66] Andradegordon P, Strickland S. Interaction of heparin with plasminogen activators and plasminogen-effects on the activation of plasminogen Biochemistry. 1986;25:4033-40.

[67] Barzu T, Lormeau JC, Petitou M, Michelson S, Choay J. Heparin-derived oligosaccharides - Affinity for acidic fibroblast growth-factor and effect on its growth-promoting activity for human-endothelial cells J Cell Physiol. 1989;140:538-48.

[68] Scully MF, Ellis V, Kakkar VV. Heparan-sulfate with no affinity for antithrombin-III and the control of hemostasis. FEBS Lett. 1988;241:11-4.

[69] Hook M, Bjork I, Hopwood J, Lindahl U. Anticoagulant activity of heparin - separation of high-activity and low-activity heparin species by affinity chromatography on immobilized antithrombin FEBS Lett. 1976;66:90-3.

[70] McCaffrey TA, Falcone DJ, Du BH. Transforming growth factor-beta-1 is a heparin-binding protein - Identification of putative heparin-binding regions and isolation of heparins with varying affinity for TGF-beta-1 J Cell Physiol. 1992;152:430-40.

[71] Ampofo SA, Wang HM, Linhardt RJ. Disaccharide compositional analysis of heparin and heparan-sulfate using capillary zone electrophoresis Anal Biochem. 1991;199:249-55.

[72] Desai UR, Wang HM, Ampofo SA, Linhardt RJ. Oligosaccharide composition of heparin and low-molecular-weight heparins by capillary electrophoresis Anal Biochem. 1993;213:120-7.

[73] Jandik KA, Gu KA, Linhardt RJ. Action pattern of polysaccharide lyases on glycosaminoglycans Glycobiology. 1994;4:289-96.

[74] Scapol L, Marchi E, Viscomi GC. Capillary electrophoresis of heparin and dermatan sulfate unsaturated disaccharides with triethylamine and acetonitrile as electrolyte additives. J Chromatogr A. 1996;735:367-74.

[75] Lindahl U, Backstrom G, Thunberg L, Leder IG. Evidence for a 3-O-sulfated D-glucosamine residue in the antithrombin-binding sequence of heparin. Proceedings of the National Academy of Sciences of the United States of America-Biological Sciences. 1980;77:6551-5.

[76] Faham S, Hileman RE, Fromm JR, Linhardt RJ, Rees DC. Heparin structure and interactions with basic fibroblast growth factor. Science. 1996;271:1116-20.

[77] Maccarana M, Casu B, Lindahl U. Minimal sequence in hepain heparan-sulfate required for binding of basic fibroblast growth-factor J Biol Chem. 1993;268:23898-905.

[78] Ernst S, Langer R, Cooney CL, Sasisekharan R. Enzymatic degradation of glycosaminoglycans Crit Rev Biochem Molec Biol. 1995;30:387-444.

[79] Fromm JR, Hileman RE, Weiler JM, Linhardt RJ. Interaction of fibroblast growth factor-1 and related peptides with heparan sulfate and its oligosaccharides. Arch Biochem Biophys. 1997;346:252-62.

[80] Ashikari-Hada S, Habuchi H, Sugaya N, Kobayashi T, Kimata K. Specific inhibition of FGF-2 signaling with 2-O-sulfated octasaccharides of heparan sulfate. Glycobiology. 2009;19:644-54.

[81] Guimond S, Maccarana M, Olwin BB, Lindahl U, Rapraeger AC. Activating and inhibitory heparin sequences for FGF-2 (Basic FGF) - Distinct requirements for FGF-1, FGF-2, and FGF-4 J Biol Chem. 1993;268:23906-14.

[82] Lyon M, Deakin JA, Mizuno K, Nakamura T, Gallagher JT. Interaction of hepatocyte growth-factor with heparan-sulfate - Elucidation of the major heparan-sulfate structural determinants. J Biol Chem. 1994;269:11216-23.

[83] Ling L, Dombrowski C, Foong KM, Haupt LM, Stein GS, Nurcombe V, et al.-Synergism between Wnt3a and heparin enhances osteogenesis via a phosphoinositide 3-kinase/Akt/RUNX2 pathway. J Biol Chem. 2010;285:26233-44.

[84] Punzon-Quijorna E, Sanchez-Vaquero V, Noval AM, Perez DG, Font AC, Ceccone G, et al. Optimized allylamine deposition for improved pluripotential cell culture. Vacuum. 2011;85:1071-5.

[85] Schroder K, Finke B, Ohl A, Luthen F, Bergemann C, Nebe B, et al. Capability of Differently Charged Plasma Polymer Coatings for Control of Tissue Interactions with Titanium Surfaces. J Adhes Sci Technol. 2010;24:1191-205.

## Claims

1. Isolated or purified heparan sulphate HS9, wherein following digestion with heparin lyases I, II and III and then subjecting the resulting disaccharide fragments to capillary electrophoresis analysis the heparan sulphate HS9 has a disaccharide composition comprising:

| Disaccharide | Normalised weight percentage |
|---|---|
| ΔUA,2S-GlcNS,6S | 26.0±3.0 |
| ΔUA,2S-GlcNS | 10.0±2.0 |
| ΔUA-GlcNS,6S | 30.6±3.0 |
| ΔUA,2S-GlcNAc,6S | 1.75±2.0 or 1.7±2.0 |
| ΔUA-GlcNS | 18.0±3.0 |
| ΔUA,2S-GlcNAc | 1.2±0.5 |
| ΔUA-GlcNAc,6S | 12.5±3.0 |

2. Isolated or purified heparan sulphate HS9 according to claim 1 obtained by a method comprising:

(i) providing a solid support having polypeptide molecules adhered to the support, wherein the polypeptide comprises a heparin-binding domain consisting of the amino acid sequence PRPSLAKKQRFRHRNRRKGYRSQRGHSRGRNQNSRR or PRPSLAKKQRFRHRNRKGYRSQRGHSRGRNQNSRR;
(ii) contacting the polypeptide molecules with a mixture comprising glycosaminoglycans such that polypeptide-glycosaminoglycan complexes are allowed to form;
(iii) partitioning polypeptide-glycosaminoglycan complexes from the remainder of the mixture;
(iv) dissociating glycosaminoglycans from the polypeptide-glycosaminoglycan complexes;
(v) collecting the dissociated glycosaminoglycans.

3. Isolated or purified heparan sulphate HS9 according to claim 2 wherein the mixture comprising glycosaminoglycans is a heparan sulphate preparation obtained from porcine intestinal mucosa.

4. A pharmaceutical composition or medicament comprising isolated or substantially purified heparan sulphate HS9 according to any one of claims 1 to 3, optionally wherein the pharmaceutical composition or medicament further comprises vitronectin protein.

5. The pharmaceutical composition or medicament of claim 4, wherein the pharmaceutical composition or medicament is in the form of a biomaterial which is coated or impregnated with heparan sulphate HS9.

6. Isolated or purified heparan sulfate HS9 according to any one of claims 1 to 3, or the pharmaceutical composition or medicament of claim 4 or claim 5, for use in a method of medical treatment.

7. A cell culture dish, plate, bottle, flask, sheetor container having a cell culture substrate comprising isolated or purified heparan sulphate HS9 according to any one of claims 1 to 3, optionally in which at least a part of the cell culture surface is coated in HS9, or wherein at least a part of the cell culture surface is impregnated with heparan sulphate HS9.

8. A cell culture dish, plate, bottle, flask, sheet or container according to claim 7 further comprising Vitronectin.

9. A method of forming a cell culture substrate, the method comprising applying isolated or purified heparan sulphate HS9 according to any one of claims 1 to 3 to a cell culture support surface.

10. An *in vitro* cell culture, comprising cells in contact with a cell culture substrate comprising isolated or purified heparan sulphate HS9 according to any one of claims 1 to 3, optionally wherein the cell culture substrate further comprises Vitronectin.

11. A method of culturing cells, the method comprising culturing cells *in vitro* in contact with a cell culture substrate comprising isolated or purified heparan sulphate HS9 according to any one of claims 1 to 3, optionally wherein the cell culture substrate further comprises Vitronectin.

12. Culture media comprising isolated or purified heparan sulphate HS9 according to any one of claims 1 to 3.

**13.** A biocompatible implant or prosthesis comprising a biomaterial and isolated or purified heparan sulphate HS9 according to any one of claims 1 to 3.

**14.** A biocompatible implant or prosthesis according to claim 13, wherein the biomaterial is coated or impregnated with isolated or purified heparan sulphate HS9, optionally wherein the implant or prosthesis is coated or impregnated with Vitronectin.

**15.** A method of forming a biocompatible implant or prosthesis, the method comprising the step of coating or impregnating a biomaterial with isolated or purified heparan sulphate HS9 according to any one of claims 1 to 3.

**Patentansprüche**

**1.** Isoliertes oder gereinigtes Heparansulfat HS9, wobei nach dem Verdau mit den Heparinlyasen I, II und III und dem Unterziehen der resultierenden Disaccharidfragmente einer Kapillar-Elektrophoreseanalyse das Heparansulfat HS9 eine Disaccharidzusammensetzung aufweist, die Folgendes umfasst:

| Disaccharid | Normal isierter Gewichtsprozentsatz |
|---|---|
| ΔUA,2S-GlcNS,6S | 26,0±3,0 |
| ΔUA,2S-GlcNS | 10,0±2,0 |
| AUA-GlcNS,6S | 30,6±3,0 |
| ΔUA,2S-GlcNAc,6S | 1,75±2,0 oder 1,7±2,0 |
| ΔUA-GlcNS | 18,0±3,0 |
| ΔUA,2S-GlcNAc | 1,2±0,5 |
| ΔUA-GlcNAc,6S | 12,5±3,0 |

**2.** Isoliertes oder gereinigtes Heparansulfat HS9 nach Anspruch 1, das durch ein Verfahren erhalten wurde, das Folgendes umfasst:

(i) Bereitstellen eines festen Trägers mit Polypeptidmolekülen, die an dem Träger haften, wobei das Polypeptid eine Heparinbindungsdomäne umfasst, die aus der Aminosäuresequenz PRPSLAKKQRFRHRNRRKGYRSQRGHSRGRNQNSRR oder PRPSLAKKQRFRHRNRKGYRSQRGHSRGRNQNSRR besteht;
(ii) Kontaktieren der Polypeptidmoleküle mit einem Gemisch, das Glykosaminoglykane umfasst, sodass die Bildung von Polypeptid-Glykosaminoglykan-Komplexen ermöglicht wird;
(iii) Trennen der Polypeptid-Glykosaminoglykan-Komplexe vom Rest des Gemischs;
(iv) Dissoziieren der Glykosaminoglykane aus den Polypeptid-Glykosaminoglykan-Komplexen;
(v) Gewinnen der dissoziierten Glykosaminoglykane.

**3.** Isoliertes oder gereinigtes Heparansulfat HS9 nach Anspruch 2, wobei das Gemisch, das Glykosaminoglykane umfasst, ein Heparansulfatpräparat ist, das aus Darmschleimhaut von Schweinen gewonnen wird.

**4.** Pharmazeutische Zusammensetzung oder Medikament, die/das isoliertes oder im Wesentlichen gereinigtes Heparansulfat HS9 nach einem der Ansprüche 1 bis 3 umfasst, wobei die pharmazeutische Zusammensetzung oder das Medikament gegebenenfalls weiters Vitronectinprotein umfasst.

**5.** Pharmazeutische Zusammensetzung oder Medikament nach Anspruch 4, wobei die pharmazeutische Zusammensetzung oder das Medikament in Form eines Biomaterials vorliegt, das mit Heparansulfat HS9 beschichtet oder imprägniert ist.

**6.** Isoliertes oder gereinigtes Heparansulfat HS9 nach einem der Ansprüche 1 bis 3 oder pharmazeutische Zusammensetzung oder Medikament nach Anspruch 4 oder Anspruch 5 zur Verwendung in einem medizinischen Behandlungsverfahren.

**7.** Zellkulturschale, -platte, -flasche, -kolben, -blatt oder -behälter mit einem Zellkultursubstrat, das isoliertes oder gereinigtes Heparansulfat HS9 nach einem der Ansprüche 1 bis 3 umfasst, wobei gegebenenfalls zumindest ein Teil der Zellkulturoberfläche mit HS9 beschichtet ist oder wobei zumindest ein Teil der Zellkulturoberfläche mit Heparansulfat HS9 imprägniert ist.

**8.** Zellkulturschale, -platte, -flasche, -kolben, -blatt oder -behälter nach Anspruch 7, der weiters Vitronectin umfasst.

**9.** Verfahren zum Bilden eines Zellkultursubstrats, wobei das Verfahren das Aufbringen von isoliertem oder gereinigtem Heparansulfat HS9 nach einem der Ansprüche 1 bis 3 auf eine Zellkultur-Trägerfläche umfasst.

**10.** In-vitro-Zellkultur, umfassend Zellen in Kontakt mit einem Zellkultursubstrat, das isoliertes oder gereinigtes Heparansulfat HS9 nach einem der Ansprüche 1 bis 3 umfasst, wobei das Zellkultursubstrat weiters gegebenenfalls Vitronectin umfasst.

**11.** Verfahren zum Kultivieren von Zellen, wobei das Verfahren das Kultivieren von Zellen in vitro in Kontakt mit einem Zellkultursubstrat, umfassend isoliertes oder gereinigtes Heparansulfat HS9 nach einem der Ansprüche 1 bis 3, umfasst, wobei das Zellkultursubstrat gegebenenfalls weiters Vitronectin umfasst.

**12.** Kulturmedium, umfassend isoliertes oder gereinigtes Heparansulfat HS9 nach einem der Ansprüche 1 bis 3.

**13.** Biokompatible(s) Implantat oder Prothese, umfassend ein Biomaterial und isoliertes oder gereinigtes Heparansulfat HS9 nach einem der Ansprüche 1 bis 3.

**14.** Biokompatible(s) Implantat oder Prothese nach Anspruch 13, wobei das Biomaterial mit isoliertem oder gereinigtem Heparansulfat HS9 beschichtet oder gereinigt ist, wobei das Implantat oder die Prothese gegebenenfalls mit Vitronectin beschichtet oder imprägniert ist.

**15.** Verfahren zur Bildung eines/r biokompatiblen Implantats oder Prothese, wobei das Verfahren den Schritt des Beschichtens oder Imprägnierens eines Biomaterials mit isoliertem oder gereinigtem Heparansulfat HS9 nach einem der Ansprüche 1 bis 3 umfasst.

**Revendications**

**1.** Sulfate d'héparane HS9 isolé ou purifié dans lequel, suite à une digestion par les héparines lyases I, II et III puis le fait de soumettre les fragments disaccharidiques résultants à une analyse par électrophorèse capillaire, le sulfate d'héparane HS9 possède une composition en disaccharides comprenant :

| Disaccharide | Pourcentage en poids normalisé |
|---|---|
| ΔUA,2S-GlcNS,6S | 26,0 ± 3,0 |
| ΔUA,2S-GlcNS | 10,0 ± 2,0 |
| ΔUA-GlcNS,6S | 30,6 ± 3,0 |
| ΔUA,2S-GlcNAc,6S | 1,75 ± 2,0 ou 1,7 ± 2,0 |
| ΔUA-GlcNS | 18,0 ± 3,0 |
| ΔUA,2S-GlcNAc | 1,2 ± 0,5 |
| ΔUA-GlcNAc,6S | 12,5 ± 3,0 |

**2.** Sulfate d'héparane HS9 isolé ou purifié selon la revendication 1, obtenu par un procédé comprenant :

(i) la mise à disposition d'un support solide comportant des molécules de polypeptide adhérées au support, où le polypeptide comprend un domaine de liaison à l'héparine consistant en la séquence d'acides aminés PRPSLAKKQRFRHRNRRKGYRSQRGHSRGRNQNSRR ou PRPSLAKKQRFRHRNRKGYRSQRGHSRGRNQNSRR ;
(ii) la mise en contact des molécules de polypeptide avec un mélange comprenant des glycosaminoglycanes de sorte à permettre la formation de complexes polypeptide-glycosaminoglycane ;

(iii) le partitionnement des complexes polypeptide-glycosaminoglycane à partir du reste du mélange ;

(iv) la dissociation des glycosaminoglycanes à partir des complexes polypeptide-glycosaminoglycane ;

(v) la collecte des glycosaminoglycanes dissociés.

3. Sulfate d'héparane HS9 isolé ou purifié selon la revendication 2, où le mélange comprenant des glycosaminoglycanes est une préparation de sulfate d'héparane obtenue à partir de muqueuse intestinale porcine.

4. Composition pharmaceutique ou médicament comprenant du sulfate d'héparane HS9 isolé ou essentiellement purifié selon l'une quelconque des revendications 1 à 3, facultativement où la composition pharmaceutique ou le médicament comprend en outre une protéine vitronectine.

5. Composition pharmaceutique ou médicament selon la revendication 4, où la composition pharmaceutique ou le médicament est sous la forme d'un biomatériau qui est revêtu ou imprégné de sulfate d'héparane HS9.

6. Sulfate d'héparane HS9 isolé ou purifié selon l'une quelconque des revendications 1 à 3, ou composition pharmaceutique ou médicament selon la revendication 4 ou la revendication 5, destiné(e) à être utilisé(e) dans un procédé de traitement médical.

7. Boîte, plaque, bouteille, flacon, feuille ou récipient de culture cellulaire comportant un substrat de culture cellulaire comprenant du sulfate d'héparane HS9 isolé ou purifié selon l'une quelconque des revendications 1 à 3, facultativement dans laquelle/lequel au moins une partie de la surface de culture cellulaire est revêtue de HS9, ou dans laquelle/lequel au moins une partie de la surface de culture cellulaire est imprégnée de sulfate d'héparane HS9.

8. Boîte, plaque, bouteille, flacon, feuille ou récipient de culture cellulaire selon la revendication 7, comprenant en outre de la vitronectine.

9. Procédé de formation d'un substrat de culture cellulaire, le procédé comprenant l'application de sulfate d'héparane HS9 isolé ou purifié selon l'une quelconque des revendications 1 à 3 sur la surface d'un support de culture cellulaire.

10. Culture cellulaire *in vitro,* comprenant des cellules en contact avec un substrat de culture cellulaire comprenant du sulfate d'héparane HS9 isolé ou purifié selon l'une quelconque des revendications 1 à 3, facultativement dans laquelle le substrat de culture cellulaire comprend en outre de la vitronectine.

11. Procédé de culture de cellules, le procédé comprenant la mise en culture de cellules *in vitro* en contact avec un substrat de culture cellulaire comprenant du sulfate d'héparane HS9 isolé ou purifié selon l'une quelconque des revendications 1 à 3, facultativement dans lequel le substrat de culture cellulaire comprend en outre de la vitronectine.

12. Milieu de culture comprenant du sulfate d'héparane HS9 isolé ou purifié selon l'une quelconque des revendications 1 à 3.

13. Implant ou prothèse biocompatible comprenant un biomatériau et du sulfate d'héparane HS9 isolé ou purifié selon l'une quelconque des revendications 1 à 3.

14. Implant ou prothèse biocompatible selon la revendication 13, dans lequel/laquelle le biomatériau est revêtu ou imprégné de sulfate d'héparane HS9 isolé ou purifié, facultativement où l'implant ou la prothèse est revêtu ou imprégné de vitronectine.

15. Procédé de formation d'un implant ou d'une prothèse biocompatible, le procédé comprenant l'étape consistant à revêtir ou à imprégner un biomatériau de sulfate d'héparane HS9 isolé ou purifié selon l'une quelconque des revendications 1 à 3.

Figure 1

Figure 2

Figure 3

Figure 4

Figure 5

Figure 6

Figure 7

Figure 8

| Disaccharides | Molar percentage (mol%) | | | |
|---|---|---|---|---|
| | Heparin | HSP$^{m}$ | HS9$^{+ve}$ | HS9$^{-ve}$ |
| IS | 66.1 | 10.5 | 26.0 | 13.0 |
| IIIS | 5.5 | 6.3 | 10.0 | 14.7 |
| IIS | 20.8 | 23.7 | 30.6 | 19.8 |
| IA | 1.1 | 2.1 | 1.75 | 1.1 |
| IVS | 2.6 | 36.8 | 18.0 | 33.3 |
| IIIA | 1.5 | 2.2 | 1.20 | 1.45 |
| IIA | 2.4 | 18.4 | 12.5 | 16.6 |

Figure 9

| Allylamine (%) | N:C |
|---|---|
| 0 | 0 |
| 50 | 0.03 ± 0.02 |
| 80 | 0.12 ± 0.02 |
| 90 | 0.17 ± 0.02 |
| 100 | 0.20 ± 0.02 |

Figure 10

Figure 11

Figure 12

Figure 13

Figure 14

Figure 15

Figure 16

Figure 17

| Δ-disaccharide | RSD (%) | |
|---|---|---|
| | Area | RT |
| IS | 2.2 | 0.4 |
| IIIS | 2.3 | 0.4 |
| IA | 2.2 | 0.5 |
| IIS | 2.3 | 0.4 |
| IVS | 2.2 | 0.5 |
| IIIA | 3.2 | 0.5 |
| IIA | 2.1 | 0.5 |
| Internal | 2.2 | 0.5 |

Figure 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010029278 A
- GB 2009000469 W
- WO 2010030244 A
- US 5843780 A
- WO 9623003 A
- US 5019087 A

### Non-patent literature cited in the description

- Remington's Pharmaceutical Sciences. Lippincott, Williams & Wilkins, 2000
- **EVANS, M.J. ; KAUFMAN, M.H.** Establishment in culture of pluripotential cells from mouse embryos. *Nature,* 1981, vol. 292, 154-156
- **MARTIN, G.R.** Isolation of a pluripotent cell line from early mouse embryos cultured in medium conditioned by teratocarcinoma stem cells. *Proc. Natl. Acad. Sci. USA,* 1981, vol. 78, 7634-7638
- **THOMSON, J.A. ; ITSKOVITZ-ELDOR, J. ; SHAPIRO, S.S. ; WAKNITZ, M.A. ; SWIERGIEL, J.J. ; MARSHALL, V.S. ; JONES, J.M.** Embryonic stem cell lines derived from human blastocysts. *Science,* 1998, vol. 282, 1145-1147
- **MATSUI et al.** Derivation of pluripotential embryonic stem cells from murine primordial germ cells in culture. *Cell,* 1992, vol. 70, 841-847
- **JIANG et al.** Pluripotency of mesenchymal stem cells derived from adult marrow. *Nature,* 2002, vol. 418, 41-49
- **KANATSU-SHINOHARA et al.** Generation of pluripotent stem cells from neonatal mouse testis. *Cell,* 2004, vol. 119, 1001-1012
- **KAJI et al.** Virus-free induction of pluripotency and subsequent excision of reprogramming factors. *Nature,* 01 March 2009
- Strategies and New Developments in the Generation of Patient-Specific Pluripotent Stem Cells. **SHINYA YAMANAKA.** Cell Stem Cell. Elsevier Inc, 01 July 2007
- **KLIMANSKAYA I ; CHUNG Y ; BECKER S ; LU SJ ; LANZA R.** Human embryonic stem cell lines derived from single blastomeres. *Nature,* 2006, vol. 444, 512
- **LEI et al.** Xeno-free derivation and culture of human embryonic stem cells: current status, problems and challenges. *Cell Research,* 2007, vol. 17, 682-688
- **CHUNG Y ; KLIMANSKAYA I ; BECKER S et al.** Embryonic and extraembryonic stem cell lines derived from single mouse blastomeres. *Nature,* 2006, vol. 439, 216-219
- **KLIMANSKAYA I ; CHUNG Y ; BECKER S et al.** Human embryonic stem cell lines derived from single blastomeres. *Nature,* 2006, vol. 444, 481-485
- **CHUNG Y ; KLIMANSKAYA I ; BECKER S et al.** Human embryonic stem cell lines generated without embryo destruction. *Cell Stem Cell,* 2008, vol. 2, 113-117
- **DUSKO ILIC et al.** Derivation of human embryonic stem cell lines from biopsied blastomeres dn human feeders with a minimal exposure to xenomaterials. *Stem Cells And Development - paper in pre-publication*
- **ZHANG X ; STOJKOVIC P ; PRZYBORSKI S et al.** Derivation of human embryonic stem cells from developing and arrested embryos. *Stem Cells,* 2006, vol. 24, 2669-2676
- **LIN et al.** Multilineage potential of homozygous stem cells derived from metaphase II oocytes. *Stem Cells,* 2003, vol. 21 (2), 152-61
- **CHRIS H. JO et al.** Fetal mesenchymal stem cells derived from human umbilical cord sustain primitive characteristics during extensive expansion. *Cell Tissue Res,* 2008, vol. 334, 423-433
- **WINSTON COSTA PEREIRA et al.** Reproducible methodology for the isolation of mesenchymal stem cells from human umbilical cord and its potential for cardiomyocyte generation. *J Tissue Eng Regen Med,* 2008, vol. 2, 394-399
- **TROYER ; WEISS.** Concise Review: Wharton's Jelly-Derived Cells Are a primitive Stromal Cell Population. *Stem Cells,* 2008, vol. 26, 591-599
- **KIM et al.** Ex vivo characteristics of human amniotic membrane-derived stem cells. *Cloning Stem Cells 2007 Winter,* 2007, vol. 9 (4), 581-94
- **CHUNG et al.** Human Embryonic Stem Cell Lines Generated without Embryo Destruction. *Cell Stem Cell.,* 2008, vol. 2 (2), 113-117
- **ANDREWS et al.** Cell Lines from Human Germ Cell Tumors. *E. J. Robertson,* 1987
- **KIM et al.** *Science,* 1997, vol. 266, 2011
- **SHAMBLOTT M.J.** *PNAS,* 1998, vol. 95, 13726-13731

- **SCHULDINER M.** *PNAS,* 2000, vol. 97, 11307-11312
- **THOMSON J.A.** *Science,* 1998, vol. 282, 1145-1147
- **REUBINOFF B.E.** *Nature Biotechnology,* 2000, vol. 18, 399-404
- **HENDERSON J.K.** *Stem Cells,* 2002, vol. 20, 329-337
- **PERA M.** *J. Cell Science,* 2000, vol. 113, 5-10
- **LINDAHL et al.** *J. Biol. Chem.,* 1998, vol. 273, 24979
- **SUGAHARA ; KITAGAWA.** *Curr. Opin. Struct. Biol.,* 2000, vol. 10, 518
- **BRICKMAN et al.** *J. Biol. Chem.,* 1998, vol. 273 (8), 4350-4359
- **BRICKMAN et al.** *Glycobiology,* 1998, vol. 8, 463
- **TANAKA, S. ; TAKIGAWA, T. ; ICHIHARA, S. ; NAKAMURA, T.** Mechanical properties of the bioabsorbable polyglycolic acid-collagen nerve guide tube. *Polymer Engineering & Science,* 2006, vol. 46, 1461-1467
- **RAJESH VASITA ; DHIRENDRA S KATTI.** Growth factor delivery systems for tissue engineering: a materials perspective. *Expert Reviews in Medical Devices,* 2006, vol. 3 (1), 29-47
- **WONG C ; INMAN E ; SPAETHE R ; HELGERSON S.** *Thromb.Haemost.,* 2003, vol. 89 (3), 573-582
- **PANDIT AS ; WILSON DJ ; FELDMAN DS.** Fibrin scaffold as an effective vehicle for the delivery of acidic growth factor (FGF-1). *J. Biomaterials Applications,* 2000, vol. 14 (3), 229-242
- **DEBLOIS COTE MF.** Doillon CJ. Heparin-fibroblast growth factor fibrin complex: in vitro and in vivo applications to collagen based materials. *Biomaterials,* 1994, vol. 15 (9), 665-672
- **LUONG-VAN et al.** In vitro biocompatibility and bioactivity of microencapsulated heparan sulphate. *Biomaterials,* 2007, vol. 28, 2127-2136
- **BOSTON CG.** *High potential diagnosis in stem cell research,* 2001, http://wwwslidesharenet/MedicineAndHealth14/stem-cells-sweden
- **GOLDRING CEP ; DUFFY PA ; BENVENISTY N ; ANDREWS PW ; BEN-DAVID U ; EAKINS R et al.** Assessing the Safety of Stem Cell Therapeutics. *Cell Stem Cell,* 2011, vol. 8, 618-28
- **ALPER J.** Geron gets green light for human trial of ES cell-derived product. *Nat Biotechnol.,* 2009, vol. 27, 213-4
- **SCHWARTZ SD ; HUBSCHMAN JP ; HEILWELL G ; FRANCO-CARDENAS V ; PAN CK ; OSTRICK RM et al.** Embryonic stem cell trials for macular degeneration: a preliminary report. *Lancet,* 2012, vol. 379, 713-20
- **LI Y ; POWELL S ; BRUNETTE E ; LEBKOWSKI J ; MANDALAM R.** Expansion of human embryonic stem cells in defined serum-free medium devoid of animal-derived products. *Biotechnol Bioeng.,* 2005, vol. 91, 688-98
- **FURUE MK ; NA J ; JACKSON JP ; OKAMOTO T ; JONES M ; BAKER D et al.** Heparin promotes the growth of human embryonic stem cells in a defined serum-free medium. *Proc Natl Acad Sci U S A.,* 2008, vol. 105, 13409-14
- **LUDWIG TE ; LEVENSTEIN ME ; JONES JM ; BERGGREN WT ; MITCHEN ER ; FRANE JL et al.** Derivation of human embryonic stem cells in defined conditions. *Nat Biotechnol.,* 2006, vol. 24, 185-7
- **WANG L ; SCHUIZ TC ; SHERRER ES ; DAUPHIN DS ; SHIN S ; NELSON AM et al.** Self-renewal of human embryonic stem cells requires insuhn-like growth factor-1 receptor and ERBB2 receptor signaling. *Blood.,* 2007, vol. 110, 4111-9
- **RODIN S ; DOMOGATSKAYA A ; STROM S ; HANSSON EM ; CHIEN KR ; INZUNZA J et al.** Long-term self-renewal of human pluripotent stem cells on human recombinant laminin-511. *Nat Biotechnol.,* 2010, vol. 28, 611-U102
- **XU C ; INOKUMA MS ; DENHAM J ; GOLDS K ; KUNDU P ; GOLD JD et al.** Feeder-free growth of undifferentiated human embryonic stem cells. *Nat Biotechnol.,* 2001, vol. 19, 971-4
- **BRAAM SR ; ZEINSTRA L ; LITJENS S ; WARD-VAN OOSTWAARD D ; VAN DEN BRINK S ; VAN LAAKE L et al.** Recombinant vitronectin is a functionally defined substrate that supports human embryonic stem cell self-renewal via alphavbeta5 integrin. *Stem Cells,* 2008, vol. 26, 2257-65
- **AMIT M ; MARGULETS V ; SEGEV H ; SHARIKI K ; LAEVSKY I ; COLEMAN R et al.** Human feeder layers for human embryonic stem cells. *Biol Reprod.,* 2003, vol. 68, 2150-6
- **NAGAOKA M ; SI-TAYEB K ; AKAIKE T ; DUNCAN SA.** Culture of human pluripotent stem cells using completely defined conditions on a recombinant E-cadherin substratum. *BMC Dev Biol.,* 2010, vol. 10, 12
- **KLIM JR ; LI LY ; WRIGHTON PJ ; PIEKARCZYK MS ; KIESSLING LL.** A defined glycosaminoglycan-binding substratum for human pluripotent stem cells. *Nat Methods,* 2010, vol. 7, 989-U72
- **MELKOUMIAN Z ; WEBER JL ; WEBER DM ; FADEEV AG ; ZHOU Y ; DOLLEY-SONNEVILLE P et al.** Synthetic peptide-acrylate surfaces for long-term self-renewal and cardiomyocyte differentiation of human embryonic stem cells. *Nat Biotechnol.,* 2010, vol. 28, 606-U95

- **VILLA-DIAZ LG ; NANDIVADA H ; DING J ; NOGUEIRA-DE-SOUZA NC ; KREBSBACH PH ; O'SHEA KS et al.** Synthetic polymer coatings for long-term growth of human embryonic stem cells. *Nat Biotechnol.,* 2010, vol. 28, 581-3
- **YAP LYW ; LI J ; PHANG IY ; ONG LT ; OW JAZ-E ; GOH JCH et al.** Defining a Threshold Surface Density of Vitronectin for the Stable Expansion of Human Embryonic Stem Cells. *Tissue Engineering Part C-Methods,* 2011, vol. 17, 193-207
- **MARSON A ; ROCK MJ ; CAIN SA ; FREEMAN LJ ; MORGAN A ; MELLODY K et al.** Homotypic fibrillin-1 interactions in microfibril assembly. *J Biol Chem.,* 2005, vol. 280, 5013-21
- **LINDAHL U ; BACKSTROM G ; HOOK M ; THUNBERG L ; FRANSSON LA ; LINKER A.** Structure of the antithrombin-binding site in heparin. *Proc Natl Acad Sci U S A.,* 1979, vol. 76, 3198-202
- **FALCONE DJ ; SALISBURY BGJ.** Fibronectin stimulates macrophage uptake of low-density lipoprotein-heparin-collagen complexes. *Arteriosclerosis,* 1988, vol. 8, 263-73
- **MAHALINGAM Y ; GALLAGHER JT ; COUCHMAN JR.** Cellular adhesion responses to the heparin-binding (HepII) domain of fibronectin require heparan sulfate with specific properties. *J Biol Chem.,* 2007, vol. 282, 3221-30
- **GANDHI NS ; MANCERA RL.** The Structure of Glycosaminoglycans and their Interactions with Proteins. *Chem Biol Drug Des.,* 2008, vol. 72, 455-82
- **VENKATARAMAN G ; SHRIVER Z ; RAMAN R ; SASISEKHARAN R.** Sequencing complex polysaccharides. *Science,* 1999, vol. 286, 537-42
- **DESAI UR ; WANG HM ; LINHARDT RJ.** Specificity studies on the heparin lyases from Flavobacterium-heparinum. *Biochemistry,* 1993, vol. 32, 8140-5
- **SHRIVER Z ; SUNDARAM M ; VENKATARAMAN G ; FAREED J ; LINHARDT R ; BIEMANN K et al.** Cleavage of the antithrombin III binding site in heparin by heparinases and its implication in the generation of low molecular weight heparin. *Proc Natl Acad Sci U S A.,* 2000, vol. 97, 10365-70
- **KARAMANOS NK ; VANKY P ; TZANAKAKIS GN ; TSEGENIDIS T ; HJERPE A.** Ion-pair high-performance liquid chromatography for determining disaccharide composition in heparin and heparan sulphate. *J Chromatogr A.,* 1997, vol. 765, 169-79
- **VYNIOS DH ; KARAMANOS NK ; TSIGANOS CP.** Advances in analysis of glycosaminoglycans: its application for the assessment of physiological and pathological states of connective tissues. *J Chromatogr B.,* 2002, vol. 781, 21-38
- **HAMPSON IN ; GALLAGHER JT.** Separation of radiolabeled glycosaminoglycan oligosaccharides by polyacrylamide-gel electrophoresis. *Biochem J.,* 1984, vol. 221, 697-705
- **SKIDMORE M AA ; YATES E ; TURNBULL JE.** Labelling heparan sulfate saccharides with chromophore, fluorescence and mass tag for HPLC and MS separations. *Methods in Molecular biology,* 2009, vol. 534, 157-69
- **LAMARI F ; MILITSOPOULOU M ; GIOLDASSI X ; KARAMANOS NK.** Capillary electrophoresis: a superior miniaturized tool for analysis of the mono-, di-, and oligosaccharide constituents of glycan moieties in proteoglycans. *Fresenius J Anal Chem.,* 2001, vol. 371, 157-67
- **KARAMANOS NK ; VANKY P ; TZANAKAKIS GN ; HJERPE A.** High performance capillary electrophoresis method to characterize heparin and heparan sulfate disaccharides. *Electrophoresis,* 1996, vol. 17, 391-5
- **SUDHALTER J ; FOLKMAN J ; SVAHN CM ; BERGENDAL K ; DAMORE PA.** Importance of size, sulfation, and anticoagulant activity in the potentiation of acidic fibroblast growth-factor by heparin. *J Biol Chem.,* 1989, vol. 264, 6892-7
- **MILITSOPOULOU M ; LAMARI FN ; HJERPE A ; KARAMANOS NK.** Determination of twelve heparin- and heparan sulfate-derived disaccharides as 2-aminoacridone derivatives by capillary zone electrophoresis using ultraviolet and laser-induced fluorescence detection. *Electrophoresis,* 2002, vol. 23, 1104-9
- **BRAMONO DS ; RIDER DA ; MURALI S ; NURCOMBE V ; COOL SM.** The Effect of Human Bone Marrow Stroma-Derived Heparan Sulfate on the Ex Vivo Expansion of Human Cord Blood Hematopoietic Stem Cells. *Pharm Res.,* 2011, vol. 28, 1385-94
- **LEVENSTEIN ME ; BERGGREN WT ; LEE JE ; CONARD KR ; LLANAS RA ; WAGNER RJ et al.** Secreted Proteoglycans Directly Mediate Human Embryonic Stem Cell-Basic Fibroblast Growth Factor 2 Interactions Critical for Proliferation. *Stem Cells.,* 2008, vol. 26, 3099-107
- **NURCOMBE V ; COOL SM.** Heparan sulfate control of proliferation and differentiation in the stem cell niche. *Crit Rev Eukaryot Gene Expr.,* 2007, vol. 17, 159-71
- **HELLEDIE T ; DOMBROWSKI C ; RAI B ; LIM ZX ; HIN IL ; RIDER DA et al.** Heparan sulfate enhances the self-renewal and therapeutic potential of mesenchymal stem cells from human adult bone marrow. *Stem Cells Dev.,* 2012, vol. 21, 1897-910
- **UYGUN BE ; STOJSIH SE ; MATTHEW HWT.** Effects of Immobilized Glycosaminoglycans on the Proliferation and Differentiation of Mesenchymal Stem Cells. *Tissue Eng Part A,* 2009, vol. 15, 3499-512

- **NISHIOKA S ; AIKAWA J ; IDA M ; MATSUMOTO I ; STREET M ; TSUJIMOTO M et al.** Ligand-binding activity and expression profile of annexins in Caenorhabditis elegans. *J Biochem.,* 2007, vol. 141, 47-55
- **FENG WY ; ZHAO LH ; WANG KY.** Interaction of polysaccharides with interferon-gamma using an improved ELISA approach. *Carbohydrate Polymers,* 2004, vol. 58, 89-94
- **PLANTE OJ ; PALMACCI ER ; SEEBERGER PH.** Automated solid-phase synthesis of oligosaccharides. *Science,* 2001, vol. 291, 1523-7
- **FOXALL C ; HOLME KR ; LIANG WH ; WEI Z.** An enzyme-linked-immunosorbent-assay using biotinylated heparan-sulfate to evaluate the interactions of heparin-like molecules and basic fibroblast growth-factor. *Anal Biochem.,* 1995, vol. 231, 366-73
- **HASAN M ; NAJJAM S ; GORDON MY ; GIBBS RV ; RIDER CC.** IL-12 is a heparin-binding cytokine. *J Immunol.,* 1999, vol. 162, 1064-70
- **PARKAR AA ; DAY AJ.** Overlapping sites on the Link module of human TSG-6 mediate binding to hyaluronan and chondroitin-4-sulphate. *FEBS Lett.,* 1997, vol. 410, 413-7
- **MAHONEY DJ ; WHITTLE JD ; MILNER CM ; CLARK SJ ; MULLOY B ; BUTTLE DJ et al.** A method for the non-covalent immobilization of heparin to surfaces. *Anal Biochem.,* 2004, vol. 330, 123-9
- **ROBINSON DE ; BUTTLE DJ ; SHORT RD ; MCARTHUR SL ; STEELE DA ; WHITTLE JD.** Glycosaminoglycan (GAG) binding surfaces for characterizing GAG-protein interactions. *Biomaterials,* 2012, vol. 33, 1007-16
- **SUZUKI S ; PIERSCHBACHER MD ; HAYMAN EG ; NGUYEN K ; OHGREN Y ; RUOSLAHTI E.** Domain-structure of vitronectin - Alignment of active-sites. *J Biol Chem.,* 1984, vol. 259, 5307-14
- **WATANABE K ; UENO M ; KAMIYA D ; NISHIYAMA A ; MATSUMURA M ; WATAYA T et al.** A ROCK inhibitor permits survival of dissociated human embryonic stem cells. *Nat Biotechnol.,* 2007, vol. 25, 681-6
- **VAN DEN BORN J ; SALMIVIRTA K ; HENTTINEN T ; OSTMAN N ; ISHIMARU T ; MIYAURA S et al.** Novel heparan sulfate structures revealed by monoclonal antibodies. *J Biol Chem.,* 2005, vol. 280, 20516-23
- **DAVID G ; BAI XM ; VANDERSCHUEREN B ; CASSIMAN JJ ; VANDENBERGHE H.** Developmental-changes in heparan-sulfate expression - insitu detection with mAbs. *J Cell Biol.,* 1992, vol. 119, 961-75
- **BAIRD A ; SCHUBERT D ; LING N ; GUILLEMIN R.** Receptor-binding and heparin-binding domains of basic fibroblast growth-factor. *Proc Natl Acad Sci U S A.,* 1988, vol. 85, 2324-8
- **ONO K ; HATTORI H ; TAKESHITA S ; KURITA A ; ISHIHARA M.** Structural features in heparin that interact with VEGF(165) and modulate its biological activity. *Glycobiology,* 1999, vol. 9, 705-11
- **HAMBARDZUMYAN A ; BILTRESSE S ; DUFRENE Y ; MARCHAND-BRYNAERT J.** An unprecedented surface oxidation of polystyrene substrates by wet chemistry under basic conditions. *J Colloid Interface Sci.,* 2002, vol. 252, 443-9
- **ROBINSON DE ; MARSON A ; SHORT RD ; BUTTLE DJ ; DAY AJ ; PARRY KL et al.** Surface gradient of functional heparin. *Advanced Materials,* 2008, vol. 20, 1166
- **MANTON KJ ; LEONG DF ; COOL SM ; NURCOMBE V.** Disruption of heparan and chondroitin sulfate signaling enhances mesenchymal stem cell-derived osteogenic differentiation via bone morphogenetic protein signaling pathways. *Stem Cells,* 2007, vol. 25, 2845-54
- **RUIZ-CALERO V ; PUIGNOU L ; GALCERAN MT.** Use of reversed polarity and a pressure gradient in the analysis of disaccharide composition of heparin by capillary electrophoresis. *J Chromatogr A.,* 1998, vol. 828, 497-508
- **ROY S ; LAI HC ; ZOUAOUI R ; DUFFNER J ; ZHOU H ; JAYARAMAN LP et al.** Bioactivity screening of partially desulfated low-molecular-weight heparins: A structure/activity relationship study. *Glycobiology,* 2011, vol. 21, 1194-205
- **CAI L ; LU J ; SHEEN V ; WANG SF.** Optimal Poly(L-lysine) Grafting Density in Hydrogels for Promoting Neural Progenitor Cell Functions. *Biomacromolecules,* 2012, vol. 13, 1663-74
- **GALLI D ; BENEDETTI L ; BONGIO M ; MALIARDI V ; SILVANI G ; CECCARELLI G et al.** In vitro osteoblastic differentiation of human mesenchymal stem cells and human dental pulp stem cells on poly-L-lysine-treated titanium-6-aluminium-4-vanadium. *J Biomed Mater Res Part A,* 2011, vol. 97A, 118-26
- **CAI L ; LU J ; SHEEN V ; WANG SF.** Promoting Nerve Cell Functions on Hydrogels Grafted with Poly(L-lysine). *Biomacromolecules,* 2012, vol. 13, 342-9
- **WHITTLE JD ; SHORT RD ; DOUGLAS CWI ; DAVIES J.** Differences in the aging of allyl alcohol, acrylic acid, allylamine, and octa-1,7-diene plasma polymers as studied by X-ray photoelectron spectroscopy. *Chem Mat.,* 2000, vol. 12, 2664-71

- **DRAKE SL ; KLEIN DJ ; MICKELSON DJ ; OEGEMA TR ; FURCHT LT ; MCCARTHY JB.** Cell-surface phosphatidylinositol-anchored heparan-sulfate proteoglycan initiates mouse melanoma cell-adhesion to a fibronectin-derived, heparin-binding synthetic peptide. *J Cell Biol.,* 1992, vol. 117, 1331-41
- **SASAKI N ; OKISHIO K ; UI-TEI K ; SAIGO K ; KINOSHITA-TOYODA A ; TOYODA H et al.** Heparan sulfate regulates self-renewal and pluripotency of embryonic stem cells. *J Biol Chem.,* 2008, vol. 283, 3594-606
- **KIM YS ; LINHARDT RJ.** Structural features of heparin and their effect of heparin cofactor-II mediated inhibition of thrombin. *Thromb Res.,* 1989, vol. 53, 55-71
- **ANDRADEGORDON P ; STRICKLAND S.** Interaction of heparin with plasminogen activators and plasminogen-effects on the activation of plasminogen. *Biochemistry,* 1986, vol. 25, 4033-40
- **BARZU T ; LORMEAU JC ; PETITOU M ; MICHELSON S ; CHOAY J.** Heparin-derived oligosaccharides - Affinity for acidic fibroblast growth-factor and effect on its growth-promoting activity for human-endothelial cells. *J Cell Physiol.,* 1989, vol. 140, 538-48
- **SCULLY MF ; ELLIS V ; KAKKAR VV.** Heparan-sulfate with no affinity for antithrombin-III and the control of hemostasis. *FEBS Lett.,* 1988, vol. 241, 11-4
- **HOOK M ; BJORK I ; HOPWOOD J ; LINDAHL U.** Anticoagulant activity of heparin - separation of high-activity and low-activity heparin species by affinity chromatography on immobilized antithrombin. *FEBS Lett.,* 1976, vol. 66, 90-3
- **MCCAFFREY TA ; FALCONE DJ ; DU BH.** Transforming growth factor-beta-1 is a heparin-binding protein - Identification of putative heparin-binding regions and isolation of heparins with varying affinity for TGF-beta-1. *J Cell Physiol.,* 1992, vol. 152, 430-40
- **AMPOFO SA ; WANG HM ; LINHARDT RJ.** Disaccharide compositional analysis of heparin and heparan-sulfate using capillary zone electrophoresis. *Anal Biochem.,* 1991, vol. 199, 249-55
- **DESAI UR ; WANG HM ; AMPOFO SA ; LINHARDT RJ.** Oligosaccharide composition of heparin and low-molecular-weight heparins by capillary electrophoresis. *Anal Biochem.,* 1993, vol. 213, 120-7
- **JANDIK KA ; GU KA ; LINHARDT RJ.** Action pattern of polysaccharide lyases on glycosaminoglycans. *Glycobiology,* 1994, vol. 4, 289-96
- **SCAPOL L ; MARCHI E ; VISCOMI GC.** Capillary electrophoresis of heparin and dermatan sulfate unsaturated disaccharides with triethylamine and acetonitrile as electrolyte additives. *J Chromatogr A.,* 1996, vol. 735, 367-74
- **LINDAHL U ; BACKSTROM G ; THUNBERG L ; LEDER IG.** Evidence for a 3-O-sulfated D-glucosamine residue in the antithrombin-binding sequence of heparin. *Proceedings of the National Academy of Sciences of the United States of America-Biological Sciences,* 1980, vol. 77, 6551-5
- **FAHAM S ; HILEMAN RE ; FROMM JR ; LINHARDT RJ ; REES DC.** Heparin structure and interactions with basic fibroblast growth factor. *Science,* 1996, vol. 271, 1116-20
- **MACCARANA M ; CASU B ; LINDAHL U.** Minimal sequence in hepain heparan-sulfate required for binding of basic fibroblast growth-factor. *J Biol Chem.,* 1993, vol. 268, 23898-905
- **ERNST S ; LANGER R ; COONEY CL ; SASISEKHARAN R.** Enzymatic degradation of glycosaminoglycans. *Crit Rev Biochem Molec Biol.,* 1995, vol. 30, 387-444
- **FROMM JR ; HILEMAN RE ; WEILER JM ; LINHARDT RJ.** Interaction of fibroblast growth factor-1 and related peptides with heparan sulfate and its oligosaccharides. *Arch Biochem Biophys.,* 1997, vol. 346, 252-62
- **ASHIKARI-HADA S ; HABUCHI H ; SUGAYA N ; KOBAYASHI T ; KIMATA K.** Specific inhibition of FGF-2 signaling with 2-O-sulfated octasaccharides of heparan sulfate. *Glycobiology,* 2009, vol. 19, 644-54
- **GUIMOND S ; MACCARANA M ; OLWIN BB ; LINDAHL U ; RAPRAEGER AC.** Activating and inhibitory heparin sequences for FGF-2 (Basic FGF) - Distinct requirements for FGF-1, FGF-2, and FGF-4. *J Biol Chem.,* 1993, vol. 268, 23906-14
- **LYON M ; DEAKIN JA ; MIZUNO K ; NAKAMURA T ; GALLAGHER JT.** Interaction of hepatocyte growth-factor with heparan-sulfate - Elucidation of the major heparan-sulfate structural determinants. *J Biol Chem.,* 1994, vol. 269, 11216-23
- **LING L ; DOMBROWSKI C ; FOONG KM ; HAUPT LM ; STEIN GS ; NURCOMBE V et al.** Synergism between Wnt3a and heparin enhances osteogenesis via a phosphoinositide 3-kinase/Akt/RUNX2 pathway. *J Biol Chem.,* 2010, vol. 285, 26233-44
- **PUNZON-QUIJORNA E ; SANCHEZ-VAQUERO V ; NOVAL AM ; PEREZ DG ; FONT AC ; CECCONE G et al.** Optimized allylamine deposition for improved pluripotential cell culture. *Vacuum,* 2011, vol. 85, 1071-5
- **SCHRODER K ; FINKE B ; OHL A ; LUTHEN F ; BERGEMANN C ; NEBE B et al.** Capability of Differently Charged Plasma Polymer Coatings for Control of Tissue Interactions with Titanium Surfaces. *J Adhes Sci Technol.,* 2010, vol. 24, 1191-205

SEQUENCE LISTING

<110> Agency for science, Technology and Research

<120> Heparan sulphate

<130> RIC/FP6985683

<150> SG 201304059-7
<151> 2013-05-27

<160> 3

<170> PatentIn version 3.3

<210> 1
<211> 36
<212> PRT
<213> Artificial sequence

<220>
<223> Synthetic construct

<400> 1

```
Pro Arg Pro Ser Leu Ala Lys Lys Gln Arg Phe Arg His Arg Asn Arg
1               5                   10                  15

Arg Lys Gly Tyr Arg Ser Gln Arg Gly His Ser Arg Gly Arg Asn Gln
            20                  25                  30

Asn Ser Arg Arg
            35
```

<210> 2
<211> 478
<212> PRT
<213> Homo sapiens

<400> 2

Met Ala Pro Leu Arg Pro Leu Leu Ile Leu Ala Leu Leu Ala Trp Val
1                   5               10                  15

Ala Leu Ala Asp Gln Glu Ser Cys Lys Gly Arg Cys Thr Glu Gly Phe
                20              25                  30

Asn Val Asp Lys Lys Cys Gln Cys Asp Glu Leu Cys Ser Tyr Tyr Gln
        35              40                  45

Ser Cys Cys Thr Asp Tyr Thr Ala Glu Cys Lys Pro Gln Val Thr Arg
    50              55                  60

Gly Asp Val Phe Thr Met Pro Glu Asp Glu Tyr Thr Val Tyr Asp Asp
65              70                  75                      80

Gly Glu Glu Lys Asn Asn Ala Thr Val His Glu Gln Val Gly Gly Pro
85                    90                95

Ser Leu Thr Ser Asp Leu Gln Ala Gln Ser Lys Gly Asn Pro Glu Gln
100                105                110

Thr Pro Val Leu Lys Pro Glu Glu Glu Ala Pro Ala Pro Glu Val Gly
115                120                125

Ala Ser Lys Pro Glu Gly Ile Asp Ser Arg Pro Glu Thr Leu His Pro
130                135                140

Gly Arg Pro Gln Pro Pro Ala Glu Glu Glu Leu Cys Ser Gly Lys Pro
145                150                155                160

Phe Asp Ala Phe Thr Asp Leu Lys Asn Gly Ser Leu Phe Ala Phe Arg
165                170                175

Gly Gln Tyr Cys Tyr Glu Leu Asp Glu Lys Ala Val Arg Pro Gly Tyr
180                185                190

Pro Lys Leu Ile Arg Asp Val Trp Gly Ile Glu Gly Pro Ile Asp Ala
195                200                205

Ala Phe Thr Arg Ile Asn Cys Gln Gly Lys Thr Tyr Leu Phe Lys Gly
210                215                220

Ser Gln Tyr Trp Arg Phe Glu Asp Gly Val Leu Asp Pro Asp Tyr Pro
225                230                235                240

Arg Asn Ile Ser Asp Gly Phe Asp Gly Ile Pro Asp Asn Val Asp Ala
245                250                255

Ala Leu Ala Leu Pro Ala His Ser Tyr Ser Gly Arg Glu Arg Val Tyr
260                265                270

Phe Phe Lys Gly Lys Gln Tyr Trp Glu Tyr Gln Phe Gln His Gln Pro
275                280                285

Ser Gln Glu Glu Cys Glu Gly Ser Ser Leu Ser Ala Val Phe Glu His
290                295                300

Phe Ala Met Met Gln Arg Asp Ser Trp Glu Asp Ile Phe Glu Leu Leu
305                310                315                320

Phe Trp Gly Arg Thr Ser Ala Gly Thr Arg Gln Pro Gln Phe Ile Ser
325                330                335

```
        Arg Asp Trp His Gly Val Pro Gly Gln Val Asp Ala Ala Met Ala Gly
                    340             345             350

        Arg Ile Tyr Ile Ser Gly Met Ala Pro Arg Pro Ser Leu Ala Lys Lys
                    355             360             365

        Gln Arg Phe Arg His Arg Asn Arg Lys Gly Tyr Arg Ser Gln Arg Gly
                    370             375             380

        His Ser Arg Gly Arg Asn Gln Asn Ser Arg Arg Pro Ser Arg Ala Met
        385             390             395             400

        Trp Leu Ser Leu Phe Ser Ser Glu Glu Ser Asn Leu Gly Ala Asn Asn
                    405             410             415

        Tyr Asp Asp Tyr Arg Met Asp Trp Leu Val Pro Ala Thr Cys Glu Pro
                    420             425             430

        Ile Gln Ser Val Phe Phe Phe Ser Gly Asp Lys Tyr Tyr Arg Val Asn
                    435             440             445

        Leu Arg Thr Arg Arg Val Asp Thr Val Asp Pro Pro Tyr Pro Arg Ser
            450             455             460

        Ile Ala Gln Tyr Trp Leu Gly Cys Pro Ala Pro Gly His Leu
        465             470             475
```

<210> 3
<211> 35
<212> PRT
<213> Artificial sequence

<220>
<223> Synthetic construct

<400> 3

```
        Pro Arg Pro Ser Leu Ala Lys Lys Gln Arg Phe Arg His Arg Asn Arg
        1               5               10              15

        Lys Gly Tyr Arg Ser Gln Arg Gly His Ser Arg Gly Arg Asn Gln
                    20              25              30

        Asn Ser Arg Arg
                    35
```